# EUROPEAN PATENT APPLICATION

(11) **EP 4 310 171 A1**
(43) Date of publication of application: **24.01.2024**
(21) Application number: 22771446.6
(22) Date of filing: 15.03.2022
(51) Int. Cl.: C12M 1/34, C12Q 1/06, C12Q 1/6869, G01N 33/483, G01N 33/53

(54) **NOVEL MEDICAL TECHNIQUE USING FOLLICULAR T-CELLS**

(30) Priority: 16.03.2021 JP 2021042670
(71) Applicant: OSAKA UNIVERSITY, Suita-shi Osaka 565-0871 (JP); KOTAI Biotechnologies, Inc., Suita-shi Osaka 565-0871 (JP)
(72) Inventor: YAMASAKI, Sho, Suita-shi, Osaka 565-0871 (JP); LU, Xiuyuan, Suita-shi, Osaka 565-0871 (JP); HOSONO, Yuki, Suita-shi, Osaka 565-0871 (JP); ISHIZUKA, Shigenari, Suita-shi, Osaka 565-0871 (JP); YAMASHITA, Kazuo, Suita-shi, Osaka 565-0871 (JP); SAX, Nicolas Claude Paul, Suita-shi, Osaka 565-0871 (JP)
(74) Representative: Vossius & Partner Patentanwälte Rechtsanwälte mbB
(86) International application number: PCT/JP2022/011710
(87) International publication number: WO 2022/196701

(57) **Abstract**

A new medical technique using follicular T cells is provided. The present disclosure is based on the finding of public TfhTCR which is specific to disease factors common to various patients, and provides a method for producing follicular T cells specific to a disease, including a step of identifying a disease-related factor or a part thereof having the ability to induce a follicular T cell reactive with the disease-related factor, a step of inducing the follicular T cell specific to the disease, by using the disease-related factor or a part thereof, and a step of obtaining the follicular T cell specific to the disease.

## Description

### [Technical Field]

The present disclosure relates to a novel medical technique using follicular T cells. More particularly, the present disclosure relates to a technique using follicular T cells as an index for diagnosing diseases.

### [Background Art]

Acquired immune responses are generated for various disease factors, but there are many unidentified points about them. Therefore, understanding the acquired immune response to disease factors becomes a key to prediction of long-term immunity and provision of information to vaccine design.

### [Summary of Invention]

### [Solution to Problem]

The present disclosurers found that when a certain infectious factor (such as a virus) is present, public TfhTCR specific to the infectious factor common to various patients is present. By reconstituting the TCR α and β chains, the present disclosurers confirmed that the T cell epitopes were presented by frequent HLA alleles. The present disclosurers found that this TCR clonotype can be used for diagnosis.

Therefore, the present disclosure provides the following.

### (item 1)

A method for evaluating a disease in a subject, comprising measuring a level or amount of a follicular T cell reactive with a disease-related factor of the disease in the subject.

### (item 2)

The method of any of the above-mentioned items, further comprising performing at least one selected from the group consisting of evaluation of the history of the aforementioned disease, evaluation of the effectiveness of a prophylactic agent or vaccine against the aforementioned disease, evaluation of the effectiveness of a therapeutic agent against the aforementioned disease, evaluation of defensive ability against re-contraction of the aforementioned disease, pathology evaluation of the aforementioned disease, and evaluation of the risk of contracting the aforementioned disease, based on the results of the aforementioned measurement.

### (item 3)

The method of any of the above-mentioned items, further comprising inspecting or diagnosing the aforementioned disease in the aforementioned subject.

### (item 4)

The method of any of the above-mentioned items, further comprising evaluating the history of infection with the aforementioned disease in the aforementioned subject.

### (item 5)

The method of any of the above-mentioned items, further comprising evaluating the effectiveness of a vaccine against the aforementioned disease and evaluating defensive ability against re-contraction of the aforementioned disease in the aforementioned subject.

### (item 6)

The method of any of the above-mentioned items, further comprising evaluating the effectiveness of a cancer immunodrug against the aforementioned disease in the aforementioned subject.

### (item 7)

The method of any of the above-mentioned items, wherein the aforementioned disease-related factor is an autoimmune disease-related factor, the aforementioned measurement further comprises measuring the level or amount of the follicular T cell reactive with the autoimmune disease-related factor, and performing risk evaluation and pathology evaluation of an autoimmune disease associated with an autoantibody of the aforementioned subject.

### (item 8)

The method of any of the above-mentioned items, wherein the aforementioned measurement is performed by ELISPOT, flow cytometry, peripheral blood mononuclear cell (PBMC) restimulation assay, next generation sequencer/genetic testing (repertoire analysis), immunostaining (histopathological examination), or in situ analysis.

### (item 9)

The method of any of the above-mentioned items, further comprising comparing the measured level, spatial distribution, or amount of the follicular T cell reactive with the aforementioned disease-related factor with a mean or median value of the level, spatial distribution, or amount of the follicular T cell reactive with the disease-related factor of a test subject not affected with the disease.

### (item 10)

The method of any of the above-mentioned items, wherein the aforementioned disease is an infectious disease or cancer.

### (item 11)

The method of any of the above-mentioned items, wherein the aforementioned disease is a viral infectious disease.

### (item 12)

The method of any of the above-mentioned items, wherein the aforementioned disease is an allergy or an autoimmune disease.

### (item 13)

A reagent or device for evaluating a disease in a subject, comprising a reagent or a device for measuring a level, spatial distribution, or amount of a follicular T cell reactive with a disease-related factor of the disease in the subject.

### (item 14)

The reagent or device of any of the above-mentioned items, comprising a reagent that specifically reacts with the follicular T cell.

### (item 15)

The reagent or device of any of the above-mentioned items, wherein the aforementioned device is used to perform ELISPOT, flow cytometry, peripheral blood mononuclear cell (PBMC) restimulation assay, next generation sequencer/genetic testing (repertoire analysis), immunostaining (histopathological examination), or in situ analysis.

### (item 16)

The reagent or device of any of the above-mentioned items, wherein the aforementioned disease is an infectious disease or cancer.

### (item 17)

The reagent or device of any of the above-mentioned items, wherein the aforementioned disease is a viral infectious disease.

### (item 18)

The reagent or device of any of the above-mentioned items, wherein the aforementioned disease is an allergy or an autoimmune disease.

### (item 19)

A system for evaluating a disease in a subject, comprising a reagent or a device for measuring a level, spatial distribution, or amount of a follicular T cell reactive with a disease-related factor of the disease in the subject, and
an evaluation unit that evaluates measurement results by the reagent or device with regard to the disease.

### (item 20)

The system of any of the above-mentioned items, wherein the aforementioned evaluation unit is configured to perform at least one selected from the group consisting of evaluation of the history of the aforementioned disease, evaluation of the effectiveness of a prophylactic agent or vaccine against the aforementioned disease, evaluation of the effectiveness of a therapeutic agent against the aforementioned disease, evaluation of defensive ability against re-contraction of the aforementioned disease, pathology evaluation of the aforementioned disease, and evaluation of the risk of contracting the aforementioned disease.

### (item 21)

The system of any of the above-mentioned items, further comprising an inspection and diagnosis unit that inspects or diagnoses the aforementioned disease in the aforementioned subject.

### (item 22)

The system of any of the above-mentioned items, further comprising an infection history evaluation unit that evaluates a history of infection with the aforementioned disease in the aforementioned subject.

### (item 23)

The system of any of the above-mentioned items, further comprising a vaccine effectiveness evaluation and re-contraction defensive ability evaluation unit that evaluates effectiveness of vaccine and re-contraction defensive ability against the aforementioned disease in the aforementioned subject.

### (item 24)

The system of any of the above-mentioned items, further comprising a cancer immunodrug effectiveness evaluation unit that evaluates effectiveness of a cancer immunodrug against the aforementioned disease in the aforementioned subject.

### (item 25)

The system of any of the above-mentioned items, wherein the aforementioned disease-related factor is an autoimmune disease-related factor, and the system comprises a follicular T cell measurement unit that measures the level, spatial distribution, or amount of the follicular T cell reactive with the autoimmune disease-related factor, and an autoimmune disease risk evaluation and pathology evaluation unit that performs risk evaluation and pathology evaluation of an autoimmune disease associated with an autoantibody of the aforementioned subject.

### (item 26)

The system of any of the above-mentioned items, comprising a reagent that specifically reacts with the follicular T cell.

### (item 27)

The system of any of the above-mentioned items, wherein the aforementioned device is used to perform ELISPOT, flow cytometry, peripheral blood mononuclear cell (PBMC) restimulation assay, next generation sequencer/genetic testing (repertoire analysis), immunostaining (histopathological examination), or in situ analysis.

### (item 28)

The system of any of the above-mentioned items, further comprising a comparison unit that compares the measured level, spatial distribution, or amount of the follicular T cell reactive with the aforementioned disease-related factor with a mean or median value of the level, spatial distribution, or amount of the follicular T cell reactive with the disease-related factor of a test subject not affected with the disease.

### (item 29)

The system of any of the above-mentioned items, wherein the aforementioned disease is an infectious disease or cancer.

### (item 30)

The system of any of the above-mentioned items, wherein the aforementioned disease is a viral infectious disease.

### (item 31)

The system of any of the above-mentioned items, wherein the aforementioned disease is an allergy or an autoimmune disease.

### (item 32)

A method for evaluating a disease in a subject, comprising a step of providing information relating to a level, spatial distribution, or amount of a follicular T cell reactive with a disease-related factor of the disease in the subject, and a step of evaluating information relating to the level, spatial distribution, or amount of the follicular T cell with regard to the disease.

### (item 33)

The method of any of the above-mentioned items, wherein the aforementioned step of evaluating further comprises performing at least one selected from the group consisting of evaluation of the history of the aforementioned disease, evaluation of the effectiveness of a prophylactic agent or vaccine against the aforementioned disease, evaluation of the effectiveness of a therapeutic agent against the aforementioned disease, evaluation of defensive ability against re-contraction of the aforementioned disease, pathology evaluation of the aforementioned disease, and evaluation of the risk of contracting the aforementioned disease.

### (item 34)

The method of any of the above-mentioned items, comprising evaluating the history of infection with the aforementioned disease in the aforementioned subject.

### (item 35)

The method of any of the above-mentioned items, comprising evaluating the effectiveness of a vaccine against the aforementioned disease and evaluating defensive ability against re-contraction of the aforementioned disease in the aforementioned subject.

### (item 36)

The method of any of the above-mentioned items, comprising evaluating the effectiveness of a cancer immunodrug against the aforementioned disease in the aforementioned subject.

### (item 37)

The method of any of the above-mentioned items, wherein the aforementioned disease-related factor is an autoimmune disease-related factor, the aforementioned measurement comprises measuring the level or amount of the follicular T cell reactive with the autoimmune disease-related factor, and performing risk evaluation and pathology evaluation of an autoimmune disease associated with an autoantibody of the aforementioned subject.

### (item 38)

The method of any of the above-mentioned items, wherein the aforementioned step of providing information is performed by ELISPOT, flow cytometry, peripheral blood mononuclear cell (PBMC) restimulation assay, next generation sequencer/genetic testing (repertoire analysis), immunostaining (histopathological examination), or in situ analysis.

### (item 39)

The method of any of the above-mentioned items, further comprising comparing the information relating to the level, spatial distribution, or amount of the follicular T cell reactive with the aforementioned disease-related factor of the disease in the aforementioned subject with a mean or median value of the information relating to the level, spatial distribution, or amount of the follicular T cell reactive with the disease-related factor of the disease in a test subject not affected with the disease.

### (item 40)

The method of any of the above-mentioned items, wherein the aforementioned disease is an infectious disease or cancer.

### (item 41)

The method of any of the above-mentioned items, wherein the aforementioned disease is a viral infectious disease.

### (item 42)

The method of any of the above-mentioned items, wherein the aforementioned disease is an allergy or an autoimmune disease.

### (item 43)

A program comprising a computer readable code for implementing, on a computer, a method for evaluating a disease in a subject, comprising a step of providing information relating to a level, spatial distribution, or amount of a follicular T cell reactive with a disease-related factor of the disease in the subject, and a step of evaluating information relating to the level, spatial distribution, or amount of the follicular T cell with regard to the disease,

### (item 44)

The program of any of the above-mentioned items, wherein the aforementioned step of evaluating further comprises performing at least one selected from the group consisting of evaluation of the history of the aforementioned disease, evaluation of the effectiveness of a prophylactic agent or vaccine against the aforementioned disease, evaluation of the effectiveness of a therapeutic agent against the aforementioned disease, evaluation of defensive ability against re-contraction of the aforementioned disease, pathology evaluation of the aforementioned disease, and evaluation of the risk of contracting the aforementioned disease.

### (item 45)

The program of any of the above-mentioned items, wherein the aforementioned method comprises evaluating the history of infection with the aforementioned disease in the aforementioned subject.

### (item 46)

The program of any of the above-mentioned items, wherein the aforementioned method comprises evaluating the effectiveness of a vaccine against the aforementioned disease and evaluating defensive ability against re-contraction of the aforementioned disease in the aforementioned subject.

### (item 47)

The program of any of the above-mentioned items, wherein the aforementioned method comprises evaluating the effectiveness of a cancer immunodrug against the aforementioned disease in the aforementioned subject.

### (item 48)

The program of any of the above-mentioned items, wherein the aforementioned disease-related factor is an autoimmune disease-related factor, and in the aforementioned method, the aforementioned measurement further comprises measuring the level or amount of the follicular T cell reactive with the autoimmune disease-related factor, and performing risk evaluation and pathology evaluation of an autoimmune disease associated with an autoantibody of the aforementioned subject.

### (item 49)

The program of any of the above-mentioned items, wherein the aforementioned step of providing information is performed by ELISPOT, flow cytometry, peripheral blood mononuclear cell (PBMC) restimulation assay, next generation sequencer/genetic testing (repertoire analysis), immunostaining (histopathological examination), or in situ analysis.

### (item 50)

The program of any of the above-mentioned items, wherein the method further comprises comparing the information relating to the level, spatial distribution, or amount of the follicular T cell reactive with the aforementioned disease-related factor of the disease in the aforementioned subject with a mean or median value of the information relating to the level, spatial distribution, or amount of the follicular T cell reactive with the disease-related factor of the disease in a test subject not affected with the disease.

### (item 51)

The program of any of the above-mentioned items, wherein the aforementioned disease is an infectious disease or cancer.

### (item 52)

The program of any of the above-mentioned items, wherein the aforementioned disease is a viral infectious disease.

### (item 53)

The program of any of the above-mentioned items, wherein the aforementioned disease is an allergy or an autoimmune disease.

### (item 54)

A computer readable recording medium storing the program of any of the above-mentioned items.

### (item 55)

A computer readable recording medium storing the program of any of the above-mentioned items.

The present disclosure further provides the following items.

### (item A1)

A pharmaceutical composition for treating or preventing a disease, comprising a disease-related factor or a part thereof or a functional equivalent thereof, having the ability to induce a follicular T cell reactive with the disease-related factor of the disease.

### (item A2)

A vaccine for a disease, comprising a disease-related factor or a part thereof or a functional equivalent thereof, having the ability to induce a follicular T cell reactive with the aforementioned disease-related factor.

### (item A3)

The composition or vaccine of any of the aforementioned items, that preferentially induces a follicular T cell.

### (item A4)

A pharmaceutical composition for treating or preventing the aforementioned disease, comprising a follicular T cell reactive with the aforementioned disease-related factor.

### (item A5)

A cell vaccine against the aforementioned disease, comprising a follicular T cell reactive with the aforementioned disease-related factor.

### (item A6)

The composition or vaccine of any of the aforementioned items, wherein the follicular T cell reactive with the aforementioned disease-related factor is reactive with at least the aforementioned disease-related factor.

### (item A7)

The composition or vaccine of any of the aforementioned items, wherein the aforementioned disease is an infectious disease or cancer.

### (item A8)

The composition or vaccine of any of the aforementioned items, wherein the aforementioned disease is a viral infectious disease.

### (item A9)

The composition of any of the aforementioned items, wherein the aforementioned composition induces the follicular T cell in an HLA type-specific manner.

### (item A10)

The composition of any of the aforementioned items, wherein the aforementioned disease-related factor or a part thereof or a functional equivalent thereof comprises 20 amino acids at maximum.

### (item A11)

The composition of any of the aforementioned items, wherein the aforementioned follicular T cell is a public follicular T cell.

### (item A12)

A method for producing a follicular T cell reactive with the aforementioned disease, comprising a step of identifying the aforementioned disease-related factor or a part thereof or a functional equivalent thereof having the ability to induce a follicular T cell reactive with the disease-related factor, a step of inducing the follicular T cell specific to the aforementioned disease, by using the disease-related factor or a part thereof or a functional equivalent thereof, and a step of obtaining the follicular T cell reactive with the disease.

### (item A13)

A method for screening for a follicular T cell reactive with the aforementioned disease, comprising
A) a step of providing a population of follicular T cells,
B) a step of contacting the population of follicular T cells with the aforementioned disease-related factor,
C) a step of measuring the reactivity of the follicular T cells with the disease-related factor, and
D) a step of selecting, from the population of follicular T cells, a follicular T cell exhibiting reactivity of not less than a given value with the disease-related factor.

### (item A14)

The method of the aforementioned item, wherein the step of measuring of the aforementioned C) is performed by ELISPOT, flow cytometry, or peripheral blood mononuclear cell (PBMC) restimulation assay.

### (item B1)

A polypeptide for presenting the aforementioned disease-related factor or a part thereof or a functional equivalent thereof having the ability to induce a follicular T cell reactive with the aforementioned disease-related factor,

### (item B2)

The polypeptide of any of the aforementioned items, that preferentially induces the follicular T cell.

### (item B3)

The polypeptide of any of the aforementioned items, wherein the follicular T cell reactive with the aforementioned disease-related factor is reactive with at least the aforementioned disease-related factor,

### (item B4)

The polypeptide of any of the aforementioned items, wherein the aforementioned disease is an infectious disease or cancer.

### (item B5)

The polypeptide of any of the aforementioned items, wherein the aforementioned disease is a viral infectious disease.

### (item B6)

The polypeptide of any of the aforementioned items, wherein the aforementioned polypeptide induces the follicular T cell in an HLA type-specific manner.

### (item B7)

The polypeptide of any of the aforementioned items, wherein the aforementioned disease-related factor or a part thereof or a functional equivalent thereof comprises 20 amino acids at maximum.

### (item B8)

The polypeptide of any of the aforementioned items, wherein the aforementioned follicular T cell is a public follicular T cell.

### (item C1)

An antibody that specifically binds to TCR that interacts with the aforementioned disease-related factor or a part thereof or a functional equivalent thereof having the ability to induce a follicular T cell reactive with the aforementioned disease-related factor.

### (item C2)

The antibody of any of the aforementioned items, that preferentially induces the follicular T cell.

### (item C3)

The antibody of any of the aforementioned items, wherein the follicular T cell reactive with the aforementioned disease-related factor is reactive with at least the aforementioned disease-related factor.

### (item C4)

The antibody of any of the aforementioned items, wherein the aforementioned disease is an infectious disease or cancer.

### (item C5)

The antibody of any of the aforementioned items, wherein the aforementioned disease is a viral infectious disease.

### (item C6)

The antibody of any of the aforementioned items, wherein the aforementioned antibody induces the follicular T cell in an HLA type-specific manner.

### (item C7)

The antibody of any of the aforementioned items, wherein the aforementioned disease-related factor or a part thereof or a functional equivalent thereof comprises 20 amino acids at maximum.

### (item C8)

The antibody of any of the aforementioned items, wherein the aforementioned follicular T cell is a public follicular T cell.

### (item D1)

A pharmaceutical composition for diagnosing a disease, comprising, as an index, a disease-related factor or a part thereof or a functional equivalent thereof, having the ability to induce a follicular T cell reactive with the aforementioned disease-related factor.

### (item D2)

The pharmaceutical composition of any of the aforementioned items, that preferentially induces the follicular T cell.

### (item D3)

The pharmaceutical composition of any of the aforementioned items, wherein the follicular T cell reactive with the aforementioned disease-related factor is reactive with at least the aforementioned disease-related factor.

### (item D4)

The pharmaceutical composition of any of the aforementioned items, wherein the aforementioned disease is an infectious disease or cancer.

### (item D5)

The pharmaceutical composition of any of the aforementioned items, wherein the aforementioned disease is a viral infectious disease.

### (item D6)

The pharmaceutical composition of any of the aforementioned items, wherein the aforementioned pharmaceutical composition induces the follicular T cell in an HLA type-specific manner.

### (item D7)

The pharmaceutical composition of any of the aforementioned items, wherein the aforementioned disease-related factor or a part thereof or a functional equivalent thereof comprises 20 amino acids at maximum.

### (item D8)

The pharmaceutical composition of any of the aforementioned items, wherein the aforementioned follicular T cell is a public follicular T cell.

### (item E1)

A composition for enhancing acquisition of immunity against the aforementioned disease, comprising a follicular T cell reactive with the aforementioned disease-related factor.

### (item E2)

The composition or vaccine of any of the aforementioned items, wherein the follicular T cell reactive with the aforementioned disease-related factor is reactive with at least the aforementioned disease-related factor.

### (item E3)

The composition or vaccine of any of the aforementioned items, wherein the aforementioned disease is an infectious disease or cancer.

### (item E4)

The composition or vaccine of any of the aforementioned items, wherein the aforementioned disease is a viral infectious disease.

### (item E5)

The composition of any of the aforementioned items, wherein the aforementioned follicular T cell is a public follicular T cell.

### (item X1)

A method for testing an item relating to a disease in a subject, comprising measuring a level or amount of a follicular T cell reactive with a disease-related factor of the disease in the subject, and comparing the level or amount with a given standard.

### (item X2)

The method of any of the above-mentioned items, wherein the aforementioned standard relates to at least one selected from the group consisting of the history of the aforementioned disease, the effectiveness of a prophylactic agent or vaccine against the aforementioned disease, the effectiveness of a therapeutic agent against the aforementioned disease, defensive ability against re-contraction of the aforementioned disease, the pathology of the aforementioned disease, and the risk of contracting the aforementioned disease.

### (item X3)

The method of any of the above-mentioned items, wherein the aforementioned standard relates to the aforementioned disease in the aforementioned subject.

### (item X4)

The method of any of the above-mentioned items, wherein the aforementioned standard relates to the infection history of the aforementioned disease in the aforementioned subject.

### (item X5)

The method of any of the above-mentioned items, wherein the aforementioned standard relates to the vaccine effectiveness and re-contraction defensive ability against the aforementioned disease in the aforementioned subject.

### (item X6)

The method of any of the above-mentioned items, wherein the aforementioned standard relates to the effectiveness of a cancer immunodrug against the aforementioned disease in the aforementioned subject.

### (item X7)

The method of any of the above-mentioned items, wherein the aforementioned disease-related factor is an autoimmune disease-related factor, the aforementioned measurement comprises a measurement of the level or amount of the follicular T cell reactive with the autoimmune disease-related factor, and the aforementioned standard relates to the risk and pathology of an autoimmune disease associated with an autoantibody of the aforementioned subject.

### (item X8)

The method of any of the above-mentioned items, wherein the aforementioned measurement is performed by ELISPOT, flow cytometry, peripheral blood mononuclear cell (PBMC) restimulation assay, next generation sequencer/genetic testing (repertoire analysis), immunostaining (histopathological examination), or in situ analysis.

### (item X9)

The method of any of the above-mentioned items, wherein the aforementioned comparison further comprises comparing the measured level, spatial distribution, or amount of the follicular T cell reactive with the aforementioned disease-related factor with a mean or median value of the level, spatial distribution, or amount of the follicular T cell reactive with the disease-related factor of a test subject not affected with the disease.

### (item X10)

The method of any of the above-mentioned items, wherein the aforementioned disease is an infectious disease or cancer.

### (item X11)

The method of any of the above-mentioned items, wherein the aforementioned disease is a viral infectious disease.

### (item X12)

The method of any of the above-mentioned items, wherein the aforementioned disease is an allergy or an autoimmune disease.

### (item X13)

A reagent or device for evaluating a disease in a subject, comprising a reagent or a device for measuring a level, spatial distribution, or amount of a follicular T cell reactive with a disease-related factor of the disease in the subject.

### (item X14)

The reagent or device of any of the above-mentioned items, comprising a reagent that specifically reacts with the follicular T cell.

### (item X15)

The reagent or device of any of the above-mentioned items, wherein the aforementioned device is used to perform ELISPOT, flow cytometry, peripheral blood mononuclear cell (PBMC) restimulation assay, next generation sequencer/genetic testing (repertoire analysis), immunostaining (histopathological examination), or in situ analysis.

### (item X16)

The reagent or device of any of the above-mentioned items, wherein the aforementioned disease is an infectious disease or cancer.

### (item X17)

The reagent or device of any of the above-mentioned items, wherein the aforementioned disease is a viral infectious disease.

### (item X18)

The reagent or device of any of the above-mentioned items, wherein the aforementioned disease is an allergy or an autoimmune disease.

### (item X19)

A system for testing an item relating to a disease in a subject, comprising a reagent or a device for measuring a level, spatial distribution, or amount of a follicular T cell reactive with a disease-related factor of the disease in the subject, and
a comparison unit that compares measurement results by the reagent or device with a given standard.

### (item X20)

The system of any of the above-mentioned items, wherein the aforementioned standard relates to at least one selected from the group consisting of the history of the aforementioned disease, the effectiveness of a prophylactic agent or vaccine against the aforementioned disease, the effectiveness of a therapeutic agent against the aforementioned disease, defensive ability against re-contraction of the aforementioned disease, pathology evaluation of the aforementioned disease, and the risk of contracting the aforementioned disease.

### (item X21)

The system of any of the above-mentioned items, wherein the aforementioned standard relates to the aforementioned disease in the aforementioned subject.

### (item X22)

The system of any of the above-mentioned items, wherein the aforementioned standard relates to the infection history of the aforementioned disease in the aforementioned subject.

### (item X23)

The system of any of the above-mentioned items, wherein the aforementioned standard relates to the vaccine effectiveness and re-contraction defensive ability against the aforementioned disease in the aforementioned subject.

### (item X24)

The system of any of the above-mentioned items, wherein the aforementioned standard relates to the effectiveness of a cancer immunodrug against the aforementioned disease in the aforementioned subject.

### (item X25)

The system of any of the above-mentioned items, wherein the aforementioned disease-related factor is an autoimmune disease-related factor, a follicular T cell measurement unit for measuring the level, spatial distribution, or amount of the follicular T cell reactive with the autoimmune disease-related factor is further comprised, and the aforementioned standard relates to the risk and pathology of an autoimmune disease associated with an autoantibody of the aforementioned subject.

### (item X26)

The system of any of the above-mentioned items, comprising a reagent that specifically reacts with the follicular T cell.

### (item X27)

The system of any of the above-mentioned items, wherein the aforementioned device is used to perform ELISPOT, flow cytometry, peripheral blood mononuclear cell (PBMC) restimulation assay, next generation sequencer/genetic testing (repertoire analysis), immunostaining (histopathological examination), or in situ analysis.

### (item X28)

The system of any of the above-mentioned items, wherein the aforementioned comparison unit compares the measured level, spatial distribution, or amount of the follicular T cell reactive with the aforementioned disease-related factor with a mean or median value of the level, spatial distribution, or amount of the follicular T cell reactive with the disease-related factor of a test subject not affected with the disease.

### (item X29)

The system of any of the above-mentioned items, wherein the aforementioned disease is an infectious disease or cancer.

### (item X30)

The system of any of the above-mentioned items, wherein the aforementioned disease is a viral infectious disease.

### (item X31)

The system of any of the above-mentioned items, wherein the aforementioned disease is an allergy or an autoimmune disease.

### (item X32)

A method for testing an item relating to a disease in a subject, comprising a step of providing information relating to a level, spatial distribution, or amount of a follicular T cell reactive with a disease-related factor of the disease in the subject, and a step of comparing information relating to the level, spatial distribution, or amount of the follicular T cell with a given standard.

### (item X33)

The method of any of the above-mentioned items, wherein the aforementioned standard relates to at least one selected from the group consisting of the history of the aforementioned disease, the effectiveness of a prophylactic agent or vaccine against the aforementioned disease, the effectiveness of a therapeutic agent against the aforementioned disease, defensive ability against re-contraction of the aforementioned disease, pathology evaluation of the aforementioned disease, and the risk of contracting the aforementioned disease.

### (item X34)

The method of any of the above-mentioned items, wherein the aforementioned standard relates to the infection history of the aforementioned disease in the aforementioned subject.

### (item X35)

The method of any of the above-mentioned items, wherein the aforementioned standard relates to the vaccine effectiveness and re-contraction defensive ability against the aforementioned disease in the aforementioned subject.

### (item X36)

The method of any of the above-mentioned items, wherein the aforementioned standard relates to the effectiveness of a cancer immunodrug against the aforementioned disease in the aforementioned subject.

### (item X37)

The method of any of the above-mentioned items, wherein the aforementioned disease-related factor is an autoimmune disease-related factor, the aforementioned measurement comprises a measurement of the level or amount of the follicular T cell reactive with the autoimmune disease-related factor, and the aforementioned standard relates to the risk and pathology of an autoimmune disease associated with an autoantibody of the aforementioned subject.

### (item X38)

The method of any of the above-mentioned items, wherein the aforementioned step of providing information is performed by ELISPOT, flow cytometry, peripheral blood mononuclear cell (PBMC) restimulation assay, next generation sequencer/genetic testing (repertoire analysis), immunostaining (histopathological examination), or in situ analysis.

### (item X39)

The method of any of the above-mentioned items, wherein the aforementioned comparison further comprises comparing the information relating to the level, spatial distribution, or amount of the follicular T cell reactive with the aforementioned disease-related factor of the disease in the aforementioned subject with a mean or median value of the information relating to the level, spatial distribution, or amount of the follicular T cell reactive with the disease-related factor of the disease in a test subject not affected with the disease.

### (item X40)

The method of any of the above-mentioned items, wherein the aforementioned disease is an infectious disease or cancer.

### (item X41)

The method of any of the above-mentioned items, wherein the aforementioned disease is a viral infectious disease.

### (item X42)

The method of any of the above-mentioned items, wherein the aforementioned disease is an allergy or an autoimmune disease.

### (item X43)

A program comprising a computer readable code for implementing, on a computer, a method for testing an item relating to a disease in a subject, comprising a step of providing information relating to a level, spatial distribution, or amount of a follicular T cell reactive with a disease-related factor of the disease in the subject, and a step of comparing information relating to the level, spatial distribution, or amount of the follicular T cell with a given standard.

### (item X44)

The program of any of the above-mentioned items, wherein the aforementioned standard relates to at least one selected from the group consisting of the history of the aforementioned disease, the effectiveness of a prophylactic agent or vaccine against the aforementioned disease, the effectiveness of a therapeutic agent against the aforementioned disease, defensive ability against re-contraction of the aforementioned disease, pathology evaluation of the aforementioned disease, and the risk of contracting the aforementioned disease.

### (item X45)

The program of any of the above-mentioned items, wherein the aforementioned standard relates to the infection history of the aforementioned disease in the aforementioned subject.

### (item X46)

The program of any of the above-mentioned items, wherein the aforementioned standard relates to the vaccine effectiveness and re-contraction defensive ability against the aforementioned disease in the aforementioned subject.

### (item X47)

The program of any of the above-mentioned items, wherein the aforementioned standard relates to the effectiveness of a cancer immunodrug against the aforementioned disease in the aforementioned subject.

### (item X48)

The program of any of the above-mentioned items, wherein the aforementioned disease-related factor is an autoimmune disease-related factor, the aforementioned method comprises measuring the level or amount of the follicular T cell reactive with the autoimmune disease-related factor, and the aforementioned standard relates to the risk and pathology of an autoimmune disease associated with an autoantibody of the aforementioned subject.

### (item X49)

The program of any of the above-mentioned items, wherein the aforementioned step of providing information is performed by ELISPOT, flow cytometry, peripheral blood mononuclear cell (PBMC) restimulation assay, next generation sequencer/genetic testing (repertoire analysis), immunostaining (histopathological examination), or in situ analysis.

### (item X50)

The program of any of the above-mentioned items, wherein the aforementioned comparison further comprises comparing the information relating to the level, spatial distribution, or amount of the follicular T cell reactive with the disease-related factor of the disease in the aforementioned subject with a mean or median value of the information relating to the level, spatial distribution, or amount of the follicular T cell reactive with the disease-related factor of the disease in a test subject not affected with the disease.

### (item X51)

The program of any of the above-mentioned items, wherein the aforementioned disease is an infectious disease or cancer.

### (item X52)

The program of any of the above-mentioned items, wherein the aforementioned disease is a viral infectious disease.

### (item X53)

The program of any of the above-mentioned items, wherein the aforementioned disease is an allergy or an autoimmune disease.

### (item X54)

A computer readable recording medium storing the program of any of the above-mentioned items.

The present disclosure is intended so that one or more of the aforementioned features can be provided not only as the explicitly disclosed combinations, but also as other combinations. Additional embodiments and advantages of the present disclosure are recognized by those skilled in the art by reading and understanding the following detailed description, as needed.

### [Advantageous Effects of Invention]

The present disclosure is based on the finding of public follicular helper T cells (Tfh) TCR specific to disease factors common to various patients, and provides Tfh specific to a composition containing an epitope specific to a disease factor for inducing follicular T cells.

### [Brief Description of Drawings]

[Fig. 1]

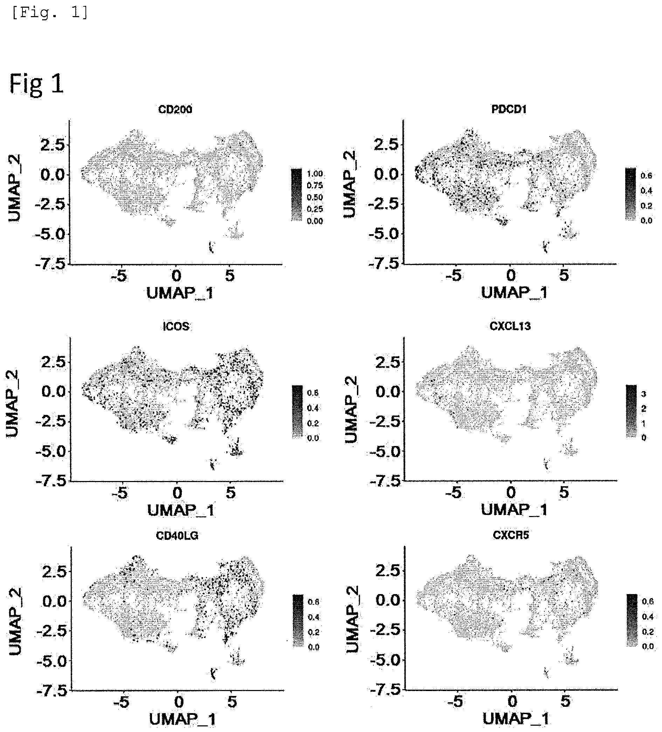

   Fig. 1 is a diagram showing the identified TCR expressed in Tfh cells derived from COVID-19 patients. UMAP projection of 18661 T cells derived from patient-derived PBMC. Each dot corresponds to a single cell. Standard Tfh cell markers CD200, ICOS, CD40LG, PDCD1, CXCL13, and CXCR5 were used to label Tfh clusters displayed in the UMAP plot.
[Fig. 2]

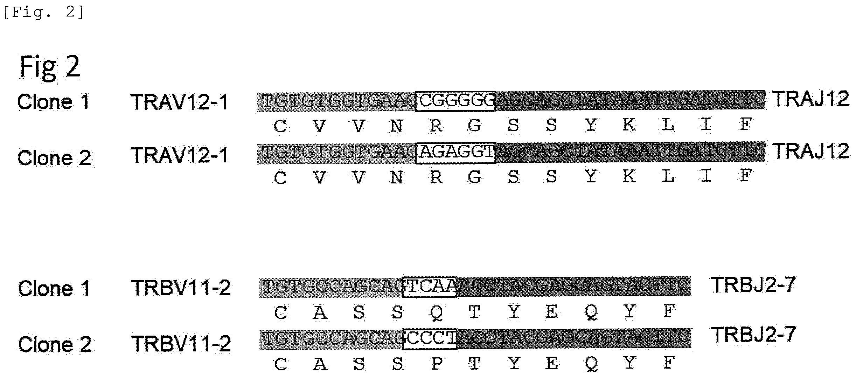

   Fig. 2 is a diagram showing the alignment of TCR CDR3 sequences of clone 1 and clone 2.
[Fig. 3-1]

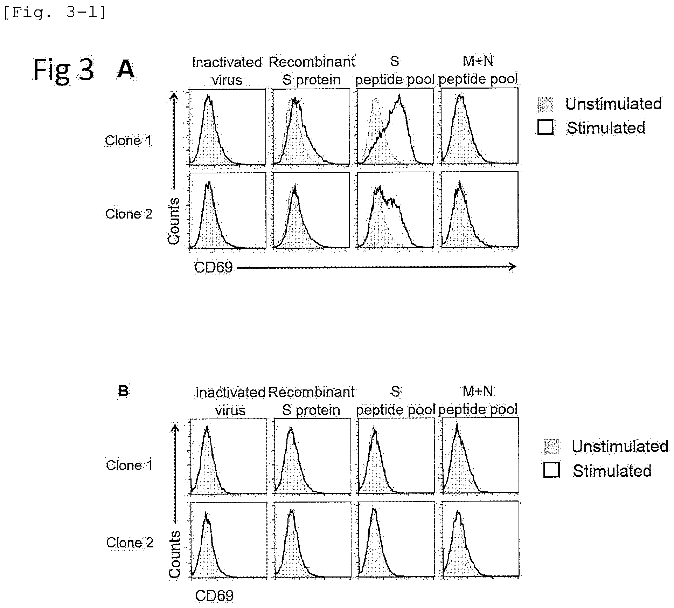

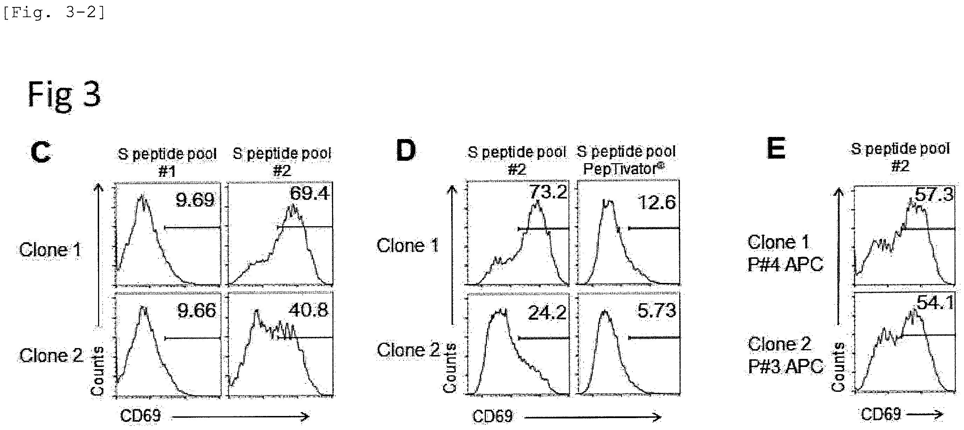

   Fig. 3 is a diagram showing that TCR cloned from Tfh cells of patients is reactive with peptides derived from the SARS-CoV-2 S protein. T-cell hybridomas expressing TCRs of clonotype 1/2 (clone 1 and clone 2, respectively) were left unstimulated (filled histograms) or stimulated with inactivated virus (equivalent to 1 µg/ml S protein), recombinant S protein (1 µg/ml), or S peptide pool and M+N peptide pool (both 1 µg/ml per peptide) (open histograms, respectively), each for 20 hr in the presence (A) or absence (B) of the corresponding donor-derived APCs, and analyzed for CD69 expression (Fig. 3A and 3B).
[Fig. 3-2]
   Fig. 3 is a diagram showing that TCR cloned from Tfh cells of patients is reactive with peptides derived from the SARS-CoV-2 S protein. Clones 1 and 2 were stimulated with S peptide pools #1 and #2 (1 ug/ml per peptide) for 20 hr in the presence of APC of the same origin (Fig. 3C). Clones 1 and 2 were stimulated with S-peptide pool #2 and S-peptide pool PepTivator (registered trademark) (both 0.3 µg/ml per peptide) for 20 hr in the presence of APC of the same origin, and then stained with CD69 (Fig. 3D). Clones 1 and 2 were stimulated with S-peptide pool #2 (1 ug/ml per peptide) for 20 hr in the presence of APCs derived from different donors and then analyzed for CD69 expression (Fig. 3E).
[Fig. 4]

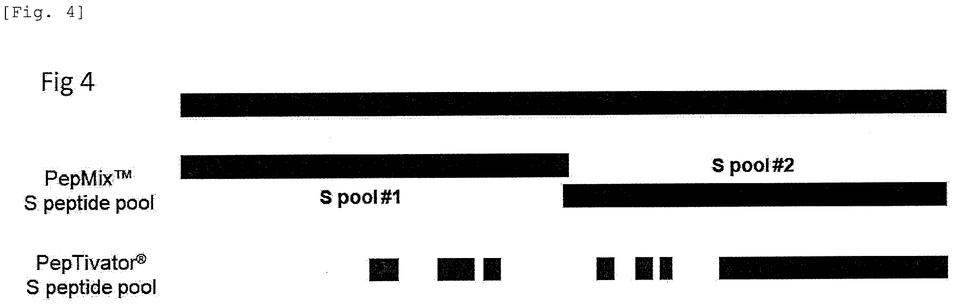

   Fig. 4 is a diagram showing the relative positions of the coverage areas of peptide pools.
[Fig. 5-1]

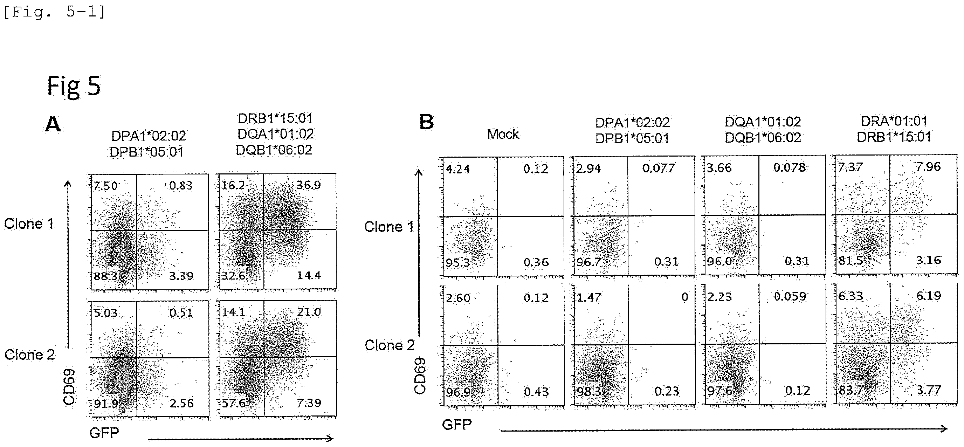

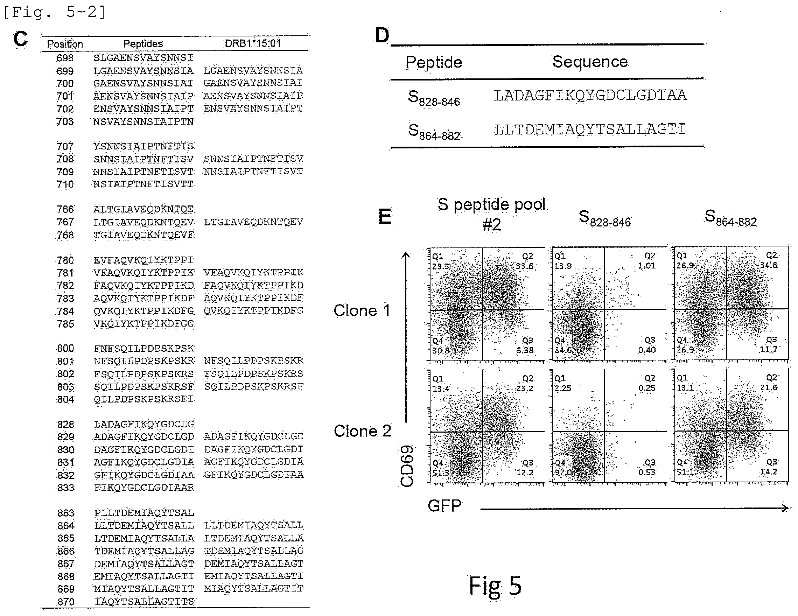

   Fig. 5 is a diagram showing the identified HLA and S protein peptides recognized by S protein-responsive TCRs. T-cell hybridomas expressing TCRs of clonotype 1/2 (clone 1 and clone 2, respectively) were stimulated with S peptide pool #2 (0.3 µg/ml per peptide) in the presence of APCs derived from the same DP or other donor PBMCs having DR/DQ alleles with the original individual (Fig. 5A). Clones 1 and 2 were stimulated with S peptide pool #2 (0.3 µg/ml per peptide) in the presence of HEK293T cells expressing the indicated HLA (Fig. 5B).
[Fig. 5-2]
   Fig. 5 is a diagram showing the identified HLA and S protein peptides recognized by S protein-responsive TCRs. Possible peptides (red: strong binding peptide, gray: weak binding peptide) presented by DRB1*15:01 to a part of S protein containing epitopes of clones 1 and 2 predicted by NetMHC (Fig. 5C). Sequences of two strong binding peptides synthesized (Fig. 5D). Clones 1 and 2 were left unstimulated (filled histograms) or stimulated with S peptide pool #2 (0.3 µg/ml/peptide) and a single peptide (1 µg/ml) (open histograms), each for 20 hr in the presence of APC of different origin, and then stained with CD69 (Fig. 5E).
[Fig. 6-1]

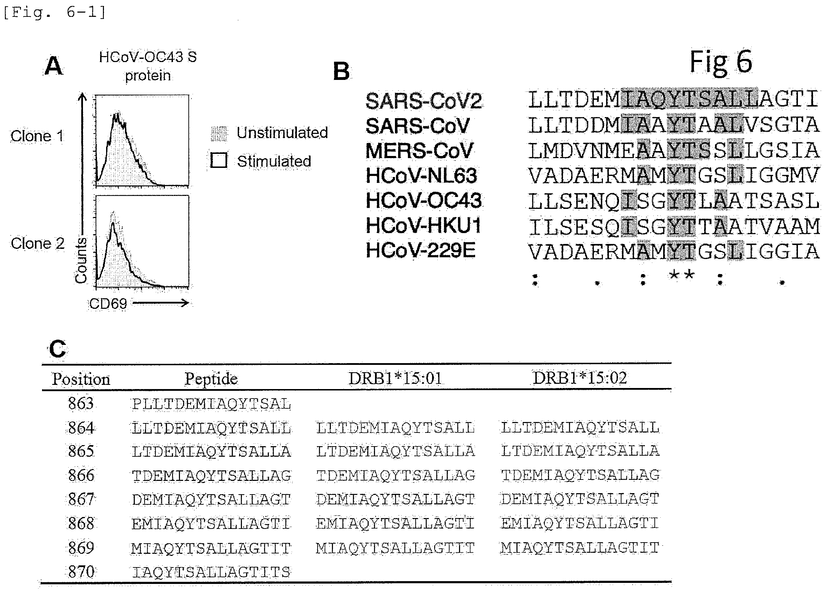

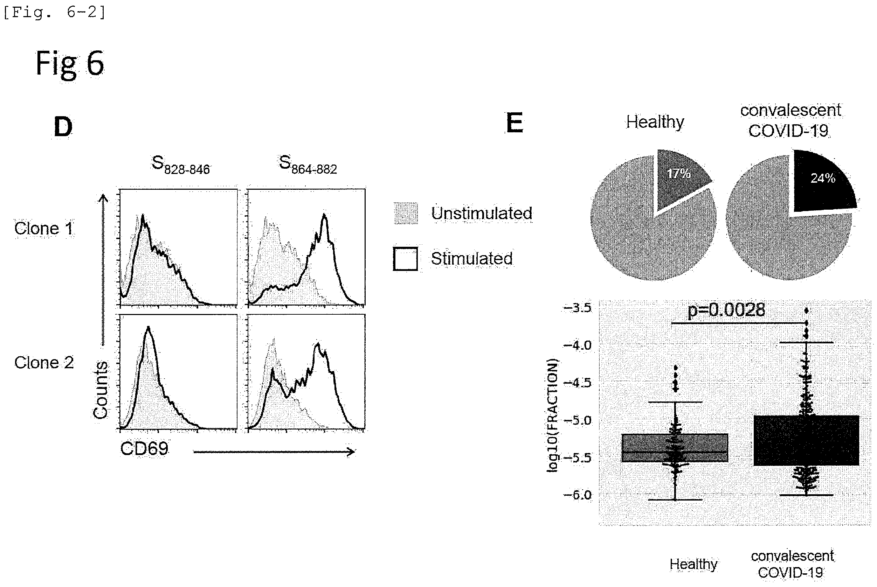

   Fig. 6 shows that S₈₆₄₋₈₈₂ is a SARS-CoV-2-specific peptide that is widely recognized by healthy populations and convalescent COVID-19 patients. T-cell hybridomas expressing TCRs of clonotype 1/2 (clone 1 and clone 2, respectively) were co-cultured with peptide pools derived from APC and HCoV-OC43 S protein (0.3 ug/ml per peptide) (Fig. 6A). Sequence alignment of the corresponding region of S₈₆₄₋₈₈₂ human coronavirus to SARS-CoV-2 (Fig. 6B). Binding capacity of peptide S₈₆₄₋₈₈₂ to DRB1*15:01 and *15:02 predicted by NetMHC4.0 server (red: strong binding peptide, gray: weak binding peptide) (Fig. 6C).
[Fig. 6-2]
   Fig. 6 shows that S₈₆₄₋₈₈₂ is a SARS-CoV-2-specific peptide that is widely recognized by healthy populations and convalescent COVID-19 patients. Clones 1 and 2 were stimulated with a single peptide in the presence of APC with DRB1*15:02 and analyzed for CD69 expression (Fig. 6D). Distribution of clonotype 1/2 in public databases of healthy donors and convalescent COVID-19 patients. Upper panel, percentage of individuals with clonotype 1/2 in both groups, lower panel, increase in clonotype 1/2 in each individual in both groups (Fig. 6E).
[Fig. 7]

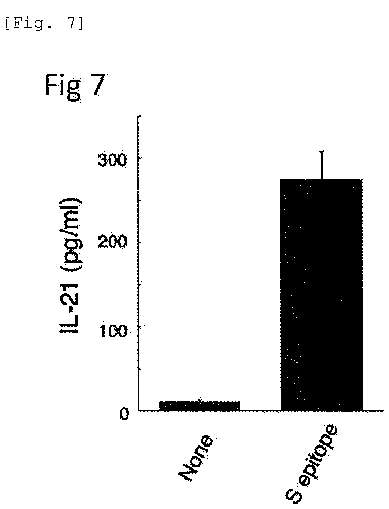

   Fig. 7 shows that a mixture of CD4-positive cells and patient-derived B cells immortalized by EBV infection which is stimulated with SARS-CoV-2 S₈₆₄₋₈₈₂ peptide have higher IL-21 concentrations in the medium compared to unstimulated cells.
[Fig. 8]

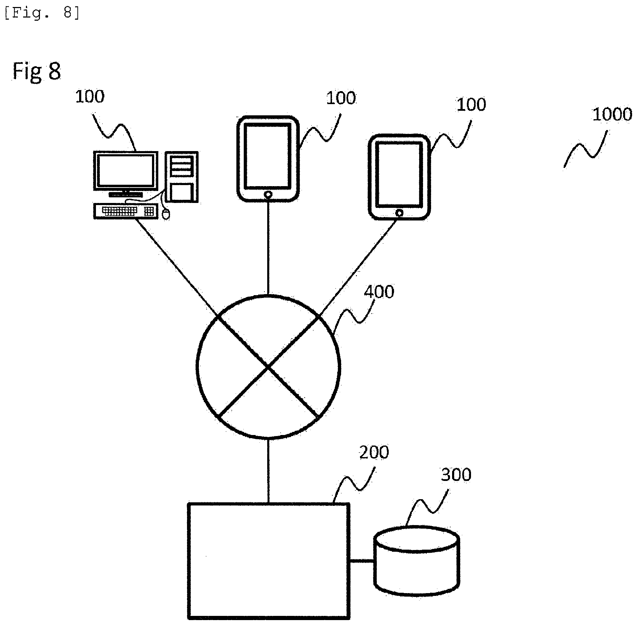

   Fig. 8 is a diagram showing an example of the configuration of the system 1000.
[Fig. 9]

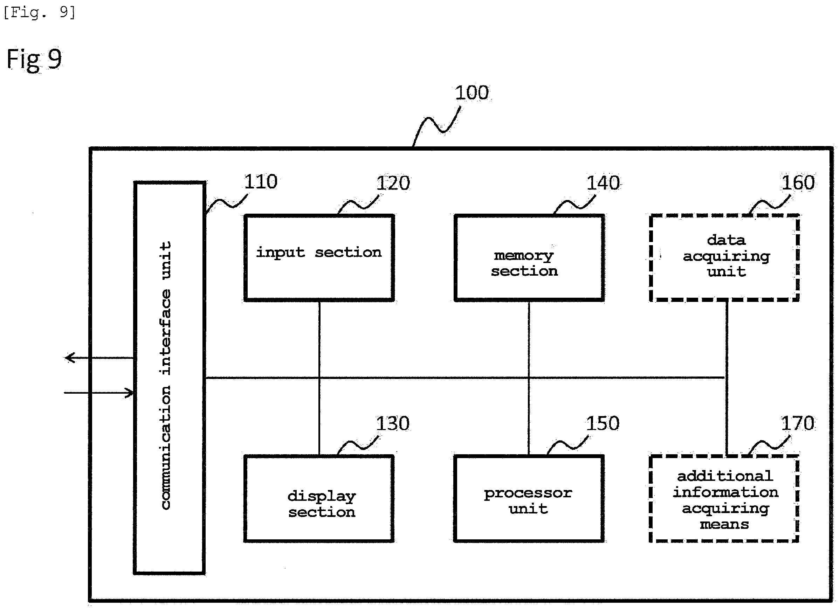

   Fig. 9 is a diagram showing an example of the configuration of the user device 100.
[Fig. 10]

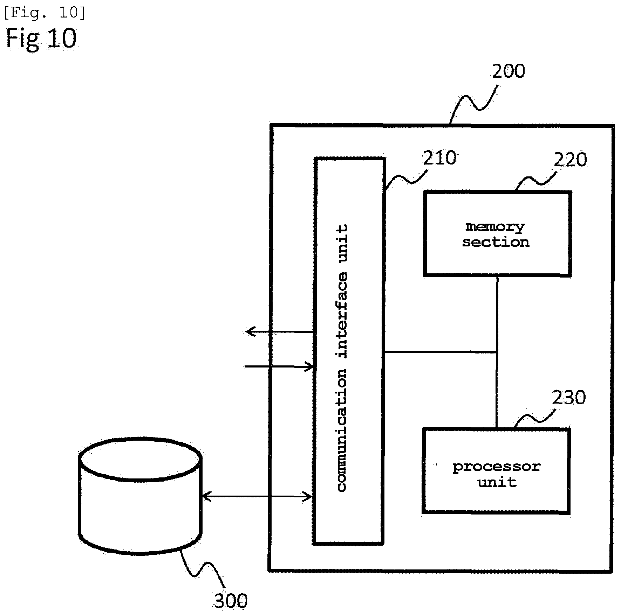

   Fig. 10 shows an example of the configuration of the server device 200.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions and/or basic technical contents of the terms that are particularly used in the present specification are described as appropriate in the following.

### (Definition etc.)

In the present specification, the "follicular T cell" refers to a helper T cell with a shared TCR that regulates B cell maturation and activation, and antibody production. It is also referred to as a follicular helper T cell, and follicular T cell is used interchangeably in the present specification to mean the same as a follicular helper T cell.

In the present specification, the "specific" means that an antibody or fragment thereof, or a cell containing same recognizes and binds a target with higher affinity than any other target.

In the present specification, the (follicular T cell) "reactive" (with a certain target) refers to a follicular T cell that reacts with at least the target and is activated. Whether it reacts with something other than the target is not questioned, and it includes those that react with something other than the target (those that are cross-reactive) and those that have low (preferential to the target) or no reactivity with something other than the target (specific to the target).

In the present specification, the "preferentially inducing" refers to inducing a target at a high rate, for example, induction of 50% or more, 55% or more, 60% or more, 65% or more, 70% or more, 75% or more, 80% or more, 85% or more, 90% or more, 95% or more, or 1000.

In the present specification, the "disease factor" includes not only infectious sources such as viruses, but also neoplasms such as cancer, factors that cause immune abnormalities in the case of autoimmune diseases, and the like.

In the present specification, the "epitope" is a molecule or moiety, also known as an antigen determinant, that is recognized by the immune system, such as an antibody, B cell or T cell, or a site in an antigen molecule to which an antibody or a lymphocyte receptor binds. When used in the present specification, the "epitope" is a molecule capable of binding to a binding moiety (e.g., an antibody or antigenbinding fragment thereof) described in the present specification. Epitopes generally consist of chemically active surface groups of molecules such as amino acids or sugar side chains and generally have specific three-dimensional structural characteristics, and specific electric charge characteristics. In the present specification, methods for determining epitopes are well known in the art and such epitopes can be determined using such well known and conventional techniques by one of ordinary skill in the art once the primary sequence of a nucleic acid or amino acid is provided. In the present specification, the "antigen" refers to any substrate that can be specifically bound by an antibody molecule. In the present specification, the "immunogen" refers to an antigen capable of initiating lymphocyte activation resulting in an antigen-specific immune response.

In the present specification, the "probe in which an epitope is presented to HLA" refers to a probe for presenting an epitope to HLA. By performing an assay using this probe, the establishment of an immune system can be examined by the presence or absence of a cell that binds to the probe. While being a probe, HLA and epitope peptide may be integrated as polypeptide in some cases, and they may not be integrated and peptide may be carried on HLA in some cases.

In the present specification, the "specifically binds to TCR" refers to specific binding to T cell receptor (TCR).

In the present specification, the "enhancing acquisition of immunity" refers to promoting acquisition of immunity such as the innate immunity and acquired immunity, against antigens. For example, maturation and activation of B cells, promoted control of antibody production, and the like by follicular T cells can be mentioned.

In the present specification, the "protective antibody" refers to an antibody that is responsible for immunity against infectious pathogens, particularly the ability to inhibit viral infection, which is observed in active immunity or passive immunity.

In the present specification, the "public follicular T cell" refers to a helper T cell having a TCR shared between individuals that controls maturation and activation of B cells, and antibody production. It is interchangeably used with public follicular helper T cells and public Tfh cells.

In the present specification, the "operably linked" means that a polypeptide encoded by a nucleic acid is linked to an element such that the polypeptide is expressed in a state exhibiting biological activity under the control of the element such as a promoter.

In the present specification, the "disease" in the present disclosure is interchangeably used with "disorder" and "condition", interpreted in a broader sense and refers to a state of bad condition or inconvenience in the mind or body of a human or animal, and refers to any condition that cannot be said a healthy condition which is not specifically defined, such as an illness, disorder, or various symptoms.

In the present specification, the "evaluation" of disease and the like refers to determining the value or significance of some aspect of the target disease by examining the merits and demerits of things, properties, abilities, and the like. Examples thereof include, but are not limited to, evaluation of the history of the disease, evaluation of the effectiveness of a prophylactic agent or vaccine against the disease, evaluation of the effectiveness of a therapeutic agent against the disease (e.g. cancer immunodrug such as immunocheckpoint agents in the case of cancer), evaluation of defensive ability against re-contraction of the disease (e.g., reinfection in the case of infectious disease), pathology evaluation of the disease (e.g., cancer, infectious disease, autoimmune disease, and allergy), and evaluation of the risk of contracting the disease.

In the present specification, the "disease-related factor" refers to a factor that directly causes the disease, as well as factors that indirectly cause the disease, such as factors that cause the direct factor, and any factor that causes a change in a disease state in a subject compared to when the disease state is not present, such as, consequential factors that occur as a result of the disease, factors that occur in parallel, factors that occur in correlation, and the like. A factor that induces a disease is referred to as "disease causative factor". For example, in the case of infectious diseases, it refers to a pathogenic factor or an infectious factor such as viruses, bacteria, protozoa, and mycoplasma; in the case of autoimmune diseases such as allergy and the like, a substance to be the immunogen (causative substance) such as allergen and the like can be mentioned; and tumor cells, toxic substances and the like can be mentioned in the case of neoplasm. On the other hand, a factor that occurs as a result of a disease is also called a "disease resultant factor" and in this case, it includes factors and metabolites related to the immune response in the subject.

In the present specification, the "reactive" in "follicular T cells reactive with disease-related factors" means that when a follicular T cell is placed under conditions under which it can interact with a disease-related factor, a change occurs in that other factor in the presence of that factor as compared to the absence of that factor. A follicular T cell that is reactive with a disease-related factor means that the follicular T cell undergoes some positive or negative change when placed under conditions where it can interact with the disease-related factor.

In the present specification, the "level" of follicular T cells means the degree of function of the T cells, and the "amount" of follicular T cells means the degree of physical amount of the T cells.

In the present specification, the "viral antigen" refers to a part or all of a virus to be a target to which an immune response is desired to be induced, and refers to an antigen that elicits an immune response when administered to a host. Peptides, the whole (lysates), and the like can be used. Alternatively, virus-like particles (VLP) may be formed and utilized. Examples of the viral antigen target include, but are not limited to, antigens and the like of HIV, hepatitis B virus, hepatitis C virus, coronavirus, norovirus, rotavirus, rabies virus, West Nile virus, papillomavirus, Zika virus, rubella virus, cytomegalovirus, influenza virus, and avian influenza virus.

In the present specification, the "bacterial antigen" refers to a part or all of a bacterium to which an immune response is desired to be induced, and refers to an antigen that elicits an immune response when administered to a host. Peptides, the whole (lysates), and the like can be used. Examples of the bacterial antigen target include, but are not limited to, antigens derived from Bacillus anthracis, Bordetella pertussis, Borrelia burgdorferi, Brucella abortus, Brucella canis, Brucella melitensis, Brucella suis, Burkholderia mallei, Burkholderia pseudomallei, Campylobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium diptheriae, Enterococcus faecalis, Enterococcus faecium, Escherichia coli, enterotoxigenic Escherichia coli, enteropathogenic Escherichia coli, Escherichia coli)157:H7, Francisella tularensis, Haemophilus influenza, Helicobacter pylori, Legionella pneumophila, Leptospira interrogans, Listeria monocytogenes, Mycobacterium leprae, Mycobacterium tuberculosis, Mycoplasma pneumoniae, Neisseria gonorrhoeae, Neisseria meningitides, Pseudomonas aeruginosa, Rickettsia rickettsia, Salmonella typhi, Salmonella typhimurium, Shigella sonnei, Staphylococcus aureus, Staphylococcus epidermidis, Staphylococcus saprophyticus, Streptococcus agalactiae, Streptococcus pneumoniae, Streptococcus pyogenes, Treponema pallidum, Vibrio cholerae, and Yersinia pestis.

In the present specification, the "fungal antigen" refers to a part or all of a fungus to be a target to which an immune response is desired to be induced, and refers to an antigen that elicits an immune response when administered to a host. Peptides, the whole (lysates), and the like can be used. Examples of the fungal antigen target include, but are not limited to, antigens derived from Aspergillus clavatus, Aspergillus flavus, Aspergillus fumigatus, Aspergillus nidulans, Aspergillus niger, Aspergillus terreus, Blastomyces dermatitidis, Candida albicans, Candida dubliniensis, Candida glabrata, Candida parapsilosis, Candida rugosa, Candida tropicalis, Cryptococcus albidus, Cryptococcus gattii, Cryptococcus laurentii, Cryptococcus neoformans, Histoplasma capsulatum, Microsporum canis, Pneumocystis carinii, Pneumocystis jirovecii, Sporothrix schenckii, Stachbotrys chartarum, Tinea barbae, Tinea captitis, Tinea corporis, Tinea cruris, Tinea faciei, Tinea incognito, Tinea nigra, Tinea versicolor, Trichophyton rubrum, and Trichophyton tonsurans.

In the present specification, the "immune abnormality" refers to any disease, disorder or condition caused or suspected to be caused, at least in part, by an abnormality of the immune system. It refers to condition in which the immune system is abnormal due to some cause, making it easier to get infections or causing allergic reactions. Immune abnormality include, but are not limited to, allergies, autoimmune diseases, and the like. When it is directed against one's own antigens, it is generally called an autoimmune disease, and when it is directed against foreign antigens, it is called an allergy. When the immune response is strong, it becomes an autoimmune disease state against self antigens, while becoming an allergic state against non-self antigens. It can be said that a person with a weak immune response develops a cancerous state against self antigens and an infectious disease against non-self antigens.

In the present specification, the "infectious disease" may be any infectious disease, and may include any kinds of infectious diseases such as a viral infectious disease (including any form of virus such as single-stranded or double-stranded DNA virus, RNA virus, etc.), bacterium infectious disease, protozoa infectious disease, Mycoplasma infection and the like, for example, tuberculosis, Coronavirus, malaria, yellow fever virus, smallpox virus, smallpox vaccination, measles/rubella, polio, epidemic parotitis (mumps)/MUMPS, rotaviral infection, varicella (possum), yellow fever, Ebola, West Nilefever, hib infection, pneumococcul infection, pertussis, Japanese encephalitis, meningococcal infection, Salmonella infection, pathogenic Escherichia coli, Toxoplasma, Zika virus, herpes virus type 1, EBV/Epstein-Barr virus (herpes virus type 4), CMV/cytomegalovirus (herpes virus type 5), influenza, MARS, rabies and diphtheria and the like.

In the present specification, the "cancer" is used in the same sense as commonly used in the pertinent field, and refers to a malignant tumor or the state of presence of such a malignant tumor that is highly atypical, proliferates more rapidly than normal cells, and can destructively infiltrate or metastasize to surrounding tissues. In the present disclosure, cancer includes, but is not limited to, solid tumors and hematopoietic tumors and is used interchangeably with "neoplasm" and "tumor" in the present specification. Cancer, neoplasm, and tumor encompass hematopoietic neoplasms as well as solid neoplasms. Examples of neoplasms include, but are not limited to, melanoma, non-small cell lung, small cell lung, lung, hepatocarcinoma, retinoblastoma, astrocytoma, glioblastoma, gingiva, tongue, leukemia, neuroblastoma, head, neck, chest, pancreas, prostate, kidney, bone, testis, ovary, mesothelioma, sarcoma, cervix, stomach and intestine, lymphoma, brain, colon, bladder, myeloma, and other malignant or benign neoplasms. More specifically, the neoplasm may be a hematopoietic neoplasm selected from the group consisting of acute myeloid leukemia, acute lymphoblastic leukemia, myelodysplastic syndrome, chronic myelomonocytic leukemia, juvenile myelomonocytic leukemia, multiple myeloma, and chronic lymphocytic leukemia.

In the present specification, the "autoimmune disease" refers to diseases in which autoantibodies (e.g., anti-Sm antibody, anti-dsDNA antibody, rheumatoid factor) against auto-components (e.g., immunoglobulins) are detected and autoimmunity is involved in the pathology. Autoimmune diseases include, but are not limited to, organ-specific autoimmune diseases (e.g., chronic thyroiditis, primary mucosal edema, thyrotoxicosis, pernicious anemia, Goodpasture syndrome, acute progressive glomerulonephritis, myasthenia gravis, pemphigus vulgaris, bullous pemphigoid, insulin-resistant diabetes, juvenile diabetes, Addison's disease, atrophic gastritis, male infertility, premature menopause, lensogenic uveitis, sympathetic pulsitis, multiple sclerosis, ulcerative colitis, primary biliary cirrhosis, chronic active hepatitis, autoimmune hemolytic anemia, paroxysmal hemoglobinuria, sudden thrombocytopenic purpura, and Sjogren syndrome), and organnonspecific autoimmune diseases (e.g., rheumatoid arthritis, systemic lupus erythematosus (SLE), discoid lupus erythematosus, polymyositis, scleroderma, and mixed connective tissue diseases. Rheumatoid arthritis, systemic lupus erythematosus, discoid lupus erythematosus, polymyositis, scleroderma, and mixed connective tissue diseases are known as collagen diseases. In other words, collagen diseases are included in systemic autoimmune diseases.

In the present specification, the "allergy" is a disease in which the immune response is excessively directed against a specific non-self antigen, and the immune response is directed against an "allergen". The "allergen" refers to an antigen that can react with the antibody of a subject with an allergic disease. Examples thereof include, but are not limited to, allergen derived from tree pollen (acacia, alder, velvet ash, beech, birch, maple, mountain cedar, red cedar, box elder, cypress, American elm, Chinese elm, togasawara, gum tree, eucalyptus, enoki, hickory, sugar maple, mesquite, casinos, konara spp, Eucalyptus trees, enoki, hickory, American linden, sugar maple, mesquite, cashew, Quercus spp., olive, pecan, pepper, pine, Japanese knotweed, Russian olive, American spruce, Japanese elder, black walnut, black willow, etc.), allergen derived from plant pollen (cottonwood, gewgaw, nagahagrass, sparrowhawk, corn, hirsutake grass, savannah corn, crowfoot, chamogaya, cornucopia, perennial rye-grass, rice, Harugaya, Japanese knotweed, daylily, red spider lily, Japanese white fir, Common Sorrel, Solidago altissima, Iso borax, shiroza, calendula, nettle, green foxtail millet, spatula psyllium, Ambrosia trifida, ragweed, Perennial ragweed, Salsola tragus, Yamayomogi Japanese mugwort, broom, Sheep sorrel etc.), allergen derived from insects (Silkworm, mites, bees, wasps, ants, cockroaches etc.), allergen derived from fungi (Alternaria, Aspergillus, Botulinum, Candida, Cephalosporium, Carbularia, Epicoccum, Epidermophyton, Fusarium, Helminthosporium, Chain Cladosporium, Kekavi, Penicillium, Pharma, Pluralia pullulans, Beetle Mold, etc..), allergen derived from animal hair (dog, cat, bird, etc.), allergen protein derived from house dust, allergen derived from food (OVA etc.), and the like. Representative diseases of "allergy" include atopic dermatitis, allergic rhinitis (hay fever, etc.), allergic conjunctivitis, allergic gastroenteritis, bronchial asthma, childhood asthma, food allergy, drug allergy, urticaria, and the like.

In the present specification, the "inflammatory diseases" refers to a disease or condition characterized by abnormal inflammation (e.g., elevated levels of inflammation compared to controls, such as healthy individuals who do not have the disease). As non-limiting examples of inflammatory diseases, atopy, asthma, autoinflammatory diseases, hypersensitivity, childhood allergic asthma, allergic asthma, inflammatory bowel disease, Celiac disease, Crohn's disease, colitis, ulcerative colitis, collagenous colitis, lymphocytic colitis, diverticulitis, irritable bowel syndrome, short bowel syndrome, blind loop syndrome, chronic sustainability diarrhea, refractory diarrhea in infancy, traveller's diarrhea, immunoproliferative small intestinal disease, chronic prostatitis, postenteritis syndrome, tropical sprue, Whipple's disease, Wolman disease, arthritis, rheumatoid arthritis, Behcet's disease, uveitis, pyoderma gangrenosum, erythema nodosum, traumatic brain damage, psoriatic arthritis, juvenile idiopathic arthritis, multiple sclerosis, systemic lupus erythematosus (SLE), myasthenia gravis, young people onset diabetes, type 1 diabetes, Guillain-Barre syndrome, Hashimoto encephalitis, Hashimoto thyroiditis, ankylopoietic spondylarthritis, psoriasis, Sjogren's syndrome, vasculitis, glomerulonephritis, autoimmune thyroiditis, bullous pemphigoid, sarcoidosis, Ichthyosis, Graves' ophthalmopathy, Addison's disease, vitiligo, acne vulgaris, pelvic inflammatory disease, reperfusion injury, sarcoidosis, graft rejection, interstitial cystitis, atherosclerosis, and atopic dermatitis can be mentioned.

HLA-binding properties should also be considered in the application of the technique of the present disclosure. Complexes of HLA class I molecules and peptides derived from disease factor antigens that are recognized by cytotoxic (killer) T cells when the killer T cells recognize disease factors and eliminate them by damaging them have been identified and such class information can be used. Certain disease factors have a common HLA type for a common antigen, and this approach can be used to identify antigen peptides for use in specific cohorts of dendritic cell preparations of the present disclosure. After immunizing transgenic mice of this specific HLA type with these synthetic peptides, peptides that can induce an immune response in killer T cells can be identified and used. Using a similar method and Tgm of HLA antigens, peptides derived from antigens of disease factors that bind to specific HLA molecules that are most frequent in the Japanese population can be identified, and this method can be used to identify antigen peptides for a specific cohort of dendritic cell preparations of the present disclosure (see below as a method: Chen, Y.-Z., Liu, G., Senju, S., Wang, Q., Irie, A., Haruta, M., ... Nishimura, Y. (2010). Identification of SARS-COV Spike Protein-Derived and HLA-A2-Restricted Human CTL Epitopes by Using a New Muramyl Dipeptide-Derivative Adjuvant. International Journal of Immunopathology and Pharmacology, 165-177. https://doi.org/10.1177/039463201002300115). Using such information, it is possible to identify viral antigens that bind to specific HLA types recognized by killer T cells that contribute to the elimination of disease factors (HLA types other than those identified are unique to those identified by separate identification). The dendritic cell vaccine of the present disclosure, which is produced by inducing differentiation of dendritic cells from human ES cells expressing these HLAs, is expected to activate killer T cells in an antigen-specific manner and prevent severe disease caused by cytokine storms and the like. Representative antigen peptides used in the present disclosure can be HLA-binding or HLA-unbinding. For binding, a single peptide can be used, but for non-binding, multiple overlapped peptides or electroporation of viral nucleic acid is preferred.

There are two major types of T cells: CD8+ T cells and CD4+ T cells. These two types of T cells are essentially different in the antigens they recognize. CD8+ T cells recognize a peptide, typically made up of nine amino acids, bound to a type of HLA (MHC) called class I. On the other hand, CD4+ T cells recognize MHC-binding peptides called class II. The length of peptides that bind to HLA (MHC) class II is considered to be, for example but not limited to, 15 to 24 amino acids. HLA (MHC) class I molecules are expressed in all somatic cells, and bind and present protein fragments produced by self-cells. HLA (MHC) class II molecules are expressed in limited cells such as antigen-presenting cells such as macrophages and dendritic cells, and B cells. Unlike normal somatic cells, antigen-presenting cells take in proteins from the outside world, degrade them, and then combine them with HLA (MHC) class I and II molecules and present them. Therefore, CD8+ T cells primarily recognize endogenous HLA (MHC) class I-restricted antigens on somatic cells, whereas CD4+ T cells recognize exogenous HLA (MHC) class II-restricted antigens on antigen-presenting cells. In humans, MHC class I is HLA A, B, C, and MHC class II is HLA DR, DQ, DP.

In the present specification, the dosage form of "pharmaceutical (composition)" is not particularly limited, and may be a solid, semi-solid, or liquid preparation, and can be selected according to the purpose of use, and the like. In the case of cellular preparations, they are generally provided as liquid preparations, but may also be provided as freezed or freeze-dried preparations. The dosage forms of pharmaceutical compositions are described, for example, in the General Rules for Pharmaceutical Preparations of the Japanese Pharmacopoeia, Seventeenth Edition, equivalents in other countries, or the like.

In the present specification, the "vaccine" refers to a factor containing antigens or cells and capable of, when administered in vivo, producing antibodies and enhancing cellular immunity, or a substance capable of producing such factors. Antigen means a selected substance and a composition for inducing an immune response to the substance in vertebrates such as human and the like.

In the present specification, the "medicament", "agent", or "factor" (all corresponding to agent in English) is used broadly and may be any substance or other elements (e.g., light, radiation, heat, electricity and other forms of energy) as long as the intended objective can be achieved. Examples of such a substance include, but are not limited to, protein, polypeptide, oligopeptide, peptide, polynucleotide, oligonucleotide, nucleotide, nucleic acid (including for example DNAs such as cDNA and genomic DNA, RNAs such as mRNA), polysaccharide, oligosaccharide, lipid, organic small molecule (e.g., hormone, ligand, information transmitting substance, organic small molecule, molecule synthesized by combinatorial chemistry, small molecule that can be used as medicine (e.g., small molecule ligand and the like), and the like) and a composite molecule thereof.

In the present specification, the "therapy" refers to a cure or improvement of a disease or condition, or suppression of condition. It refers to the prevention of exacerbation, preferably maintaining the current condition, more preferably alleviation, further preferably disappearance of a disease or disorder, in case where such state occurs, including being capable of exerting an improving effect or preventing effect on a disease or one or more conditions accompanying the disease in a patient. A suitable therapy based on preliminary diagnosis may be referred to as "companion therapy" or "tailor made therapy" and a diagnostic agent therefor may be referred to as "companion diagnostic agent".

In the present specification, the "prophylaxis" means preventing the onset of a disease or condition.

In the present specification, the "treatment" means any treatment for a disease or condition and includes treatment and prophylaxis.

In the present specification, the "diagnosis" refers to identifying various parameters associated with a disease, disorder, or condition (e.g., cancer, viral infection and the like) or the like in a test subject to determine the current or future state of such a disease, disorder, or condition. The condition in the body can be investigated by using the method, device, or system of the present disclosure. Such information can be used to select various parameters of the disease, disorder, or condition of a test subject, a formulation to be administered for treatment or prevention or method and the like. In the present specification, the "diagnosis" when narrowly defined refers to diagnosis of the current state, but when broadly defined includes "early diagnosis", "predictive diagnosis", "prediagnosis" and the like. Since the diagnostic method of the present disclosure in principle can utilize what comes out from a body and can be conducted away from a medical practitioner such as a physician, the present disclosure is industrially useful. In order to clarify that the method can be conducted away from a medical practitioner such as a physician, "assisting" "predictive diagnosis, prediagnosis or diagnosis" may be particularly recited.

In the present specification, the "test subject" refers to an entity which is to be subjected to diagnosis, detection, or treatment, and the like in the present disclosure (e.g., an organism such as a human, a cell, blood, serum, etc. which has been taken out from an organism, or the like).

In the present specification, the "sample" refers to any substance obtained from a test subject or the like, and includes, for example, a serum or the like. Those skilled in the art can appropriately select a preferable sample based on the descriptions in the present specification.

In the present specification, the "kit" refers to a unit generally providing portions to be provided (e.g., inspection drug, diagnostic drug, therapeutic drug, antibody, label, manual and the like) into two or more separate sections. This form of a kit is preferred when a composition that should not be provided in a mixed state and is preferably mixed immediately before use for safety or the like is intended to be provided. Preferably, such a kit advantageously comprises an instruction or manual describing how the provided portions (e.g., inspection drug, diagnostic drug, or therapeutic drug are used or how a reagent should be handled. When the kit is used in the present specification as a reagent kit, the kit generally contains an instruction or the like describing how to use an inspection drug, diagnostic drug, therapeutic drug, antibody and the like.

In the present specification, the "instruction" is a document with an explanation of the method of use of the present disclosure for a physician or other users. The instruction has a description of the detection method of the present disclosure, method of use of a diagnostic agent, or a direction for administration of a medicament or the like. Further, an instruction may have a description instructing oral administration or administration to the esophagus (e.g., by injection or the like) as a site of administration. The instruction is prepared in accordance with a format defined by the regulatory agency of the country in which the present disclosure is practiced (e.g., the Ministry of Health, Labor and Welfare in Japan, Food and Drug Administration (FDA) in the U.S. or the like), with an explicit description showing approval by the regulatory agency. The instruction is a socalled package insert and is typically provided in, but not limited to, paper media. The instruction may also be provided in a form such as electronic media (e.g., web sites provided on the Internet or emails).

In the present specification, the "functional equivalent" is any entity that has the same target function but a different structure relative to the original entity of interest. Thus, it is understood that a functional equivalent of a "disease-related factor" or a part thereof includes one that is not the disease-related factor or a part thereof itself, but a mutant or variant (e.g., nucleic acid analog, amino acid sequence variant, etc.) of the disease-related factor or a part thereof, which has the biological action of the disease-related factor or a part thereof, and one that, at the time of action, can be transformed into the disease-related factor or a part thereof itself, or a mutant or variant of the disease-related factor or a part thereof (e.g., nucleic acids encoding the disease-related factor or a part thereof itself or a mutant or variant of the disease-related factor or a part thereof, and vectors, viruses, liposomes, cells, and the like containing the nucleic acids). It is understood that, in the present disclosure, the functional equivalent of a disease-related factor or a part thereof can be used in the same manner as the disease-related factor or a part thereof, even if not particularly mentioned. Functional equivalents can be found by searching databases and the like. In the present specification, the "search" refers to the use of a certain nucleic acid sequence to find other nucleic acid sequences having specific functions and/or properties, either electronically or by biological or other means. Examples of the electronic search include, but are not limited to, BLAST (Altschul et al., J. Mol. Biol. 215:403-410 (1990)), FASTA (Pearson & Lipman, Proc. Natl. Acad. Sci., USA 85:2444-2448 (1988)), Smith and Waterman method (Smith and Waterman, J. Mol. Biol. 147:195-197 (1981)), Needleman and Wunsch method (Needleman and Wunsch, J. Mol. Biol. 48:443-453 (1970)), and the like. Examples of the biological search include, but are not limited to, stringent hybridization, macroarrays in which genomic DNA is attached to a nylon membrane or the like or microarrays in which genomic DNA is attached to a glass plate (microarray assay), PCR, and in situ hybridization. In the present specification, the genes used in the present disclosure are intended to also include corresponding genes identified by such electronic search and biological search.

In the present specification, the "protein", "polypeptide", "oligopeptide" and "peptide" are used herein in the same meaning and refer to an amino acid polymer of any length. The polymer may be straight, branched or cyclic. An amino acid may be a naturally-occurring, non-naturally occurring or altered amino acid. The term may also encompass those assembled into a complex of multiple polypeptide chains. The term also encompasses naturally-occurring or artificially altered amino acid polymers. Examples of such an alteration include disulfide bond formation, glycosylation, lipidation, acetylation, phosphorylation, and any other manipulation or alteration (e.g., conjugation with a labeling component). The definition also encompasses, for example, polypeptides comprising one or more analogs of an amino acid (e.g., including non-naturally occurring amino acids and the like), peptide-like compounds (e.g., peptoids) and other alterations known in the art. In the present specification, the "amino acid" is a general term for organic compounds having an amino group and a carboxyl group. When an antibody according to an embodiment of the present disclosure contains a "specific amino acid sequence", any amino acid in the amino acid sequence may be chemically modified. Also, any amino acid in the amino acid sequence may form a salt or solvate. Also, any amino acid in the amino acid sequence may be L-type or D-type. Even in such cases, it can be said that the protein according to the embodiment of the present disclosure contains the above-mentioned "specific amino acid sequence". Examples of chemical modifications that amino acids contained in proteins undergo in vivo include N-terminal modification (e.g., acetylation, myristoylation, etc.), C-terminal modification (e.g., amidation, glycosylphosphatidylinositol addition, etc.), side chain modification (e.g., phosphorylation, sugar chain addition etc.), and the like. Amino acids can be natural or non-natural as long as they fulfill the purposes of the present disclosure.

In the present specification, the "polynucleotide", "oligonucleotide", and "nucleic acid" are used herein to have the same meaning and refer to a polymer of nucleotides of any length. The terms also encompass "oligonucleotide derivative" or "polynucleotide derivative". The "oligonucleotide derivative" and "polynucleotide derivative" are interchangeably used and refer to an oligonucleotide or polynucleotide comprising a derivative of a nucleotide or having a bond between nucleotides that is different from ordinary bonds. Specific examples of such oligonucleotides include: 2'-O-methyl-ribonucleotide; oligonucleotide derivatives with a phosphodiester bond in an oligonucleotide converted into phosphorothioate bond; oligonucleotide derivatives with a phosphodiester bond in an oligonucleotide converted into an N3'-P5' phosphoramidate bond; oligonucleotide derivatives with a ribose and a phosphodiester bond in an oligonucleotide converted into a peptide nucleic acid bond; oligonucleotide derivatives with a uracil in an oligonucleotide substituted with a C-5 propynyl uracil; oligonucleotide derivatives with a uracil in an oligonucleotide substituted with a C-5 thiazole uracil; oligonucleotide derivatives with a cytosine in an oligonucleotide substituted with a C-5 propynyl cytosine; oligonucleotide derivatives with a cytosine in an oligonucleotide substituted with a phenoxazine-modified cytosine; oligonucleotide derivatives with a ribose in DNA substituted with a 2'-O-propylribose; oligonucleotide derivatives with a ribose in an oligonucleotide substituted with a 2'-methoxyethoxy ribose; and the like. Unless noted otherwise, specific nucleic acid sequences are intended to encompass sequences that are explicitly set forth, as well as their conservatively altered variants (e.g., degenerate codon substitutes) and complementary sequences. Specifically, a degenerate codon substitute can be achieved by making a sequence in which the third position of one or more selected (or all) codons is substituted with a mixed base and/or deoxyinosine residue (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). In the present specification, the "nucleic acid" is also interchangeably used with gene, cDNA, mRNA, oligonucleotide, and polynucleotide. In the present specification, the "nucleotide" may be naturally-occurring or non-naturally-occurring.

In the present specification, the "gene" refers to a factor defining a genetic trait, and "gene" may refer to "polynucleotide", "oligonucleotide", and "nucleic acid".

In the present specification, the "homology" of genes refers to the degree of identity of two or more genetic sequences with respect to one another, and having "homology" generally refers to having a high degree of identity or similarity. Therefore, the identity or similarity of sequences is higher when homology of two genes is higher. Whether two types of genes have homology can be found by direct comparison of sequences or by a hybridization method under stringent conditions for nucleic acids. When two genetic sequences are directly compared, the genes are homologous typically if DNA sequences are at least 50% identical, preferably at least 70% identical, and more preferably at least 80%, 90%, 95%, 96%, 97%, 98%, or 99% identical between the genetic sequences. Thus, in the present specification, the "homolog" or "homologous gene product" refers to a protein in another species, preferably mammal, exerting the same biological function as a protein constituent of a complex which will be further described herein. Such a homolog may be also called "ortholog gene product". It is understood that such homolog, homologous gene product, ortholog gene product, and the like can also be used as long as they meet the purpose of the present disclosure.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.2.28 (published on April 2, 2013 or may be more recent one) of the NCBI. Herein, values for identity generally refer to a value obtained when aligned under the default conditions using BLAST described above. However, when a higher value is obtained by changing a parameter, the highest value is considered the value of identity. When identity is evaluated in a plurality of regions, the highest value thereamong is considered the value of identity. Similarity is a value calculated by taking into consideration a similar amino acid in addition to identity.

In one embodiment of the present disclosure, "several" may be, for example, 8, 7, 6, 5, 4, 3, or 2, and may be not more than any of those values. It is known that polypeptides that have undergone deletion, addition, insertion, or substitution with other amino acids of one or several amino acid residues retain biological activity thereof (Mark et al., Proc Natl Acad Sci USA.1984 Sep; 81(18): 5662-5666., Zoller et al., Nucleic Acids Res. 1982 Oct 25; 10(20):6487-6500., Wang et al., Science. 1984 Jun 29; 224(4656):1431-1433.). It is possible to determine whether or not it is a functional equivalent by measuring the activity by an appropriate method by which deletion, etc., have been made.

In one embodiment of the present disclosure, "90% or more" may be, for example, 90, 95, 96, 97, 98, 99, or 100% or more, and may be within the range of any two of those values. The above-mentioned "homology" may be calculated as the percentage of the number of homologous amino acids between two or more amino acid sequences, according to methods known in the art. Before calculating the percentage, the amino acid sequences of the groups of amino acid sequences to be compared are aligned and gaps are introduced in part(s) of the amino acid sequences if necessary to maximize the percentage of the same amino acids. Methods for alignment, calculation method of percentage, comparison method, and computer programs related thereto are conventionally well known in the technique field (e.g., BLAST, GENETYX etc.). In the present specification, the "homology" can be expressed in terms of values measured by BLAST of NCBI unless otherwise specified. Blastp can be used as the default setting for the algorithm for comparison of amino acid sequences in BLAST. The measurement results are quantified as Positives or Identities.

The substance in the present disclosure may be purified. In the present specification, the "purified" substance or biological factor (e.g., nucleic acid, protein or the like) refers to a substance or a biological agent wherein at least a part of an agent naturally accompanying the substance or biological agent has been removed. Thus, the purity of a biological agent in a purified biological agent is generally higher than the purity in the normal state of the biological agent (i.e., concentrated). The term "purified" as used herein refers to the presence of preferably at least 75% by weight, more preferably at least 85% by weight, still more preferably at least 95% by weight, and most preferably at least 98% by weight of a biological agent of the same type. The substance or biological agent used in the present disclosure is preferably a "purified" substance. An "isolated" substance or biological agent (e.g., nucleic acid, protein or the like) used in the present specification refers to a substance or a biological agent wherein an agent naturally accompanying the substance or biological agent has substantially been removed. The term "isolated" used in the present specification varies according to the purpose thereof and thus is not necessarily shown in purity. Where necessary, it refers to the presence of preferably at least 75% by weight, more preferably at least 85% by weight, still more preferably at least 95% by weight, and most preferably at least 98% by weight of a biological agent of the same type. The substance used in the present disclosure is preferably an "isolated" substance or biological agent.

In the present specification, the "corresponding" amino acid, nucleic acid, or portion refers to an amino acid or a nucleotide which has or is expected to have, in a certain polypeptide molecule or polynucleotide molecule (e.g., polynucleotide encoding spike protein, etc.), similar action as a predetermined amino acid, nucleotide, or portion in a reference polypeptide or a polynucleotide for comparison, and, particularly in the case of enzyme molecules, refers to an amino acid which is present at a similar position in an active site and makes a similar contribution to catalytic activity, and refers to the corresponding part (e.g., heparan sulfate, etc.) in the case of composite molecules. For example, for an antisense molecule, it can be a similar moiety in an ortholog corresponding to a specific moiety of the antisense molecule. A corresponding amino acid can be a specific amino acid subjected to, for example, cysteination, glutathionylation, S-S bond formation, oxidation (e.g., oxidation of methionine side chain), formylation, acetylation, phosphorylation, glycosylation, myristylation or the like. Alternatively, a corresponding amino acid can be an amino acid responsible for dimerization. Such a "corresponding" amino acid or nucleic acid may be a region or a domain over a certain range. Thus, it is referred herein as a "corresponding" region or domain in such a case. Such corresponding region or domain is useful in designing a composite molecule in the present specification.

In the present specification, the "corresponding" gene (e.g., polynucleotide sequence or molecule) refers to a gene (e.g., polynucleotide sequence or molecule) in a certain species which has or is expected to have similar action as a predetermined gene in a reference species for comparison. When there is a plurality of genes having such action, the corresponding gene refers to a gene having the same evolutionary origin. Hence, a gene corresponding to a certain gene may be an ortholog of such a gene. Thus, each specific gene in humans can find a corresponding specific gene in other animals, especially mammals. Such a corresponding gene can be identified by using a technique that is well known in the art. For example, a corresponding gene in a certain animal (e.g., mouse), or gene (e.g., S protein, etc.) to be the standard of the corresponding gene, can be found by search on a database containing the sequence for the animal using the sequence of a reference gene (e.g., gene of humans) as a query sequence.

In the present specification, the "fragment" refers to a polypeptide or polynucleotide with a sequence length of 1 to n-1 with respect to the full length polypeptide or polynucleotide (with length n). The length of a fragment can be appropriately changed in accordance with the objective. Examples of the lower limit of such a length include 3, 4, 5, 6, 7, 8, 9, 10, 15, 20, 25, 30, 40, 50 and more amino acids for a polypeptide. Lengths represented by an integer that is not specifically listed herein (e.g., 11 and the like) also can be suitable as a lower limit. Further, examples of the length include 5, 6, 7, 8, 9, 10, 15, 20, 25, 30, 40, 50, 75, 100, and more nucleotides for a polynucleotide. Lengths represented by an integer that is not specifically listed herein (e.g., 11 and the like) also can be suitable as a lower limit. In the present specification, such a fragment is understood to be within the scope of the present disclosure, for example, when a full length version functions as a marker or a target molecule, as long as the fragment itself also functions as a marker or a target molecule.

According to the present disclosure, the term "activity" refers to the function of a molecule in its broadest sense and can be taken into account in evaluating functional equivalents. Not intended to be limitative, the activity generally includes a biological function, biochemical function, physical function, and chemical function of a molecule. Examples of activity include enzymatic activity, an ability to interact with other molecules, an ability to activate, promote, stabilize, inhibit, suppress, or destabilize functions of other molecules, stability, and an ability to localize at a specific position in a cell. When applicable, the term also relates to a function of a protein complex in the broadest sense.

In the present specification, the "biological function", when referring to a certain gene or a nucleic acid molecule or a polypeptide related thereto, refers to a specific function that the gene, the nucleic acid molecule or the polypeptide may have in a living body. Examples of such a function include, but are not limited to, production of a specific antibody, enzyme activity, impartation of resistance and the like. In the present specification, biological function can be exerted by "biological activity". In the present specification, the "biological activity" refers to the activity possibly possessed by a certain agent (e.g., polynucleotide, protein or the like) in a living body. The biological activity encompasses an activity of exerting a variety of functions (e.g., follicular T cells inducing ability), and also encompasses, for example, an activity of activating or inactivating other molecule by an interaction with a certain molecule. When two agents interact, the biological activity thereof may be a bond between the two molecules and a biological change induced thereby, and for example, two molecules are considered to be bound together if precipitating one molecule using an antibody results in co-precipitation of the other molecule. Observation of such co-precipitation is one example of a determination approach. For example, when a certain agent is an enzyme, the biological activity thereof encompasses enzyme activity thereof. In another example, when a certain agent is a ligand, binding to a receptor corresponding to the ligand is encompassed. Such biological activity can be measured by a technique that is well known in the art. Therefore, the "activity" refers to various measurable indicators that indicate or reveal binding (either directly or indirectly) or affect response (i.e., have a measurable effect in response to some exposure or stimulus), such as the affinity of a compound that binds directly to the polypeptide or polynucleotide of the present disclosure, or the amount of upstream or downstream protein or other similar measure of function after some stimulus or event.

In the present specification, the "expression" of a gene, a polynucleotide, a polypeptide, or the like refers to the gene or the like being subjected to a certain action in vivo to be converted into another form. Preferably, expression refers to a gene, a polynucleotide, or the like being transcribed and translated into a form of a polypeptide. However, transcription to make an mRNA also can be one embodiment of expression. Therefore, the "expression product" in the present specification includes such polypeptide or protein, or mRNA. More preferably, such a polypeptide form can be a form which has undergone post-translation processing.

Those with amino acid sequences with one or more amino acid insertions, substitutions or deletions, or addition to one or both ends in an amino acid sequence can be used as a functional equivalent in the present disclosure. In the present specification, "one or more amino acid insertions, substitutions or deletions, or addition to one or both ends in an amino acid sequence" refers to an alteration with a substitution of a plurality of amino acids or the like to the extent that can occur naturally by a well-known technical method such as site-directed mutagenesis or by natural mutation. A modified amino acid sequence may include, for example, those with insertion, substitutions, deletion, or addition to one or both ends in an amino acid sequence of 1 to 30, preferably 1 to 20, more preferably 1 to 9, further preferably 1 to 5, particularly preferably 1 or 2, amino acids. The altered amino acid sequence may preferably be an amino acid sequence having one or more (preferably 1 or several, or 1, 2, 3, or 4) conservative substitutions in the amino acid sequence, such as the S protein of the virus. The "conservative substitution" means the replacement of one or more amino acid residues with other chemically similar amino acid residue so as not to substantially alter the function of the protein. Examples include the substitution of one hydrophobic residue by another hydrophobic residue, and the substitution of one polar residue by another polar residue having the same charge. Functionally similar amino acids that can make such substitutions are known in the field for each amino acid. Specific examples of non-polar (hydrophobic) amino acids include alanine, valine, isoleucine, leucine, proline, tryptophan, phenylalanine, and methionine. Examples of polar (neutral) amino acids include glycine, serine, threonine, tyrosine, glutamine, asparagine, and cysteine. Examples of positively charged (basic) amino acids include arginine, histidine, and lysine. Examples of negatively charged (acidic) amino acids include aspartic acid and glutamic acid.

### (Preferred embodiments)

Preferred embodiments of the present disclosure are described below. Embodiments provided below are provided to facilitate the understanding of the present disclosure. The scope of the present disclosure should not be limited to the following descriptions. Thus, it is apparent that those skilled in the art can make appropriate modifications within the scope of the present disclosure by referring to the descriptions herein. The following embodiments of the present disclosure can be used alone or in combination.

The present disclosure relates to any medical techniques such as a method for evaluating a disease in a subject, comprising measuring the level or amount of follicular T cells reactive with a disease-related factor of the disease in the subject, a composition, a system, and a program related thereto, a recording medium storing same, and the like.

In the present disclosure, the medical techniques of the present disclosure further includes performing, based on the results of the measurement of the level or amount of follicular T cells reactive with a disease-related factor, at least one selected from the group consisting of evaluation of the history of the aforementioned disease, evaluation of the effectiveness of a prophylactic agent or vaccine against the aforementioned disease, evaluation of the effectiveness of a therapeutic agent against the aforementioned disease, evaluation of defensive ability against re-contraction of the aforementioned disease, pathology evaluation of the aforementioned disease, and evaluation of the risk of contracting the aforementioned disease.

In one embodiment, these evaluations include, but are not limited to, for example, evaluation of infection history, evaluation of effectiveness of vaccine (presence or absence of Tfh induction), evaluation of re-contraction defensive ability, evaluation of effectiveness of cancer immunodrug (immunocheckpoint and the like), risk evaluation of autoimmune disease associated with autoantibody (predisease period), pathology evaluation, and the like.

In one aspect, the present disclosure provides a method for producing follicular T cells specific to a disease, including a step of identifying a disease-related factor or a part thereof having the ability to induce follicular T cells specific to the disease-related factor of the disease, a step of inducing follicular T cells specific to the disease by using the disease-related factor or a part thereof, and a step of obtaining follicular T cells specific to the disease.

In one aspect, the present disclosure provides a method for screening for follicular T cells reactive with a disease, including A) a step of providing a population of follicular T cells, B) a step of contacting the population of follicular T cells with the disease-related factor, C) a step of measuring the reactivity of the follicular T cells with the disease-related factor, or a part thereof, or a functional equivalent thereof, and D) a step of selecting, from the population of follicular T cells, follicular T cells exhibiting reactivity of not less than a given value with the disease-related factor.

In one embodiment, A) the step of providing a population of follicular T cells can be performed by collection of blood samples from a test subject, biopsy, or the like.

In one embodiment, B) the step of contacting the population of follicular T cells with the disease-related factor or a part thereof or a functional equivalent thereof can be performed by, for example, adding a disease-related factor or a part thereof or a functional equivalent thereof to a culture medium in which a population of follicular T cells is cultured, followed by culture, incubation or standing still as necessary.

In one embodiment, C) the step of measuring the reactivity of the follicular T cells with the disease-related factor, or a part thereof, or a functional equivalent thereof can be performed by ELISPOT, flow cytometry, peripheral blood mononuclear cell (PBMC) restimulation assay, next generation sequencer/genetic testing (repertoire analysis), immunostaining (histopathological examination), or in situ analysis.

In one embodiment, the given value may be the mean or median value of the reactivity of follicular T cells with a disease-related factor or a part thereof or a functional equivalent thereof in a test subject not affected with the disease.

### <Diagnosis>

In one aspect, the present disclosure provides a method for inspecting or diagnosing a disease, including measuring the level or amount of follicular T cells reactive with a disease-related factor.

In another aspect, the present disclosure provides a method for testing a disease in a subject, comprising measuring the level or amount of follicular T cells reactive with a disease-related factor of the disease in the subject, and comparing the level or amount with a given standard. In some embodiment, the given standard may be the mean or median value of follicular T cells in a healthy subject. In other embodiment, the given standard may be a reference value in a paper.

In another aspect, the present disclosure provides a method for evaluation of infection history of a disease, a method for evaluation of vaccine efficacy and re-contraction defensive ability against a disease, a method for evaluation of effectiveness of cancer immunodrugs (immune checkpoint and the like) on a disease, a method for risk evaluation of autoimmune disease associated with autoantibody (predisease period), and pathology evaluation thereof. These methods include measuring the level or amount of follicular T cells reactive with a disease-related factor.

In some embodiment, the aforementioned measurement may be performed by ELISPOT, flow cytometry, peripheral blood mononuclear cell (PBMC) restimulation assay, next generation sequencer/genetic testing (repertoire analysis), immunostaining (histopathological examination), or in situ analysis.

In one embodiment, the level or amount of follicular T cells reactive with a disease-related factor can be measured, for example, as follows.

Peripheral blood derived from a subject can be contacted with a probe containing an infection source-specific epitope, and the amount of follicular T cells in the peripheral blood that bind to the probe can be measured by ELISPOT or flow cytometry. ELISPOT and flow cytometry are exemplified in other part of the present specification.

The ELISPOT used in the present disclosure can be used. The ELISPOT assay used here uses a plate coated with an antibody that can detect IL-21 produced by follicular T cells. Peripheral blood mononuclear cells (PBMC) are added to the well and stimulated with an antigen derived from the infection source for 36 hr. Secreted IL-21 binds to capture antibody around the producing cells. After cells are removed by washing, cytokine antibody for detection is added and the spots are detected. As a negative control, the assay is performed without stimulation with an antigen derived from the infection source.

In the present disclosure, flow cytometry can be utilized in which peripheral blood mononuclear cells (PBMC) are stimulated for 36 hr using an antigen derived from an infection source. FSC SSC gating, PI negative gating, and CD3 positive gating are performed, and FCM analysis of double positive fractions of CD4, CXCR5, or triple positive fractions of HLA tetramers bound to infection source-derived antigens is performed. A sample without stimulation by infection source-derived antigens is used as a negative control for comparison.

In a specific embodiment, a disease can be inspected or diagnosed by comparing the level or amount of measured follicular T cells reactive with a disease-related factor with given values (e.g., measured value of negative control and measured value of healthy subject).

In another aspect, the present disclosure provides a method for evaluation of infection history of a disease, including measuring the level or amount of follicular T cells reactive with a disease-related factor. While antibodies are frequently used to evaluate infection history, it is beneficial to identify infection source-specific immune cells for infection history because, compared to antibodies, specific immune cells are maintained for a longer period of time.

In one embodiment of the evaluation of infection history of a disease, the level or amount of follicular T cells reactive with a disease-related factor can be measured, for example, as follows.

Peripheral blood derived from a subject can be contacted with a probe containing an infection source-specific epitope, and the amount of follicular T cells in the peripheral blood that bind to the probe can be measured by ELISPOT or flow cytometry. ELISPOT and flow cytometry are exemplified in other part of the present specification.

In a specific embodiment, evaluation of infection history of a disease can be performed by comparing the level or amount of measured follicular T cells reactive with a disease-related factor with given values (e.g., measured value of negative control and measured value of healthy subject).

In another aspect, the present disclosure provides a method for evaluation of vaccine efficacy and re-contraction defensive ability against a disease, including measuring the level or amount of follicular T cells reactive with a disease-related factor. Infection source-specific immune cells can be used to evaluate the defensive ability of immune cells because, compared to antibodies, they are maintained for a longer period of time and can respond rapidly upon re-contraction.

In one embodiment of the measurement of the level or amount of follicular T cells reactive with a disease-related factor, the level or amount of follicular T cells reactive with a disease-related factor can be measured, for example, as follows.

Peripheral blood derived from a subject can be contacted with a probe containing an infection source-specific epitope, and the amount of follicular T cells in the peripheral blood that bind to the probe can be measured by ELISPOT or flow cytometry. ELISPOT and flow cytometry are exemplified in other part of the present specification.

In a specific embodiment, evaluation of vaccine efficacy and re-contraction defensive ability against a disease can be performed by comparing the level or amount of measured follicular T cells reactive with a disease-related factor with given values (e.g., measured value of negative control and measured value of healthy subject).

In some aspect, the present disclosure provides a method for evaluation of effectiveness of cancer immunodrugs (immune checkpoint and the like) on a disease, including measuring the level or amount of follicular T cells reactive with a cancer-related factor.

In one embodiment of the measurement of the level or amount of follicular T cells reactive with a cancer-related factor, the level or amount of follicular T cells reactive with a disease-related factor can be measured, for example, as follows.

Peripheral blood derived from a subject can be contacted with a probe containing an infection source-specific epitope, and the amount of follicular T cells in the peripheral blood that bind to the probe can be measured by ELISPOT or flow cytometry. ELISPOT and flow cytometry are exemplified in other part of the present specification.

In a specific embodiment, evaluation of effectiveness of cancer immunodrugs (immune checkpoint and the like) on a disease can be performed by comparing the level or amount of measured follicular T cells reactive with a disease-related factor with given values (e.g., measured value of negative control and measured value of healthy subject).

In a specific aspect, the present disclosure provides a method for risk evaluation of autoimmune disease associated with autoantibody (predisease period) and pathology evaluation thereof, including measuring the level or amount of follicular T cells reactive with an autoimmune disease-related factor. There are many autoimmune diseases associated with autoantibodies, among which in diseases where autoantibodies are considered pathogenic (e.g., ANCA-associated vasculitis), the presence or absence/increase or decrease of follicular T cells that interact with B cells that produce autoantibodies may lead to the onset or aggravation/relapse of the disease as the B cells mature.

In one embodiment of the measurement of the level or amount of follicular T cells reactive with an autoimmune disease-related factor, the level or amount of follicular T cells reactive with a disease-related factor can be measured, for example, as follows.

Peripheral blood derived from a subject can be contacted with a probe containing an infection source-specific epitope, and the amount of follicular T cells in the peripheral blood that bind to the probe can be measured by ELISPOT or flow cytometry. ELISPOT and flow cytometry are exemplified in other part of the present specification.

In a specific embodiment, risk evaluation of autoimmune disease associated with autoantibody (predisease period), and pathology evaluation thereof can be performed by comparing the measured level or amount of follicular T cells reactive with a disease-related factor with given values (e.g., measured value of negative control and measured value of healthy subject).

In specific embodiments, diagnosis or testing can be performed by measuring the aforementioned various amounts or levels described above. The following specific examples of diagnoses and tests can be envisioned, which can be applied to autoimmune diseases, other diseases (neurodegenerative diseases, etc.), and those with altered immunity repertoire.
1. evaluation of infection history
2. evaluation of vaccine efficacy (presence or absence of Tfh induction)
3. evaluation of re-contraction defensive ability
4. effectiveness evaluation of cancer immunodrugs (immune checkpoint and the like)
5. risk evaluation of autoimmune disease associated with autoantibody (predisease period), and pathology evaluation thereof

In one embodiment, the present disclosure can provide an evaluation technique for infection history.

While antibodies are frequently used to evaluate infection history, it is beneficial to identify infection source-specific immune cells for infection history because, compared to antibodies, specific immune cells are maintained for a longer period of time. T-cell detection method using probes containing infection source-specific epitopes is beneficial as a simple method to detect immune cells established and maintained after infection. The presence or absence of probe-reactive T cells can be easily detected by a system using ELISPOT or flow cytometry using peripheral blood mononuclear cells (PBMCs). If probe-reactive T cells are detected, infection in the past can be determined. In addition, if more probe-reactive T cells are detected compared to healthy subjects, infection in the past can be determined.

### Application to infection diagnosis

It is known that a specific response by immune cells is established within several days to about two weeks after infection and is maintained for a long period thereafter. Therefore, using selected epitopes, it is possible to determine the infection history from the immune status after infection, after the pathogen of the infectious disease has been cleared from the body, or even when suppressed to an undetectable level.

### Specific steps for evaluation of infection history

Using epitopes related to specific infectious diseases and specimens from patients or recoverers that are considered to have acquired immunity against specific infectious diseases, the reactivity of follicular T cells is confirmed and epitopes that induce follicular T cells are identified. Here, in order to ensure that the response is caused by immune cells specific to a specific infectious disease, the sequence conservation of the epitope with other infectious disease antigens and disease-related antigens is evaluated, desirably cross-reactivity is evaluated using actual specimens or epitope-reactive T cells, and epitopes specific to the particular infectious disease in interest are selected. This ensures that only those with a history of infection with a specific infectious disease will react to the selected epitope, and the steps can be used to evaluate infection history.

In one embodiment, the present disclosure provides evaluation of vaccine efficacy and evaluation of re-contraction defensive ability.

Infection source-specific immune cells can be used to evaluate the defensive ability of immune cells because, compared to antibodies, they are maintained for a longer period of time and can respond rapidly upon re-contraction. Among the many T cell fractions, follicular T cells are an important fraction for inducing and strengthening humoral immunity (affinity maturation). In particular, subjects with T cells that react with a probe containing the infection source epitope that induces follicular T cells are judged to have stronger or qualitatively superior infection defensive ability. Specific responses by immune cells react only with vaccine antigens. Thus, by observing the reactivity with the epitope, it is possible to determine the presence or absence and strength of immunity against the vaccine target disease after vaccination.

### Specific steps for evaluation of vaccine efficacy and re-contraction defensive ability

Among the T cells epitopes contained in the vaccine, epitopes that induce follicular T cells are identified by confirming the reactivity of follicular T cells by using samples from patients or recoverers of vaccine target disease that are considered to have acquired immunity against the vaccine target disease. Here, in order to ensure that the response is caused by immune cells specific to the vaccine antigen, the sequence conservation of the epitope with other infectious disease antigens and disease-related antigens is evaluated, desirably cross-reactivity is evaluated using actual specimens or epitope-reactive T cells, and follicular T cell epitopes specific to the vaccine are selected. As a result, it is ensured that reaction with the selected epitope occurs only when an infection history with the vaccine target disease or vaccine inoculation exists, and this can be used in the evaluation of infection history. Since specific responses by immune cells react only to vaccine antigens, the presence or absence and strength of immunity against the vaccine target disease can be determined after vaccination by observing the reactivity with the epitope. This makes it possible to evaluate vaccine efficacy and re-contraction defensive ability.

As described above, it is considered effective to examine probe-reactive cells against PBMCs as an immune evaluation method established after infection or vaccination. As the evaluation method, ELISPOT or flow cytometry can be used in the same manner as above. These methods can be used to detect whether and at what proportion the T cells that responded to the epitope probe were in fact follicular T cells by changing the detected cytokines or combining surface antigen probes, and an experimental system can be designed according to detection sensitivity and judgment criteria.

Follicular T cells are characterized by surface antigens and cytokines they release. Therefore, ELISPOT can confirm whether the reacted T cells are follicular T cells by detecting cytokines specific to follicular T cells, such as IL-21, which are released from the cells after reaction with the epitope, and flow cytometry can confirm whether the reacted T cells are follicular T cells by capturing cell surface antigens characteristic of follicular T cells such as CXCR5. In addition, the predominance of follicular T cells in T cells induced by each epitope can be evaluated by capturing together other T cell-specific cytokines and surface antigens. The cytokines released can be compared with those before epitope stimulation or with positive/negative controls prepared in advance to evaluate the induction ability of the epitope-specific follicular T cells and further, based on these values, to evaluate the immunity established after infection or vaccination.

In the present disclosure, evaluation of re-contraction defensive ability can be performed in another embodiment.

It is possible to understand the strength of immunity against infectious diseases of interest that are maintained as immune memory, by detecting the responses of follicular T cells specific to vaccine antigens or infectious disease antigens by the ELISPOT and flow cytometry described above. Strictly speaking, large-scale epidemiological studies may be required, but a relationship between the measured level or amount of follicular T cell response and defense against infection or prevention of aggravation (evaluation of the presence or absence of infection of subjects in a given period and evaluation of correlation between the number of people with infection and the measured level or amount of follicular T-cell response in the subject) can be used as an indicator of the re-contraction defensive ability.

In one embodiment, the present disclosure provides effectiveness evaluation of cancer immunodrugs (immune checkpoint and the like).

Higher antitumor properties are expected if a humoral immune response to cancer antigens is induced. Therefore, subjects in whom more follicular T cells, which induce an antitumor humoral immune response, are detected are more likely to have their antitumor immunity activated, and drugs that enhance antitumor immunity of the subjects, such as immune checkpoint inhibitors, are considered to be more effective.

Tumor-specific T cell in individual patients is extremely low in the amount in the periphery and very difficult to detect ex vivo. However, a method of tumor-specific T cell enrichment, such as co-culturing with the patient's tumor, allows for detection of tumor responses. Since the response of cytotoxic (CD8+) T cells is assumed to be stronger, CD8+ T cells are removed from the periphery, and CD4+ T cells and antigen-presenting cells (such as immortalized B cells) are co-cultured with the patient's tumor or patient-derived tumor cell line to facilitate the observation of tumor-specific follicular T cell responses, and the antitumor immunity of the subjects can be evaluated based on the measured level or amount of follicular T cell responses. More preferably, ex vivo evaluation of follicular T cells existing in the local tumor or tumor microenvironment can be performed using a surgical or biopsy sample performed before administration of a cancer immunodrug. It is considered that a more accurate effectiveness evaluation can be made by evaluating not only the number of follicular T cells and the level of cytokines, but together with chemokines such as CXCL13, which are considered to induce follicular T cells in the future, and further, IL-7 which is associated with tertiary lymphoid structures suggested to have a good correlation with prognosis and conversely, the levels of inhibitory cytokines such as IL-10, which are suggested to be negatively correlated.

By evaluating the reactivity of T cells obtained from tumor biopsy specimens or peripheral blood with epitope probes, it is considered to be possible to evaluate the degree of antitumor immune activity in subjects and to help select treatment methods. In addition, by combining epitope probes and flow cytometry to evaluate the activation and exhaustion of epitope-specific follicular T cells (tumor-specific follicular T cells) during drug administration, it is expected to examine whether antitumor immune activity is maintained. This is useful in determining whether immune checkpoint inhibitors will continue to be effective or whether treatment should be discontinued and another treatment method should be sought.

The activation of individual follicular T cells is evaluated, for example, by the expression of IL-21 and ICOS, and the production capacity of IL-21. The degree of exhaustion is generally evaluated using PD-1, but other exhaustion markers such as LAG3, TIM3, and TIGIT can also be used.

The method for evaluating the degree of T cell exhaustion used in the present specification can be performed using any technique known in the art. For example, the degree of exhaustion of T cells can be evaluated by the percentage of PD-1 positive cells (in the cell fraction of interest) by flow cytometry and equivalent tests. The degree of exhaustion can vary from 20% to 100%. Although not limitatively, the degree of exhaustion can be measured based on the method described in "PD-1BlockadeTherapy Promotes Novel Infiltration of Tumor-Specific T-CellClonotypes" https://papers.ssrn.com/sol3/papers.cfm?abstract_id=3796528) .

In one embodiment, the present disclosure provides risk evaluation of autoimmune disease associated with autoantibody (predisease period), and pathology evaluation thereof.

There are many autoimmune diseases associated with autoantibodies, among which in diseases where autoantibodies are considered pathogenic (e.g., ANCA-associated vasculitis), the presence or absence/increase or decrease of follicular T cells that interact with B cells that produce autoantibodies may lead to the onset or aggravation/relapse of the disease as the B cells mature. Therefore, it is considered that disease risk (before onset) and disease activity can be evaluated by detecting follicular T cells that present epitopes derived from disease-specific self-antigens.

Evaluation is possible by detecting follicular T cells presenting epitopes derived from autoantibodies that cause autoimmune diseases. The test method involves examining probe-reactive cells in PBMC collected from subjects, by using ELISPOT or flow cytometry.

If an increase in follicular T cells is confirmed compared to the negative control, it can be determined that the disease risk (before onset) or disease activity is high.

### (Production method of antigen)

The peptides and the like of the present disclosure can be produced using various methods such as chemical synthesis, genetic engineering, and production using microorganisms.

Those skilled in the art will understand from this disclosure and their knowledge in the art that there are a variety of ways to generate antigens. In general, antigens can be generated either in vitro or in vivo. An antigen may be produced in vitro as a peptide or polypeptide, which may then be formulated into a personalized neoplastic vaccine or immunogenic composition and administered to a subject. As described in further detail in the present specification, such in vitro production can be carried out by various methods known to those skilled in the art, for example, peptide synthesis or expression of peptides/polypeptides from DNA or RNA molecules in any of a variety of bacterial, eukaryotic, or viral recombinant expression systems, followed by purification of the expressed peptides/polypeptides. Alternatively, the antigen may be produced in vivo by introducing an antigenencoding molecule (e.g., DNA, RNA, viral expression system, etc.) into a subject, followed by expression of the encoded antigen. Methods of in vitro and in vivo production of antigens are also further described herein when they relate to methods of delivery of pharmaceutical compositions and combination therapies.

### In vitro synthesis of peptide/polypeptide

Proteins or peptides can be made by any technique known to those of skill in the art, including expression of proteins, polypeptides or peptides by standard molecular biology techniques, proteins from natural supply source, translation in vitro from natural sources, or isolation of peptides, or chemical synthesis of proteins or peptides. Nucleotide and protein, polypeptide and peptide sequences corresponding to various genes have been previously disclosed and can be consulted in computerized databases known to those skilled in the art. One such database is the National Center for Biotechnology Information's Genbank and GenPept databases located on the National Institutes of Health website. Coding regions of known genes can be amplified and/or expressed using the techniques disclosed in the present specification or as known to those of skill in the art. Alternatively, various commercially available protein, polypeptide and peptide preparations are known to those skilled in the art.

Peptides can be easily chemically synthesized using reagents that do not contain contaminating bacteria or animal substances (Merrifield RB: "Solid phase peptide synthesis I. Synthesis of tetrapeptides". J. Am. Chem. Soc. 85:2149-54, 1963). In specific embodiments, the preparation of antigen peptides is performed by (1) parallel solid-phase synthesis on a multichannel instrument using homogeneous synthesis and cleavage conditions; (2) purification by column stripping on a RP-HPLC column; and rewashing between peptides, but without exchange; followed by (3) analysis by the most informative and limited set of assays. For an individual patient set of peptides, a good manufacturing practice (GMP) footprint can be defined, thus requiring a suite switching procedure only between different patient peptide syntheses.

Alternatively, antigen peptides may be produced in vitro using nucleic acids (e.g., polynucleotides) encoding antigen peptides of the present disclosure. Polynucleotides may be, for example, DNA, cDNA, PNA, CNA, RNA, either single-stranded and/or double-stranded, or in natural or stabilized form, a polynucleotide having a phosphorothioate backbone, etc., or a combination thereof, and may or may not contain introns, as long as it encodes a peptide. In one embodiment, peptides are produced using in vitro translation. There are many exemplary systems available to those skilled in the art (e.g., Retic Lysate IVT Kit, Life Technologies, Waltham, MA).

Expression vectors capable of expressing polypeptides can also be prepared. Expression vectors for various cell types are well known in the art and can be selected without undue experimentation. Generally, the DNA is inserted into an expression vector, such as a plasmid, in the proper orientation and correct reading frame for expression. If desired, the DNA may be ligated to appropriate transcriptional and translational regulatory control nucleotide sequences recognized by the desired host (e.g., bacteria). However, such control is generally available in expression vectors. Next, the vector is introduced to cloning host bacteria using standard techniques (e.g., see Sambrook et al.(1989) Molecular Cloning, A Laboratory Manual, Cold Spring Harbor Laboratory, Cold Spring Harbor, N.Y.).

Expression vectors containing the isolated polynucleotides, as well as host cells containing the expression vectors are also contemplated. Antigen peptides may be provided in the form of RNA or cDNA molecules that encode the desired antigen peptide. One or more antigen peptides of this disclosure may be provided by a single expression vector.

In one aspect, the present disclosure provides a method for testing items relating to a disease in a subject, including measuring the level or amount of follicular T cells reactive with a disease-related factor of the disease in the subject, and comparing the level or amount with a predetermined standard.

In some embodiment, the standard relates to at least one selected from the group consisting of history of disease, effectiveness of prophylactic agent or vaccine against a disease, effectiveness of therapeutic agents against a disease, defensive ability against re-contraction of a disease, pathology of a disease, and risk of contracting a disease.

In some embodiment, the standard relates to the aforementioned disease in the aforementioned subject, and a mean or median value of the level or amount of follicular T cells reactive with a disease-related factor of a disease in a healthy human can be used as the standard. If higher than the standard, the test subject may have the disease.

In some embodiment, the standard relates to the infection history of the aforementioned disease in the aforementioned subject, and the presence or absence of infection source-specific immune cell can be used as the standard. When an infection source-specific immune cell is found, infection history may be present.

In some embodiment, the standard relates to the vaccine effectiveness/re-contraction defensive ability on the aforementioned disease in the aforementioned subject, and the presence or absence of infection source-specific immune cell can be used as the standard. When infection source-specific immune cell is found, it is determined that the vaccine is effective and re-contraction defensive ability is present.

In some embodiment, the standard relates to the effectiveness of a cancer immunodrug on the aforementioned disease in the aforementioned subject, and the level of immune cell having an antitumor (epitope) activity can be used as the standard, and the activation and degree of exhaustion of epitope-specific follicular T cells (tumor-specific follicular T cells) can also be used as the standard. When the activity of epitope-specific follicular T cells has increased, it is determined that the cancer immunodrug is effective.

In some embodiment, the disease-related factor is an autoimmune disease-related factor, the measurement includes measurement of the level or amount of follicular T cells reactive with the autoimmune disease-related factor, and the standard relates to the risk and pathology of an autoimmune disease associated with an autoantibody of the aforementioned subject. The presence or absence of autoimmune disease-related factor-specific immune cell can be used as the standard. When an autoimmune disease-related factor-specific immune cell is present, autoimmune disease may be present.

In one aspect, the present disclosure provides a system for testing an item relating to a disease in a subject, including a reagent or a device for measuring the level, spatial distribution, or amount of follicular T cells reactive with a disease-related factor of the disease in the subject, and a comparison unit that compares the measurement results by the reagent or device with a predetermined standard.

In one aspect, the present disclosure provides a method for testing an item relating to a disease in a subject, including a step of providing information relating to the level, spatial distribution, or amount of follicular T cells reactive with a disease-related factor of the disease in the subject, and a step of comparing the information relating to the level, spatial distribution, or amount of follicular T cells with a predetermined standard.

In one aspect, the present disclosure provides a program containing computer readable code for implementing, on a computer, a method for testing an item relating to a disease in a subject, including a step of providing information relating to the level, spatial distribution, or amount of follicular T cells reactive with a disease-related factor of the disease in the subject, and a step of comparing the information relating to the level, spatial distribution, or amount of follicular T cells with a predetermined standard.

In one aspect, the present disclosure provides a computer readable recording medium storing the above-mentioned program.

### <Probe>

In one aspect, the present disclosure provides a polypeptide for presenting a disease-related factor or a part thereof having the ability to induce follicular T cells specific to the disease-related factor. Such polypeptide can be used for inspection or diagnosis in the present disclosure, and is also called a probe.

### <Probe: modified method>

In another aspect, the present disclosure provides an antibody that specifically binds to TCR that interacts with a disease-related factor or a part thereof having the ability to induce follicular T cells specific to the disease-related factor. Such antibody can be used for the inspection or diagnosis of the present disclosure and is also called a probe.

### (Treatment and prophylaxis, medicament, and vaccine)

The present disclosure can be provided as a medicament. The components contained in the medicament or pharmaceutical composition can be referred to as a medicine and the like. The medicament of the present disclosure can be provided as a vaccine (including cell vaccine) or a probe.

In one aspect, the present disclosure provides a pharmaceutical composition for treating or preventing a disease, containing a disease-related factor or a part thereof, and having the ability to induce follicular T cells specific to a disease-related factor.

In another aspect, the present disclosure provides a vaccine for a disease, containing a disease-related factor or a part thereof, and having the ability to induce follicular T cells specific to a disease-related factor.

In another aspect, the present disclosure provides a pharmaceutical composition for treating or preventing a disease, containing follicular T cells specific to a disease-related factor.

In some aspect, the present disclosure provides a cell vaccine for a disease, containing follicular T cells specific to a disease-related factor.

In one embodiment, the disease can be an infectious disease or cancer. In another embodiment, the disease can be a viral infection. In certain embodiments, the compositions of the present disclosure can induce follicular T cells in an HLA type-specific manner. In some embodiments, a disease-related factor can be an amino acid, a nucleic acid, a virus, or a cell. In other embodiments, a disease-related factor may be a factor that directly or indirectly causes the disease, or may be a factor that results from the development of the disease. For example, when the disease is a viral disease, the disease-related factor can be a virus that causes a viral disease. In some embodiments, the disease-related factor or a part thereof contains up to 20 amino acids. In certain embodiments, the follicular T cells of the present disclosure can be public follicular T cells.

In some aspect, the present disclosure relates to a TCR-T cell therapy.

In some aspect, for example, the present disclosure provides a composition containing follicular T cells specific to a disease-related factor and for enhancing immunity against the disease.

In one aspect, the present disclosure provides a pharmaceutical composition for treating or preventing a disease, containing a disease-related factor or a part thereof or a functional equivalent thereof, and having the ability to induce follicular T cells reactive with the disease-related factor.

In one aspect, the present disclosure provides a vaccine for a disease, containing a disease-related factor or a part thereof or a functional equivalent thereof, and having the ability to induce follicular T cells reactive with the aforementioned disease-related factor. In one embodiment, the aforementioned composition or vaccine may preferentially induce follicular T cells, or induce follicular T cells while inducing cells other than follicular T cells.

In one aspect, the present disclosure provides a pharmaceutical composition containing follicular T cells reactive with a disease-related factor, and for treating or preventing a disease.

In one aspect, the present disclosure provides a cell vaccine for a disease, containing follicular T cells reactive with a disease-related factor.

In one embodiment, the follicular T cells reactive with the aforementioned disease-related factor in the present disclosure is reactive with at least a disease-related factor. In other embodiment, the aforementioned disease is an infectious disease or cancer, and the aforementioned disease is a viral infectious disease in a specific embodiment. In another embodiment, the aforementioned composition induces follicular T cells in a HLA type-specific manner. In some embodiment, the follicular T cells are public follicular T cells.

When the present disclosure is administered, in the case of oral administration, it may be formulated into various forms such as tablets, granules, fine granules, powders, capsules, and the like. Additives commonly used in formulations such as binders, encapsulating agents, excipients, lubricants, disintegrants and wetting agents may be contained. In addition to these, preparations for oral administration may be formulated as liquids such as oral solutions, suspensions, emulsions, syrups, and the like, or as dry formulations that are redissolved at the time of use.

For parenteral administration, the formulation may be formulated in unit-dose ampoules or multi-dose containers or tubes, and may also contain additives such as stabilizers, buffers, preservatives, and tonicity agents. In addition, the preparation for parenteral administration may be formulated into a powder that can be redissolved in a suitable carrier (sterile water, etc.) at the time of use.

The "active ingredient" in the present specification refers to an ingredient contained in the composition of the present disclosure in an amount necessary to achieve the desired effect of treatment, prevention, or inhibition of progression. Other ingredients may also be included as long as the effectiveness is not impaired below the desired level. In addition, the medicaments, compositions, etc. of the present disclosure may be formulated. In addition, the administration route of the medicament, composition, etc. of the present disclosure may be either oral or parenteral, and can be appropriately set according to the form of the preparation.

As the pharmaceutically acceptable salt, for example, inorganic acid salts (such as hydrochlorides, hydrobromides, phosphates, and sulfates), or salts of organic acids (such as acetates, propionates, malonates, and benzoate) may be used.

Pharmaceutically acceptable salts in pharmaceutical compositions further include liquids (water, saline, glycerol and ethanol). Additionally, auxiliary agents (wetting agents, emulsifying agents or pH buffering agents) may be present in the composition. The carrier allows the pharmaceutical composition to be formulated as tablets, pills, sugar-coated tablets, solutions, gels, syrups, slurries, or suspensions for ingestion by a test subject.

The scope of the present disclosure is compositions present in some forms of administration; such forms include, but are not limited to, suitable forms for parenteral administration, such as injection or infusion (e.g., bolus or continuous infusion). When the products are injected or infused, they may take the form of suspensions, solutions or emulsions in oily or aqueous vehicles, and may contain formulatory agents (suspending agents, stabilizing agents, and/or dispersing agents). Alternatively, it may be in dry form, for reconstitution prior to use with a suitable sterile liquid.

Once formulated, the compositions of the present disclosure can be administered directly to a test subject. In one embodiment, the composition is adapted for administration to a mammal (e.g., a human test subject).

The pharmaceutical compositions of the present disclosure can be administered by any number of routes (including but not limited to oral, intravenous, intramuscular, intraarterial, intramedullary, intraperitoneal, intrathecal, intraventricular, transdermal, transdermal, topical, subcutaneous, intranasal, enteral, sublingual, intravaginal, and rectal routes). A hypospray can also be used to administer the pharmaceutical compositions of the present disclosure. Typically, the therapeutic compositions are prepared as injectables, either as liquid solutions or suspensions. Solid forms suitable for solution in, or suspension in, liquid vehicles prior to injection can be prepared.

Direct delivery of the composition is generally accomplished by subcutaneous, intraperitoneal, intravenous, intramuscular injection, or delivered to the interstitial space of the tissue. The composition can be administered to the lesion. The administration treatment can be a single dose regimen or a multiple dose regimen. The medication provides instructions related to the frequency of administration (e.g., whether it should be delivered daily, weekly, monthly, etc.). The number and dosage may also depend on the severity of the symptoms.

The composition of the present disclosure can be prepared in a variety of forms. For example, the composition can be prepared as an injectable, either as a liquid solution or suspension. Solid forms suitable for solution in, or suspension in, liquid vehicles prior to injection can be prepared (lyophilized compositions (such as Synagis (trademark) and Herceptin (trademark)) for reconstitution with sterile water containing preservatives). The composition may be prepared for topical administration, for example, as an ointment, cream or powder. The compositions can be prepared for oral administration, for example, as tablets or capsules, sprays, or syrups (optionally flavored). The composition may be prepared for pulmonary administration as an inhaler, for example, using a fine powder or spray. The composition may be prepared as a suppository or pessary. The compositions may be prepared for nasal, aural, or ocular administration, for example, as drops. The composition may be in the form of a kit, designed such that a combined composition is reconstituted immediately prior to administration to a test subject. For example, lyophilized antibodies can be provided in the form of a kit with sterile water or sterile buffer.

In another aspect, the disclosure can be provided as a vaccine. In some aspects, the present disclosure provides methods of preventing diseases and the like by using vaccines and the like.

In some embodiments, the pharmaceutical compositions of the present disclosure can be vaccine compositions for administration to humans to enhance immunity. The vaccine composition may further include one or more adjuvants. Examples of adjuvants that may be included in vaccine compositions are provided in the present disclosure below. In some embodiments, vaccine compositions can include cellular vaccines. In exemplary embodiments, examples of suitable adjuvants include:
(1) oil-in-water emulsion formulations (with or without muramyl polypeptides or other specific immunostimulatory substances such as bacterial cell wall components), such as MF59 (trademark) (containing 5% squalene, 0.5% Tween 80, and 0.5% sorbitan trioleate) and SAF (containing 10% squalene, 0.4% Tween 80, 5% Pluronic (trademark) block polymer L121, and threonyl (thr-)MDP), (2) saponin adjuvant, for example, QS21, STIMULON (trademark) (CambridgeBiosciente, Workester, MA), ABISCO (registered trademark) (Isconova, Sweden), or Iscomatrux Signs) (registered trademark) (CommonwealthSerumLaboratories, Australia), (3) complete Freund's adjuvant (CFA) and incomplete Freund's adjuvant (IFA), (4) oligonucleotides containing a CpG motif in which the cytosine is unmethylated, i.e., containing at least one CG dinucleotide (e.g., Krieg, Vaccine (2000)19:618 - 622; Krieg, CurrOpinMolTher (2001)3:15 - 24; WO98/40100, WO98/55495, WO98/37919, and WO98/52581), and (5) metal salts, including aluminum salts (alum, aluminum phosphate, aluminum hydroxide, etc.); (6) saponins and oil-in-water emulsions (e.g. WO99/11241) .

In one aspect, a disease can be treated or prevented by administering the pharmaceutical composition for treating or preventing a disease in the present disclosure containing a disease-related factor or a part thereof or a functional equivalent thereof, which has the ability to induce follicular T cells reactive with a disease-related factor to a test subject affected with or at risk of being affected with the disease.

The treatment or prevention of the present disclosure designs vaccines by a method in which a substance containing the identified follicular T cell epitope, or its functional equivalent is 1. combined with other vaccine antigens/nucleic acids for purposes such as B cell induction, or 2. expressed independently of other antigens in viral vectors or VLPs, or 3. combined with substances that aim to induce immunity other than B cells + Tfh, such as cytotoxic T cell epitopes. Vaccine effectiveness is determined by optimizing the combination of the above, the number and balance of antigens presented, and the adjuvant to be combined using HLA-humanized mice. Although drug efficacy can be evaluated to some extent using animal models, it is difficult to fully reproduce human immunity, which is a difficult point when targeting T cells. In the case of tumors, the administration method is most likely locally to the tumor, but is not limited thereto.

In one aspect, the treatment or prophylaxis of the present disclosure can be performed by
1) a step of binding a substance containing the identified follicular T cell epitope, or its functional equivalent to other vaccine antigens/nucleic acids, or expressing same independently in viral vector or VLP
2) a step of mixing same with something that induces immunity different from B cells (such as cytotoxic T cell epitopes) or Tfh, and
3) a step of administering the product obtained by 2).

### (Computer program or recording medium storing same, and user equipment that constitutes system or part of the system, as techniques for evaluating disease in subject)

In one aspect of the present disclosure, provided are a method for evaluating a disease in a subject, a computer program for realizing this or a recording medium storing this, a user equipment that constitutes a system or part of that system; and the like.

System 1000 includes at least one user device 100, a server device 200 connected to the at least one user device 100 via network 400, and database unit 300 connected to the server device 200 (Fig. 8).

The user device 100 can be any terminal device such as dedicated medical instrument, smart phones, tablet computers, smart watches, laptop computers, desktop computers, medical wearable devices, and the like. The user device 100 can communicate with the server device 200 via the network 400. Here, the type of the network 400 does not matter. For example, the user device 100 may communicate with the server device 200 via internet, or may communicate with the server device 200 via a LAN. Although three user devices 100 are depicted in Fig. 8, the number of the user devices 100 is not limited to this. The number of the user devices 100 can be the same or different any number greater than or equal to one.

The server device 200 is capable of communicating with at least one user device 100 via the network 400. In addition, the server device 200 can communicate with the database unit 300 connected to the server device 200.

For example, the database unit 300 connected to the server device 200 may store information relating to follicular T cells of a plurality of targets acquired in advance, any health and medical information relating to the subject, and the like. A plurality of stored information can be utilized, for example, to build a trained model. For example, the database unit 300 may store the built trained model.

Fig. 9 shows an example of the configuration of the user device 100.

The user device 100 has a communication interface section 110, an input section 120, a display section 130, a memory section 140 and a processor section 150.

The communication interface unit 110 controls communication via the network 400. The processor unit 150 of the user device 100 can receive information from outside the user device 100 and can transmit information to the outside of the user device 100 via the communication interface unit 110. For example, the processor unit 150 of the user device 100 can receive information from the server device 200 and can transmit information to the server device 200 via the communication interface unit 110. The communication interface section 110 may control communications in any manner.

The input unit 120 allows a user to input information into the user device 100. It does not matter in what manner the input unit 120 enables the user to input information to the user device 100. For example, if the input unit 120 is a touch panel, the user may input information by touching the touch panel. Alternatively, if the input unit 120 is a mouse, the user may input information by operating the mouse. Alternatively, if the input unit 120 is a keyboard, the user may input information by pressing keys on the keyboard. Alternatively, if the input unit 120 is a microphone, the user may input information by voice.

The display 130 may be any display for displaying information.

The memory unit 140 stores programs for executing processes in the user device 100, data required for executing the programs, and the like. The memory unit 140 stores a part or all of a program for evaluating medical techniques of, for example, evaluation of history of diseases of target, evaluation of effectiveness of prophylactic agent or vaccine for the disease, evaluation of effectiveness of therapeutic agent for the disease, evaluation of defensive ability against re-contraction of the disease, pathology evaluation of the disease, and evaluation of the risk of contracting the disease. The memory unit 140 may store applications that implement arbitrary functions. The memory unit 140 may store an application for obtaining information about the level or amount of follicular T cells, for example, by measuring biochemical information about the follicular T cells. Thereby, the user device 100 has a part that implements the measurement of information relating to follicular T cells. The memory unit 140 may store, for example, an application to obtain information on the level or amount of follicular T cells from biochemical information about follicular T cells. Thereby, the user device 100 has a part that implements the function of measuring the electro-oculography. In addition, the memory unit 140 may store an application for obtaining ocular information by other means. Here, it does not matter how the program is stored in the memory unit 140. For example, the program may be pre-installed in memory unit 140. Alternatively, the program may be installed in memory unit 140 by being downloaded via network 400. Memory unit 140 may be implemented by any storage means.

The processor unit 150 controls the operation of the user device 100 as a whole. The processor unit 150 reads a program stored in the memory unit 140 and executes the program. This allows the user device 100 to function as a device that executes desired steps. The processor unit 150 may be implemented by a single processor or multiple processors.

The user device 100 can include data acquiring unit 160 in addition to the configuration described above. The data acquiring unit 160 is any means for acquiring biochemical information of follicular T cells, which is exemplified by biochemical means such as ELISA and the like. The data acquiring unit 160 is, for example, a device that realizes ELISA. The data acquiring unit 160 may be, for example, a light absorption means built in the user device 100 or an external FACS device attached to the user device 100.

The user device 100 can include an additional information acquiring means 170 in addition to the above-described configuration or instead of the data acquiring unit 160 described above. The additional information acquiring means 170 is any means for acquiring additional information. The additional information acquiring means 170 is, for example, a means for acquiring information relating to infectious disease, vaccine, cancer, allergy, and autoimmune disease. For example, the additional information acquiring means 170 may be a biotechnology device such as a PCR that identifies an infectious disease in the user device 100, or may be an antigen-antibody reaction measuring device attached to the user device 100. For example, when the user device 100 is combined with another medical device, the other medical device can be used as the additional information acquiring means 170.

Although each component of the user device 100 is provided in the user device 100 in the example shown in Fig. 9, the present disclosure is not limited to this. Any of the components of user device 100 may be provided external to user device 100. For example, when the input unit 120, the display unit 130, the memory unit 140, the processor unit 150, and the imaging unit 160 are each configured by separate hardware components, each hardware component may be connected via an arbitrary network. At this time, the type of network does not matter. Each hardware component may be connected via a LAN, wirelessly, or wired, for example. The user device 100 is not limited to a particular hardware configuration. For example, it is within the scope of the present disclosure to configure the processor unit 150 with analog circuitry rather than digital circuitry. The configuration of the user device 100 is not limited to those described above as long as its functions can be realized.

Fig. 10 shows an example of the configuration of the server device 200.

The server device 200 includes a communication interface section 210, a memory section 220 and a processor section 230.

The communication interface section 210 controls communication via network 400. The communication interface section 210 also controls communication with the database section 300. The processor unit 230 of the server device 200 can receive information from outside the server device 200 via the communication interface unit 210 and can transmit information to the outside of the server device 200. The processor unit 230 of the server device 200 can receive information from the user device 100 via the communication interface unit 210 and can transmit information to the user device 100. The communication interface unit 210 may control communications in any manner.

The memory unit 220 stores programs required for executing processes of the server device 200, data required for executing the programs, and the like. For example, a part or all of a program for analyzing follicular T cells and evaluating diseases based thereon is stored. The memory unit 220 may store, for example, an application for acquiring information of the level or amount of follicular T cells, and an application for evaluating medical techniques of, for example, evaluation of history of diseases of target, evaluation of effectiveness of prophylactic agent or vaccine for the disease, evaluation of effectiveness of therapeutic agent for the disease, evaluation of defensive ability against re-contraction of the disease, pathology evaluation of the disease, and evaluation of the risk of contracting the disease. The memory unit 220 may store, for example, an application for acquiring information of follicular T cells and other medical techniques by other means. The memory unit 220 may be implemented by any storage means.

The processor unit 230 controls the operation of the server device 200 as a whole. The processor unit 230 reads a program stored in the memory unit 220 and executes the program. This allows the server device 200 to function as a device that executes desired steps. The processor unit 230 may be implemented by a single processor or multiple processors.

In the example shown in Fig. 10, each component of the server device 200 is provided within the server device 200, but the present disclosure is not limited to this. Any one of the components of server device 200 may be provided outside server device 200. For example, when the memory unit 220 and the processor unit 230 are each composed of separate hardware components, each hardware component may be connected via an arbitrary network. At this time, the type of network does not matter. Each hardware component may be connected via a LAN, wirelessly, or wired, for example. The server device 200 is not limited to a specific hardware configuration. For example, it is within the scope of the present disclosure to configure the processor portion 230 with analog circuitry rather than digital circuitry. The configuration of the server device 200 is not limited to the above as long as the functions can be realized.

In the examples illustrated in Figs. 8 to 10, the database unit 300 is provided outside the server device 200, but the present disclosure is not limited to this. It is also possible to provide the database unit 300 inside the server device 200. At this time, the database section 300 may be implemented by the same storage means as the storage means in which the memory section 220 is implemented, or by a storage means different from the storage means in which the memory section 220 is implemented. In any case, the database unit 300 is configured as a storage unit for the server device 200. The configuration of the database unit 300 is not limited to a specific hardware configuration. For example, the database unit 300 may be configured with a single hardware component, or may be configured with a plurality of hardware components. For example, the database unit 300 may be configured as an external hard disk device of the server device 200, or configured as a cloud storage connected via a network.

### (General technology)

Any molecular biological methods, biochemical methods, and microbiological methods used in the present specification are well known and conventionally used in the art. For example, they are described in Current Protocols in Molecular Biology (http://onlinelibrary.wiley.com/book/10.1002/0471142727) and Molecular Cloning: A Laboratory Manual (Fourth Edition) (http://www.molecularcloning.com) and the like, and relevant portions of these (which may be all of them) are incorporated herein by reference.

In the present specification, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present invention is not limited to the embodiments and Examples specifically described herein and is limited only by the scope of claims.

### [Example]

In the present Examples, based on the Declaration of Helsinki, necessary informed consent was obtained from the test subjects and the following experiments were conducted in compliance with the ethical regulations stipulated by the university. In addition to the reagents described in Examples, those obtained from Wako Pure Chemical Industries, Sigma-Aldrich, etc. can also be used.

### (Example 1: Virus case (general case))

In this Example, a single cell-based RNA sequencing platform (Chromium from 10xGenomics) is used to analyze SARS-CoV-2 specific T cell subsets and their clonotypes.

### (Separation of peripheral blood mononuclear cell (PBMC) and plasma collection)

Peripheral blood mononuclear cells (PBMCs) are prepared from six individuals, including healthy donors and virally infected patients (e.g., influenza virus infected patients). Preparation is carried out as follows. Whole blood was collected into heparin-coated tubes and centrifuged at 1500 rpm for 10 min to separate the cellular fraction and plasma. The plasma is removed from the cell pellet and store at -80°C. Next, PBMCs are separated by density gradient sedimentation and red blood cells are lysed using ACK lysis buffer. The separated PBMCs are stored frozen at -80°C in STEM-CELLBANKER (Zenoaq Resource).

Cryopreserved PBMC was thawed and washed with RPMI1640 medium supplemented with 5% human AB serum. 5×10⁵ PBMCs are stimulated with inactive virus (e.g., influenza virus) containing 1 µg/ml S protein, recombinant S protein (1 µg/ml), S peptide pool (1 µg/ml/peptide), or M+N peptide pool (1 µg/ml). (The stimulatory protein is appropriately selected depending on the virus. Recombinant virus proteins are prepared based on known literature). As PepMix influenza virus (various proteins) and the like, commercially available products may be used (for example, Funakoshi company). 20 hr at 37°C, followed by staining with anti-human CD3 (HIT3a), CD69 (FN50), CD137 (4B4-1) and TotalSe (trademark)-C Hashtag (all purchased from BioLegend). CD3+CD69+ or CD3+CD137+ cells are sorted by cell sorter SH-800S cells (SONY) and analyzed for TCR sequences along with RNA expression by single cell VDJ-RNA-seq analysis as described below.

### (Single-cell-based transcriptome and TCR repertoire analysis)

The following reagents were used for single cell capture and library preparation. Chromium Single Cell 5'Library&Gel Bead Kit, PN-1000165; Chromium Next GEM Chip G Single Cell Kit, PN-1000120; Chromium Single Index Kit T Set A, PN-1000213; Chromium Single Cell 5'Feature Barcode Library Kit, PN-1000080; Single Index Kit N Set A, PN-1000212; Chromium single cell V(D)J concentration kit, human T cells, PN-1000005. A single cell suspension containing about 2×10⁴ cells are loaded onto a Chromium microfluidic chip to generate single-cell gel-bead-in-emulsions using a Chromium controller (10XGenomics) according to the manufacturer's instructions. Barcoded cellderived RNA for each sample was then reverse transcribed in a gel-bead-in-emulsion using the Veriti Thermal Cycler (Thermo Fisher Scientific), and all subsequent steps to generate single-cell libraries are performed according to the manufacturer's protocol, with 14 cycles for cDNA amplification. Approximately 50 ng of cDNA is then used for 14 cycles of gene expression library amplification in parallel with TCR library cDNA enrichment and library construction. The library fragment size is confirmed using the Agilent 2100 Bioanalyzer (Agilent). The library was sequenced on an Illumina NovaSeq6000 in paired-end mode (read1: 28bp; read2: 91bp). Raw reads are processed by Cell Ranger 3.1.0 (10XGenomics). Gene expressionbased clustering is performed using the Seurat R package (v3.1, Hafemeister, C., Satija, R. Normalization and variance stabilization of single-cell RNA-seq data using regularized negative binomial regression. Genome Biol 20, 296 (2019).https://doi.org/10.1186/s13059-019-1874-1). Briefly, cells with more than 10% mitochondrial content, less than 200 detected genes, or more than 4000 genes were considered outliers (dying cells, empty droplets and doublets, respectively) and filtered out. Since the SeuratSCTransform function was used for normalization and all samples were processed simultaneously, the data are integrated without performing batch effect correction. HashTag oligo demultiplexing is performed on CLR-normalized HashTagUMI counts and clonotypes are matched to gene expression data via droplet barcodes using a Python script. Only cells assigned a single hashtag and beta chain clonotype are retained for downstream analysis.

### (Bulk TCR sequence)

1 - 3×10⁵ PBMCs were lysed with QIAzol, then full-length cDNA was synthesized using SMARTer technology (Takara Bio) and variable regions of TCRα and β genes are amplified using TRAC/TRBC specific primers. After sequencing the variable region amplicons, each pair of reads was assigned clonotype (defined as TR(A/B)V and TR(A/B)J genes and complementarity determining region (CDR) 3) using MiXCR software (Bolotin, D., Poslavsky, S., Mitrophanov, I. et al. MiXCR: software for comprehensive adaptive immunity profiling. Nat Methods 12, 380-381 (2015).https://doi.org/10.1038/nmeth.3364). For each alpha/beta clonotype, expansion is defined as the proportion of reads for that clone divided by the total number of reads for the alpha/beta chain, respectively, and the proportions are log10 transformed for plotting and statistical analysis.

By UMAP plots and single-cell gene expression, it is confirmed that the circulating Tfh cluster is composed of cells expressing Tfh-related genes such as CD200, PDCD1, ICOS, CXCL13, and CD40LG. Furthermore, not less than 1100 pairs of TCRs are identified within the Tfh cluster.

Among these, TCRαβ pairs shared between patients are detected.

These TCRαβ pairs are designated as clones 1 and 2, derived from patients X1 and X2, respectively, sharing identical Vα, Jα, Vβ, and Jβ usage. Furthermore, it may be found that the CDR3α sequences of clones 1 and 2 are identical, and that CDR3β is identical except for one amino acid.

In patient X2, clone 2 is detected as two different barcoded cells that share exactly the same TCR sequence.

To examine the antigen-specificity of TCR, clonotypes 1 and 2, the respective TCR α and β chains are transfected into TCR-deficient T-cell hybridomas and TCR-reconstituted CD3+ cells are established.

These TCR transfectants are stimulated with various antigens in the presence of Epstein-Barr virus (EBV)-transformed B cells derived from each corresponding patient.

Responsiveness is confirmed for clones 1 and 2. This can be confirmed to be consistent with the initial antigen-specificity revealed by single cell analysis.

Since activation was excluded in the absence of antigen-presenting cells, it is confirmed that activation is mediated by MHC molecules.

Of the two pools, only the latter pool stimulated clones 1 and 2, indicating that antigenic peptides are contained in the latter's specific locations.

However, it is confirmed that another available peptide pool, containing a large number of peptides, does not activate clones 1 and 2.

These results confirm the location of the antigenic epitopes of clone 1 and clone 2.

The HLA alleles constraining clones 1 and 2 are then determined to further reduce the number of peptide candidates for the epitope.

It is found that antigen-presenting cells (APCs) from both patients X1 and X2 can activate clones 1 and 2. This indicates that APCs are interchangeable.

Therefore, each of the MHC class II alleles shared between X1 and X2, such as DRB1*15:01, DPA1*02:02-DPB1*05:01, DQA1*01:02-DQB1*06, and the like, is considered an HLA candidate. These alleles are then individually transfected to examine recognition of the S peptide by clones 1 and 2.

Both clones 1 and 2 respond to S peptide pool #2 only in the presence of DRA*01:01 and DRB1*15:01, and DRA is monomorphic, suggesting that the responsiveness is determined by DRB1*15:01.

Therefore, NetMHC server software (http://www.cbs.dtu.dk/services/NetMHCIIpan/) is used to search for candidate peptides predicted to bind to DRA-DRB1*15:01.

Several strong binders are predicted as candidates within narrowed regions of specific viral proteins. Therefore, by synthesizing two peptides and stimulating clones 1 and 2, a particular peptide can be determined as an epitope for both clones 1 and 2.

It may be found that the TCRs of clones 1 and 2 differ by one amino acid within CDR3. In this case, since both TCRs recognize the same epitope presented by the same MHC molecule and are from different donors, it is understood to suggest that clones 1 and 2 are public clones. This epitope can be interpreted as having low homology with the corresponding amino acid sequences of other influenza viruses.

Indeed, it can be found that neither clone 1 nor 2 responds at all to peptide pools and total proteins derived from other viral proteins in the presence of APC expressing DRA-DRB1*15:01. Furthermore, it is suggested that clone 1/2 is unlikely to cross-react with related influenza viruses.

It is next examined whether MHC class II alleles other than DRB1*15:01 can present this antigenic peptide recognized by clone 1/2.

As a result, specific amino acid regions can also be predicted as strong binders to DRB1*15:02 and 15:01. Furthermore, the amino acid sequence identity between DRB1*15:01 and *15:02 is 99.6%. Therefore, it is speculated that DRB1*15:02 is also the allele responsible for epitope recognition, and APC from another individual who has DRB1*15:02 but not DRB1*15:01 can be tested. As a result, DRB1*15:02 can be identified as being able to stimulate both clones 1 and 2 in the presence of this particular peptide.

Considering the frequency of DRB1*15:02 (10.6%) and *15:01 (7.7%) in the Japanese population (http://hla.or.jp/), if these clonotypes were present, clones 1 and 2 could be expected to respond to this peptide in 18.3% of the population. Similar restriction specificity can be tested for other HLA types.

Next, the occurrence of the clone 2/3 clonotype in a cohort that was considered not to be infected with influenza virus was investigated and it was found that a certain number of people had the TCRβ clonotype of clone 1/2. This is similar to the ratio in this Example.

Next, considering that clone 1/2 is this virus-specific T cell exhibiting a Tfh profile, it is hypothesized that clone 1/2 was clonally expanded during infection to promote protective humoral immunity and the following examination is performed.

DRB1*15:01/15:02 is not a minor allele in the world (see http://www.allelefrequencies.net/tools/Report.aspx). Thus, frequency of clone 1/2 clonotypes in healthy individuals and individuals who have recovered from infection is examined in a public database of US citizens (immune response actions to COVID-19 events; ImmuneRace, https://immunerace.adaptivebiotech.com/). Clonotypes are detected at a certain frequency in each of the healthy group and the recovered group. Notably, it is observed that the frequency of this clonotype among each individual was significantly increased in the recovered group compared to healthy controls, suggesting that this particular proteinspecific clone is clonally expanded in different individuals during infection. Interestingly, the frequency of clonotype 1/2 in recovered patients is greater than the allele frequency of DRB1*15:01 or 15:02 worldwide (11.3%). Thus, it is possible that clone 1/2 recognizes other epitopes from this virus presented by other HLA alleles.

Taken together, the first identification of public T cell clones that recognize SARS-CoV-2 and its epitopes will contribute to the development of diagnostic strategies or options and next-generation vaccines.

### (Example 2: Virus case (in the case of SARS-CoV-2))

In this Example, a single cell-based RNA sequencing platform (Chromium from 10xGenomics) was used to analyze SARS-CoV-2-specific T cell subsets and their clonotypes.

### (Separation of peripheral blood mononuclear cell (PBMC) and plasma collection)

Peripheral blood mononuclear cells (PBMCs) are prepared from six individuals, including healthy donors and COVID-19 patients in the recovery stage (Tables 1 and 2). Preparation was carried out as follows. Whole blood was collected into heparin-coated tubes and centrifuged at 1500 rpm for 10 min to separate the cellular fraction and plasma. The plasma was removed from the cell pellet and store at -80°C. Next, PBMCs were separated by density gradient sedimentation and red blood cells were lysed using ACK lysis buffer. The separated PBMCs were stored frozen at -80°C in STEM-CELLBANKER (Zenoaq Resource).

**Table 1: Participant characteristics**

| [Table 1] | | | |
|---|---|---|---|
| | | COVID19 Patients | Unexposed |
| | | N = 5 | N = 1 |
| Age (years) | | A-B (Median = C, IQR = D) | 31 |

| Gender | | | |
|---|---|---|---|
| | Male (%) | E% (F/G) | 100% (1/1) |
| | Female (%) | | N/A |

| Nationality | | | |
|---|---|---|---|
| | Japanese (%) | | 100% (1/1) |
| | Others (%) | | N/A |

| Peak Disease | | | |
|---|---|---|---|
| | Mild | N/A | N/A |
| | Moderate I | 20% (1/5) | N/A |
| | Moderate II | 20% (1/5) | N/A |
| | Severe | 60% (3/5) | N/A |

**Table 2: COVID-19 disease severity classifications in Japan**

| [Table 2] | | |
|---|---|---|
| | Saturation of Percutaneous Oxygen (SpO₂) | Clinical Symptoms and Conditions |
| Mild | SpO₂ ≥ 96% | No respiratory symptoms. |
| | | Only cough but no dyspnea. |
| Moderate I | 93% <SpO₂ < 96% | Dyspnea or abnormal shadow on chest X-ray or computed tomography. |
| Moderate II | SpO₂ ≤ 93% | Oxygenation is necessary. |
| Severe | | Intensive Care Unit level care or Mechanical ventilation is necessary. |

Cryopreserved PBMC was thawed and washed with RPMI1640 medium supplemented with 5% human AB serum. 5×10⁵ PBMCs were stimulated with inactivated SARS-CoV-2 containing 1 µg/ml S protein, recombinant S protein (1 pg/ml), S peptide pool (1 pg/ml/peptide), or M+N peptide pool (1 pg/ml). (Inactivated SARS-CoV-2 virus was provided by Mr. Shioda and Mr. Nakayama (Research Institute of Microbial Diseases, Osaka University). Recombinant SARS-CoV-2S protein was prepared as described in Amanat F, et al. bioRxiv.2020. PepMix SARS-CoV-2 (spike glycoprotein) (including pools #1 and #2) was purchased from JPT Peptide Technologies. PepTivator SARS-CoV-2 Prot_M and _N were purchased from MiltenyiBiotec.). 20 hr at 37°C, followed by staining with anti-human CD3 (HIT3a), CD69 (FN50), CD137 (4B4-1) and TotalSeq (trademark)-C Hashtag (all purchased from BioLegend). CD3+CD69+ or CD3+CD137+ cells were sorted by cell sorter SH-800S cells (SONY) and analyzed for TCR sequences along with RNA expression by single cell VDJ-RNA-seq analysis as described below.

### (Single-cell-based transcriptome and TCR repertoire analysis)

The following reagents were used for single cell capture and library preparation. Chromium Single Cell 5'Library&Gel Bead Kit, PN-1000165; Chromium Next GEM Chip G Single Cell Kit, PN-1000120; Chromium Single Index Kit T Set A, PN-1000213; Chromium Single Cell 5'Feature Barcode Library Kit, PN-1000080; Single Index Kit N Set A, PN-1000212; Chromium single cell V(D)J concentration kit, human T cells, PN-1000005. A single cell suspension containing about 2×10⁴ cells were loaded onto a Chromium microfluidic chip to generate single-cell gel-bead-in-emulsions using a Chromium controller (10XGenomics) according to the manufacturer's instructions. Barcoded cell-derived RNA for each sample was then reverse transcribed in a gel-bead-in-emulsion using the Veriti Thermal Cycler (Thermo Fisher Scientific), and all subsequent steps to generate single-cell libraries were performed according to the manufacturer's protocol, with 14 cycles for cDNA amplification. Approximately 50 ng of cDNA was then used for 14 cycles of gene expression library amplification in parallel with TCR library cDNA enrichment and library construction. The library fragment size was confirmed using the Agilent 2100 Bioanalyzer (Agilent). The library was sequenced on an Illumina NovaSeq6000 in paired-end mode (read1: 28bp; read2: 91bp). Raw reads were processed by Cell Ranger 3.1.0 (10XGenomics). Gene expression-based clustering was performed using the Seurat R package (v3.1, Hafemeister, C., Satija, R. Normalization and variance stabilization of single-cell RNA-seq data using regularized negative binomial regression. Genome Biol 20, 296 (2019).https://doi.org/10.1186/s13059-019-1874-1). Briefly, cells with more than 10% mitochondrial content, less than 200 detected genes, or more than 4000 genes were considered outliers (dying cells, empty droplets and doublets, respectively) and filtered out. Since the SeuratSCTransform function was used for normalization and all samples were processed simultaneously, the data were integrated without performing batch effect correction. HashTag oligo demultiplexing was performed on CLR-normalized HashTagUMI counts and clonotypes were matched to gene expression data via droplet barcodes using a Python script. Only cells assigned a single hashtag and beta chain clonotype were retained for downstream analysis.

1 - 3×10⁵ PBMCs were lysed with QIAzol, then full-length cDNA was synthesized using SMARTer technology (Takara Bio) and variable regions of TCRα and β genes were amplified using TRAC/TRBC specific primers. After sequencing the variable region amplicons, each pair of reads was assigned clonotype (defined as TR(A/B)V and TR(A/B)J genes and complementarity determining region (CDR) 3) using MiXCR software (Bolotin, D., Poslavsky, S., Mitrophanov, I. et al. MiXCR: software for comprehensive adaptive immunity profiling. Nat Methods 12, 380-381 (2015).https://doi.org/10.1038/nmeth.3364). For each alpha/beta clonotype, expansion was defined as the proportion of reads for that clone divided by the total number of reads for the alpha/beta chain, respectively, and the proportions were log10 transformed for plotting and statistical analysis.

By UMAP plots and single-cell gene expression, it was confirmed that the circulating Tfh cluster was composed of cells expressing Tfh-related genes such as CD200, PDCD1, ICOS, CXCL13, and CD40LG (Fig. 1A). Furthermore, not less than 1100 pairs of TCRs were identified within the Tfh cluster (Table 3).

**[Table 3-1]**

| **TRBV** | **CDR3β amino acid** | **TRB J** | **TRAV** | **CDR3α amino acid** | **TRA J** | **CLUS TER** | **Patient** | **Stimulation** | **4-1BB.1 vl** | **IFNG. lvl** | **CD69. lvl** | **Seurat.barcode** |
|---|---|---|---|---|---|---|---|---|---|---|---|---|
| TRBV2 0-1 | CSASPGLNTDTQ YF | TRB J2-3 | TRAV8-3 | CAVLTGGYNKLI F | TRA J4 | 47605 | HC | recombin ant S protein | Low/ Nega | Low/ Nega | Low/ Nega | DB1_TGGTTAGAGC GCCTTG |
| TRBV2 0-1 | CSASGTGEVGEL FF | TRB J2-2 | TRAV4 | CLVARGGYQKV TF | TRA J13 | 48063 | HC | Peptide pool M+N | Low/ Nega | Low/ Nega | High | DB1_AACTCCCGTC CTGCTT |
| TRBV3 0 | CAWKQGWTEAFF | TRB J1-1 | TRAV8-4 | CAVSDLYGNNR LAF | TRA J7 | 25270 | HC | Peptide pool S | Low/ Nega | Low/ Nega | Low/ Nega | DB1_AGCGTCGTCA GCTCTC |
| TRBV7-7 | CASSTSGNTGELF F | TRB J2-2 | TRAV8-4 | CAVSDSGAGSY QLTF | TRA J28 | 35098 | HC | inactivat ed virus | High | Mediu m | Mediu m | DB1_ATGTGTGAGG AGTACC |
| TRBV7-6 | CASSLTGLPIYNE QFF | TRB J2-1 | TRAV25 | CAGLGAGNMLT F | TRA J39 | 22074 | HC | recombin ant S protein | Low/ Nega | Low/ Nega | Low/ Nega | DB1_TTGCCGTCAT GTCCTC |
| TRBV2 0-1 | CSATDRANYGYT F | TRB J1-2 | TRAV35 | CAASGINAGKST F | TRA J27 | 47996 | HC | Peptide pool M+N | Low/ Nega | Low/ Nega | Low/ Nega | DB1_TGTTCCGGTC CTCCAT |
| TRBV2 7 | CASSSPGQGAYN EQFF | TRB J2-1 | TRAV2 | CAVGPPGFNKF YF | TRA J21 | 22783 | HC | Peptide pool M+N | Low/ Nega | Low/ Nega | Low/ Nega | DB1_CATCGAACAC GGTAGA |
| TRBV6-5 | CASRTVNTEAFF | TRB J1-1 | TRAV25 | CAISGNARLMF | TRA J31 | 27505 | HC | recombin ant 9 protein | Low/ Nega | Low/ Nega | Mediu m | DB1_CCAATCCAGT GGTAGC |
| TRBV1 8 | CASSLGQVYEQY F | TRB J2-7 | TRAV26 -2 | CILRESKAGNM LTF | TRA J39 | 45929 | HC | Peptide pool S | Low/ Nega | Low/ Nega | Low/ Nega | DB1_CCAGCGATCT ATCCCG |

**[Table 3-2]**

| | | | | | | | | | | | | |
|---|---|---|---|---|---|---|---|---|---|---|---|---|
| TRBV2 | CATEAGETQYF | TRB J2-5 | TRAV1-2 | CAVRALTGGGN KLTF | TRA J10 | 28997 | HC | Peptide pool S | Mediu m | Low/ Nega | Low/ Nega | DB1_TGCGCAGGTT GCTCCT |
| TRBV5-1 | CASSLVSDYEQYF | TRB J2-7 | TRAV17 | CATDEGDYKLS F | TRA J20 | 43708 | HC | Peptide pool M+N | Low/ Nega | High | Low/ Nega | DB1_CTCCTAGCAC TGCCAG |
| TRBV6-5 | CASSQTTGVVEQ YF | TRB J2-7 | TRAV8-4 | CAVSDRIKAAG NKLTF | TRA J17 | 37974 | HC | recombin ant S protein | Low/ Nega | Low/ Nega | Low/ Nega | DB1_GCTTCCATCG TAGGAG |
| TRBV2 0-1 | CSARAFEGELFF | TRB J1-4 | TRAV12 -2 | CAVRRTDYKLS F | TRA J20 | 26900 | HC | Peptide pool M+N | Mediu m | Low/ Nega | Low! Nega | DB1_TCGGGACCAC TTACGA |
| TRBV9 | CASSVWGSGGNE QFF | TRB J2-1 | TRAV17 | CATEGSGYSTLT F | TRA J11 | 11503 | patie nt#1 | Peptide pool S | Low/ Nega | Low/ Nega | High | DB1_TGAGAGGTCC GCATCT |
| TRBV1 2-4 | CASSLGLQGNQP QHF | TRB 11-5 | TRAV17 | CASRASGSRLTF | TRA J58 | 20367 | patie nt#1 | Peptide pool S | High | Low/ Nega | Low/ Nega | DB1_AACGTTGCAC AAGACG |
| TRBV2 0-1 | CSAKTSGRGETQ YF | TRB J2-5 | TRAV13 -2 | CAENSGGSNYK LTF | TRA J53 | 47690 | patie nt#1 | Peptide pool S | High | Low/ Nega | Low/ Nega | DB1_GGCTCGACAC CTATCC |
| TRBV6-5 | CASSSISGLNQPQ HF | TRB 11-5 | TRAV12 -3 | CAMSAGSGTYK YIF | TRA J40 | 20204 | patie nt#1 | Peptide pool S | Mediu in | Low/ Nega | Low/ Nega | DB1_CGCGTTTGTA AACCTC |
| TRBV1 9 | CASSIRSSYEQYF | TRB J2-7 | TRAV27 | CAGVGGGSQGN LIF | TRA J42 | 46110 | patie nt#1 | Peptide pool M+N | Low/ Nega | High | Low/ Nega | DB1_CACATTTGTA GCTCCG |
| TRBV2 | CASSPGQGNSPL HF | TRB J1-6 | TRAV8-4 | CAVSAASARQLT F | TRA J22 | 30803 | patie nt#1 | Peptide pool S | Low/ Nega | Low/ Nega | Low/ Nega | DB1_ATTGGACGTA GCGCAA |
| TRBV1 1-2 | CASSWDGDNSPL HF | TRB 11-6 | TRAV3 | CAVRDKTGRRA LTF | TRA J5 | 30866 | patie nt#1 | Peptide pool S | High | Low/ Nega | Low/ Nega | DB1_TAAGAGAGTG TAATGA |

**[Table 3-3]**

| TRBV7-7 | CASSLRYGGELFF | TRB J2-2 | TRAV36 /DV7 | CAVDSGTYRYIF | TRA J40 | 40982 | patie nt#2 | reeombin ant S protein | Mediu m | Low/ Nega | Low/ Nega | DB2_GTAGGCCCAA TGGTCT |
|---|---|---|---|---|---|---|---|---|---|---|---|---|
| TRBV3-1 | CASSHDRAEQYF | TRB J2-7 | TRAV30 | CGTEIGTDKLIF | TRA J34 | 26138 | patie nt#2 | inactivat ed virus | Low/ Nega | Low/ Nega | Low/ Nega | DB2_CACTCCAGTA TAGGGC |
| TRBV9 | CASSERTSGNGE QFF | TRB J2-1 | TRAV12 -2 | CAVSPPGGSYIP TF | TRA J6 | 11505 | patie nt#2 | recombin ant S protein | Low/ Nega | Low/ Nega | Low/ Nega | DB2_GATCTAGCAT TCCTGC |
| TRBV3-1 | CASSPRAGAGGE LFF | TRB J2-2 | TRAV38 -1 | CAFMKYNNND MRF | TRA J43 | 14496 | patie nt#2 | Peptide pool M+N | Low/ Nega | High | Low/ Nega | DB2_AGCTCTCGTT GACGTT |
| TRBV2 4-1 | CATSRTGGGTAE QYF | TRB J2-7 | TRAV5 | CAERGAAGNKL TF | TRA J17 | 8497 | patie nt#2 | recombin ant S protein | Low/ Nega | Low/ Nega | Low/ Nega | DB2_CGTGTCTTCT TGTACT |
| TRBV5-4 | CASSLGAGGPYN EQFF | TRB J2-1 | TRAV9-2 | CALTLTGNQFY F | TRA J49 | 22442 | patie nt#2 | recombin ant S protein | Mediu m | Low/ Nega | High | DB2_ACCGTAATCG CTAGCG |
| TRBV2 0-1 | CSASRGAGWMET QYF | TRB J2-5 | TRAV12 -1 | GVVNRGSNYKL TF | TRA J53 | 48660 | patie nt#2 | inactivat ed virus | Low/ Nega | Mediu m | Mediu in | DB2_CTAATGGTCG CGTAGC |
| TRBV2 8 | CASSLSPPRQGSE SPLHF | TRB J1-6 | TRAV14 /DV4 | CAMREGRTGGF KTIF | TRA J9 | 7178 | patie nt#2 | Peptide pool S | Low/ Nega | Low/ Nega | Low/ Nega | DB2_GAAGCAGCAA GCGCTC |
| TRBV5-1 | CASSSSSVSYEQY F | TRB J2-7 | TRAV38 -1 | CAFVSTGANSK LTF | TRA J56 | 36273 | patie nt#2 | inactivat ed virus | Low/ Nega | High | Low/ Nega | DB2_GACGTGCTCA GCTGGC |
| TEBV1 9 | CASSIAGAGKQFF | TRB J2-1 | TRAV12 -2 | CAFEHGSSNTG KLIF | TRA J37 | 45762 | patie nt#3 | Peptide pool M+N | High | Low/ Nega | Low/ Nega | DB2_GAGCAGAAG GCATTGG |
| TRBV1 1-2 | CASSRTYEQYF | TRB J2-7 | TRAV12 -1 | CVVNMNYGGS QGNLIF | TRA J42 | 29298 | patie nt#3 | Peptide pool S | Mediu m | Low/ Nega | Mediu m | DB2_GAGCAGACAT GCATGT |

**[Table 3-4]**

| | | | | | | | | | | | | |
|---|---|---|---|---|---|---|---|---|---|---|---|---|
| TRBV5-1 | CASSSSVQMNTE AFF | TRB J1-1 | | | | 14219 | patie nt#3 | recombin ant S protein | High | Low/ Nega | Low/ Nega | DB2_GCATACAGTG TGAATA |
| TRBV2 7 | CASSSVTGVSLRE QFF | TRB J2-1 | TRAV22 | CAVEGSGTYKYI F | TRA J40 | 21747 | patie nt#3 | inactivat ed virus | Low/ Nega | Low/ Nega | Low/ Nega | DB2_GCCAAATAGT TGTCGT |
| TRBV6-6 | CASNNRAGNTIY F | TRB J1-3 | TRAV21 | CAVNQRSYDKV IF | TRA J50 | 39348 | patie nt#3 | inactivat ed virus | Mediu in | High | Low! Nega | DB2_GCGACCATCA AACCGT |
| TRBV5-1 | CASSLPGAWNTE AFF | TRB J1-1 | TRAV3 | CAVRDPSGSAR QLTF | TRA J22 | 14278 | patie nt#3 | recombin ant S protein | Low/ Nega | Low/ Nega | Low/ Nega | DB2_GCTTGAATCC CGACTT |
| TRBV5-6 | CASSQAYEQYF | TRB J2-7 | TRAV23 /DV6 | CAASYGGATNK LIF | TRA J32 | 29063 | patie nt#3 | inactivat ed virus | Low/ Nega | High | Mediu m | DB2_TGTATTCAGG TGACCA |
| TRBV9 | CASSVGVGSDTQ YF | TRB J2-3 | TRAV36 ZDV7 | CAVAVQGAQKL VF | TRA J54 | 36451 | patie nt#3 | Peptide pool S | Mediu m | Mediu m | Low/ Nega | DB2_GGCTGGTAGT ATGACA |
| TRBV1 0-3 | CAISESGVNLGG NQPQHF | TRB 11-5 | TRAV12 -2 | CAVRAVGDKIIF | TRA J30 | 1470 | patie nt#3 | recombin ant S protein | Low/ Nega | Low/ Nega | Low/ Nega | DB2_GGTGAAGGTT AAGAAC |
| TRBV1 2-5 | CASGLVASPGAGE LFF | TRB J2-2 | TRAV35 | CAGGVTGTASK LTF | TRA J44 | 8006 | patie nt#3 | recombin ant S protein | High | Low/ Nega | Low/ Nega | DB2_GTCATTTGTC TGCCAG |
| TRBV3 0 | CAWSVLSGRPFG GGANVLTF | TRB J2-6 | TRAV12 -2 | CAVKKSWASYD KVIF | TRA J50 | 1261 | patie nt#3 | inactivat ed virus | Mediu m | Low/ Nega | Mediu m | DB2_GTGCAGCCAG TCTTCC |
| TRBV2 4-1 | CATSDLSGNQPQ HF | TRB J1-5 | | | | 30566 | patie nt#3 | inactivat ed virus | Low/ Nega | Low/ Nega | Low/ Nega | DB2_GTTAAGCGTG AACCTT |
| TRBV1 8 | CASSPDSTDTQYF | TRB J2-3 | TRAV23 /DV6 | CAASKYNTDKL IF | TRA J34 | 45926 | patie nt#3 | inactivat ed virus | Low/ Nega | High | Mediu m | DB2_TCATTACAGA AGGTTT |
| TRBV2 5-1 | CASSESGDNYGY TF | TRB J1-2 | TRAV21 | CALKGSDYKLS F | TRA J20 | 40481 | patie nt#3 | Peptide pool S | Mediu m | High | Low/ Nega | DB2_TGAGCCGGTA GAGCTG |

**[Table 3-5]**

| | | | | | | | | | | | | |
|---|---|---|---|---|---|---|---|---|---|---|---|---|
| TRBV5-1 | CASSLTGVRDGE QFF | TRB J2-1 | TRAV25 | CAGPLGSNFGN EKLTF | TRA J48 | 10652 | patie nt#3 | inactivat ed virus | Low/ Nega | Low/ Nega | Low/ Nega | DB2_CTGTTTACAA GTCTAC |
| TRBV7-6 | CASSSITEGGYTF | TRB 11-2 | | | | 31840 | patie nt#3 | recombin ant S protein | Low/ Nega | Low/ Nega | Low/ Nega | DB2_TTAACTCCAA GTTCTG |
| TRBV7-8 | CASSLGGVRADT QYF | TRB J2-3 | TRAV9-2 | CASDPSAGNNR KLIW | TRA J38 | 18009 | patie nt#3 | recombin ant S protein | Low/ Nega | Low/ Nega | High | DB2_TTCTTAGAGT TCGCAT |
| TRBV1 5 | CATSQDRVGSPL HF | TRB J1-6 | TRAV9-2 | CALSGSGSARQ LTF | TRA J22 | 30315 | patie nt#3 | recombin ant 9 protein | High | Low/ Nega | Low/ Nega | DB2_TTTGGTTCAT CGGTTA |
| TRBV5-1 | CASSLPGAWNTE AFF | TRB J1-1 | TRAV3 | CAVRDPSGSAR QLTF | TRA J22 | 14278 | patie nt#3 | recombin ant S protein | Low/ Nega | Low/ Nega | Low/ Nega | DB2_GGAATAATCA GCGATT |
| TRBV1 1-3 | CASSLVQVGRRY NEQFF | TRB J2-1 | | | | 5459 | patie nt#3 | inactivat ed virus | Low/ Nega | Low/ Nega | High | DB2_CTGAAACCAT GTCGAT |
| TRBV2 0-1 | CSASLARGQGNS PLHF | TRB 11-6 | TRAV12 -1 | CVVNYNFNKFY F | TRA J21 | 48327 | patie nt#3 | inactivat ed virus | Low/ Nega | Low/ Nega | Loul Nega | DB2_GCTCTGTAGG TTACCT |
| TRBV7-3 | CASSRAGGTVDT QYF | TRB J2-3 | TRAV2 | CAANNAGNMLT F | TRA J39 | 17392 | patie nt#3 | Peptide pool S | Low/ Nega | High | Low/ Nega | DB2_CTCGTACTCG CTTAGA |
| TRBV7-8 | CASSSDRGPGTG ELFF | TRB J2-2 | TRAV12 -3 | CAMKRGGSQG NLIF | TRA J42 | 23585 | patie nt#3 | inactivat ed virus | Low/ Nega | Low/ Nega | Mediu m | DB2_CTCTACGCAT GCATGT |
| TRBV1 2-3 | CASSIWDILAKNI QYF | TRB J2-4 | TRAV29 /DV5 | CAAPGYQKVTF | TRA J13 | 22111 | patie nt#3 | Peptide pool M+N | Low/ Nega | Mediu m | Low/ Nega | DB2_AAAGTAGCAA TAACGA |
| TRBV1 8 | CASSPPGLMNTE AFF | TRB J1-1 | TRAV8-1 | CAVNAGGNNRL AF | TRA J7 | 39092 | patie nt#3 | Peptide pool S | Low/ Nega | Low/ Nega | Low/ Nega | DB2_AAAGTAGGTA CGAAAT |

**[Table 3-6]**

| | | | | | | | | | | | | |
|---|---|---|---|---|---|---|---|---|---|---|---|---|
| TRBV1 2-5 | CASGLFRTGSLEK LFF | TRB J1-4 | TRAV6 | CALGSNARLMF | TRA J31 | 14643 | patie nt#3 | Peptide pool M+N | Low/ Nega | Low/ Nega | Low/ Nega | DB2_ACGGAGAGTA ATCACC |
| TRBV1 8 | CASSPGQGDTQY F | TRB J2-3 | TRAV13 -1 | CASLRSNNARL MF | TRA J31 | 45925 | patie nt#3 | recombin ant S protein | Mediu m | Low/ Nega | Low/ Nega | DB2_ATAAGAGGTG TCCTCT |
| TRBV5-6 | CASSPLGRSYGYT F | TRB J1-2 | TRAV5 | GAESLLDGQKL LF | TRA J16 | 40230 | patie nt#3 | Peptide pool S | Low/ Nega | Low/ Nega | High | DB2_ATTGGACTCG CCATAA |
| TRBV1 5 | CATSRDPGIQETQ YF | TRB J2-5 | TRAV3 | CAVSALYNTDK LIF | TRA J34 | 9815 | patie nt#3 | inactivat ed virus | Low/ Nega | Low/ Nega | Low/ Neg a | DB2_CAACCAAGTA TGAAAC |
| TRBV5-1 | CASSRMTGSTDT QYF | TRB J2-3 | TRAV12 -1 | CVVNPHARLMF | TRA J31 | 16197 | patie nt#3 | Peptide pool S | High | High | Low/ Nega | DB2_CAAGAAAGTG CGGTAA |
| TRBV2 0-1 | CSADRGLAGSTD TQYF | TRB J2-3 | TRAV9-2 | CALIPNNARLM F | TRA J31 | 47295 | patie nt#3 | inactivat ed virus | High | High | Low/ Nega | DB2_CACACTCGTG AGGGTT |
| TRBV5-1 | CASNLQGAGDTE AFF | TRB J1-1 | TRAV13 -1 | CAASKQGTGFQ KLVF | TRA J8 | 14285 | patie nt#3 | inactivat ed virus | Low/ Nega | Low/ Nega | Low/ Nega | DB2_CAGATCATCT GACCTC |
| TRBV1 1-2 | CASSQTYEQYF | TRB J2-7 | TRAV12 -1 | CVVNRGSSYKLI F | TRA J12 | 29298 | patie nt#3 | recombin ant S protein | Low/ Nega | Mediu m | Low/ Nega | DB2_CAGGTGCTCT CCGGTT |
| TRBV1 1-3 | CASSLIRGYEQYF | TRB J2-7 | TRAV38 -1 | CAFMKHVDSGA GSYQLTF | TRA J28 | 31105 | patie nt#3 | Peptide pool M+N | Low/ Nega | Mediu m | Low/ Nega | DB2_ACGCCGATCT CGTATT |
| TRBV1 1-2 | CASSLRGRNIQYF | TRB J2-4 | TRAV26 -1 | CIVKKLTGGGN KLTF | TRA J10 | 44828 | patie nt#3 | inactivat ed virus | Low/ Nega | Low/ Nega | Low/ Nega | DB2_CATTCGCAGT CATCCA |
| TRBV6-2 | CASSSTSGSTYPT EQFF | TRB J2-1 | TRAV13 -1 | CAASISNTGNQF YF | TRA J49 | 2571 | patie nt#3 | inactivat ed virus | Low/ Nega | Mediu m | High | DB2_CATTCGCAGT ACGCCC |
| TRBV1 1-2 | CASSLDPVSETQY F | TRB J2-5 | TRAV26 -1 | CIVKKLTGGGN KLTF | TRA J10 | 38007 | patie nt#3 | inactivat ed virus | Low/ Nega | Low/ Nega | Low/ Nega | DB2_CGTTCTGTCA TCTGTT |

**[Table 3-7]**

| | | | | | | | | | | | | |
|---|---|---|---|---|---|---|---|---|---|---|---|---|
| TRBV7-9 | CASSLGDSGNTIY F | TRB J1-3 | TRAV8-4 | CAVINSGGYQK VTF | TRA J13 | 36754 | patie nt#3 | Peptide pool M+N | Mediu m | Low/ Nega | Low/ Nega | DB2_CTCGTACCAG GGTACA |
| TRBV2 8 | CASSLAEGGHGN TIYF | TRB J1-3 | TRAV8-4 | CAVRAGTASKLT F | TRA J44 | 50955 | patie nt#3 | inactivat ed virus | Low/ Nega | Low/ Nega | Low/ Nega | DB2_CGGAGTCTCG GCGGTT |
| TRBV6-1 | CASSVYYGYTF | TRB J1-2 | TRAV29 ZDV5 | CAASRLGTASKL TF | TRA J44 | 26565 | patie nt#3 | inactivat ed virus | Mediu m | Low/ Nega | Low/ Nega | DB2_CGGAGTCCAG CCAGAA |
| TRBV7-9 | CASSLVGEQYF | TRB J2-7 | | | | 29162 | patie nt#3 | recombin ant S protein | Low/ Nega | Low/ Nega | Low/ Nega | DB2_CGTCTACAGA GTACCG |
| TRBV1 8 | CASSRSLGLMNT EAFF | TRB d 1-1 | | | | 15086 | patie nt#3 | inactivat ed virus | Low/ Nega | Low/ Nega | Low/ Nega | DB2_CCCTCCTAGA GCAATT |
| TRBV4-2 | CASSQADRGESP LHF | TRB 11-6 | TRAVS-4 | CAVSDRGSARQ LTF | TRA J22 | 19609 | patie nt#3 | inactivat ed virus | Low/ Nega | Low/ Nega | Low/ Nega | DB2_CCAGCGAAGC TAACTC |
| TRBV1 4 | CASSRTSGWTDT QYF | TRB J2-3 | TRAV8-3 | CAVGARTGRRA LTF | TRA J5 | 8189 | patie nt#3 | inactivat ed virus | Low/ Nega | Low/ Nega | Low/ Nega | DB2_CCAATCCGTT CACGGC |
| TRBV2 8 | CASSTMSGVETQ YF | TRB J2-5 | TRAV19 | CALSANTGRRA LTF | TRA J5 | 37164 | patie nt#3 | Peptide pool S | Mediu in | Low/ Nega | Low/ Nega | DB2_CGCCAAGCAT ATACGC |
| TRBV5-4 | CASRAAGTGAWG YTF | TRB J1-2 | TRAV38 -1 | CAFMKYNNND MRF | TRA J43 | 20875 | patie nt#4 | inactivat ed virus | Low/ Nega | Low/ Nega | Low/ Nega | DB3_GCTTGAAAGG CTAGCA |
| TRBV5-5 | CASSRGLAGIPTG ELFF | TRB J2-2 | TRAV29 /DV5 | CAASPMNRDDK IIF | TRA J30 | 5395 | patie nt#4 | Peptide pool M+N | High | Mediu m | Low/ Nega | DB3_GCACATACAT AGGATA |
| TRBV2 0-1 | CSARLLGQGASY EQYF | TRB J2-7 | TRAV8-3 | CQKLLF | TRA J16 | 47226 | patie nt#4 | Peptide pool M+N | Mediu m | Mediu m | Low/ Nega | DB3_GGAGCAACAA ACGTGG |
| TRBV6-1 | CASSEFGTGEQFF | TRB J2-1 | TRAV26 -1 | CIVRVKGGGSN YKLTF | TRA J53 | 42080 | patie nt#4 | inactivat ed virus | Low/ Nega | Low/ Nega | Low/ Nega | DB3_GGCCGATCAC ATCCAA |

**[Table 3-8]**

| | | | | | | | | | | | | |
|---|---|---|---|---|---|---|---|---|---|---|---|---|
| TRBV6-2 | CASTRGVTDTQY F | TRB J2-3 | TRAV36 /DV7 | CADLTQGGSEK LVF | TRA J57 | 45445 | patie nt#4 | Peptide pool M+N | Low/ Nega | Low/ Nega | High | DB3_GGCTGGTGTA GTGAAT |
| TRBV2 | CASSPHDYEQFF | TRB J2-1 | TRAV22 | CAVKGLGSTGN QFYF | TRA J49 | 27381 | patie nt#4 | Peptide pool M+N | Low/ Nega | Low/ Nega | Mediu in | DB3_GGGTTGCTCC GTACAA |
| TRBV2 0-1 | CSALPAGGRNEQ YF | TRB J2-7 | TRAV36 /DV7 | CAVASQGGSEK LVF | TRA J57 | 47821 | patie nt#4 | inactivat ed virus | Low/ Nega | Low/ Nega | High | DB3_GTAGGCCAGA TGTTAG |
| TRBV1 1-2 | CASSPTYEQYF | TRB J2-7 | TRAV12 -1 | CVVNRGSSYKLI F | TRA J12 | 29298 | patie nt#4 | Peptide pool S | Low/ Nega | High | Low/ Nega | DB3_GTCACAAGTG AAAGAG |
| TRBV7-2 | CASSLDGARSYN EQFF | TRB J2-1 | TRAV21 | CAVGSNYQLIW | TRA J33 | 23764 | patie nt#4 | recombin ant 9 protein | Low/ Nega | Low/ Nega | Mediu in | DB3_TAGTGGTCAG CGATCC |
| TRBV5-1 | CASAVGGLTNEK LFF | TRB J1-4 | TRAV20 | CAVGFQGGSEK LVF | TRA J57 | 10942 | patie nt#4 | recombin ant S protein | Low/ Nega | Low/ Nega | Low/ Nega | DB3_TACGGTAAGG AGCGAG |
| TRBV1 0-3 | CAITRDTDGYTF | TRB J1-2 | TRAV12 -1 | CVVNNARLMF | TRA J31 | 41543 | patie nt#4 | inactivat ed virus | Low/ Nega | Low/ Nega | Low/ Nega | DB3_TACTTGTGTA GTACCT |
| TRBV1 2-3 | CASSLVTGSTEAF F | TRB J1-1 | TRAV12 -3 | CAMSRPGAGSY QLTF | TRA J28 | 34575 | patie nt#4 | recombin ant S protein | High | Low/ Nega | Low/ Nega | DB3_TAGCCGGCAT GGGACA |
| TRBV7-3 | CASSLAGGSTDT QYF | TRB J2-3 | TRAV2 | CASHNAGNMLT F | TRA J39 | 17390 | patie nt#4 | Peptide pool S | Low/ Nega | Low/ Nega | Low/ Nega | DB3_TCAGGTAGTG GCAAAC |
| TRBV7-2 | CASSPMGDSADT QYF | TRB J2-3 | TRAV8-1 | CAVNAGRRALT F | TRA J5 | 17435 | patie nt#4 | Peptide pool S | Low/ Nega | Low/ Nega | Mediu m | DB3_TCCACACGTG ATGCCC |
| TRBV2 5-1 | CASSPGSGHNEQ FF | TRB J2-1 | TRAV9-2 | CALSGGWDSGY STLTF | TRA J11 | 34790 | patie nt#4 | recombin ant S protein | Low/ Nega | High | Low/ Nega | DB3_TGACGGCCAA GTACCT |

**[Table 3-9]**

| | | | | | | | | | | | | |
|---|---|---|---|---|---|---|---|---|---|---|---|---|
| TRBV1 1-2 | CASSLGIDEQFF | TRB J2-1 | TRAV23 /DV6 | CAAKGGSQGNL IF | TRA J42 | 27964 | patie nt#4 | Peptide pool M+N | Mediu m | High | Low/ Nega | DB3_TGCACCTAGC AATATG |
| TRBV4-3 | CASSQERVSGYE QYF | TRB J2-7 | TRAV13 -1 | CAASISGSARQL TF | TRA J22 | 16803 | patie nt#4 | Peptide pool S | Mediu m | Low/ Nega | Low/ Nega | DB3_GCACATACAG ACGCAA |
| TRBV2 0-1 | CSARDIGGGTSPL HF | TRB J1-6 | TRAV23 /DV6 | CAATSATSGTYK YIF | TRA J40 | 19940 | patie nt#4 | Peptide pool S | Mediu m | Low/ Nega | Low/ Nega | DB3_TTCTACAAGT AGATGT |
| TR.BV1 1-2 | CASSPTYEQYF | TRB J2-7 | TRAV12 -1 | CVVNRGSSYKLI F | TRA J12 | 29298 | patie nt#4 | Peptide pool S | Low/ Nega | High | Low/ Nega | DB3_TACAGTGCAG GAATCG |
| TRBV5-1 | CASSAMVGQETQ YF | TRB J2-5 | TRAV13 -1 | CALGDSGYALN F | TRA J41 | 35856 | patie nt#4 | inactivat ed virus | Low/ Nega | Low/ Nega | Low/ Nega | DB3_GAGTCCGCAG ACGCAA |
| TRBV2 | CASRKADRRQGLI RSYEQYF | TRB J2-7 | TRAV1-2 | CARGKPGANNL FF | TRA J36 | 1220 | patie nt#4 | Peptide pool S | Low/ Nega | Low/ Nega | High | DB3_CGATTGAAGG ATATAC |
| TRBV6-5 | CASRPEKRSGNTI YF | TRB J1-3 | TRAV9-2 | CALSPSILYYGQ NFVF | TRA J26 | 15305 | patie nt#4 | Peptide pool S | Mediu m | High | Mediu m | DB3_GAAGCAGCAC CAGCAC |
| TRBV1 1-3 | CASSLGGQETQY F | TRB J2-5 | TRAV9-2 | CAPHTGGTSYG KLTF | TRA J52 | 31107 | patie nt#4 | Peptide pool S | Low/ Nega | LowY Nega | High | DB3_TTTGGTTGTT CGCGAC |
| TRBV2 | CASSAHRGDEKL FF | TRB J1-4 | TRAV12 -1 | CVVNNPNAGN MLTF | TRA J39 | 32292 | patie nt#4 | recombin ant S protein | Low/ Nega | Low/ Nega | Low/ Nega | DB3_ACGAGGAGTA GCGTGA |
| TRBV4-3 | CASSQERVSGYE QYF | TRB J2-7 | TRAV13 -1 | CAASISGSARQL TF | TRA J22 | 16803 | patie nt#4 | Peptide pool S | High | Low/ Nega | Low/ Nega | DB3_AGCATACAGT TAACGA |
| TRBV4-3 | CASSQDIGESPLH F | TRB J1-6 | TRAV8-3 | CAVTSGTYKYIF | TRA J40 | 30270 | patie nt#4 | inactivat ed virus | Low/ Nega | High | Low/ Nega | DB3_AGTGTCACAG CTTAAC |
| TRBV1 1-2 | CASSSSPGTSGTG TGELFF | TRB J2-2 | TRAV8-3 | CAVGARWTGNQ FYF | TRA J49 | 781 | patie nt#4 | Peptide pool S | Mediu m | High | Low/ Nega | DB3_CAACTAGCAT TCCTCG |

**[Table 3-10]**

| | | | | | | | | | | | | |
|---|---|---|---|---|---|---|---|---|---|---|---|---|
| TRBV4-2 | CASSAQGEGTEA FF | TRB J1-1 | TRAV17 | CATGSGYSTLTF | TRA J11 | 32454 | patie nt#4 | recombin ant S protein | Low/ Nega | High | Low/ Nega | DB3_CATGGCGAGC AGCGTA |
| TRBV2 0-1 | CSARSPRGGAYE QYF | TRB J2-7 | | | | 48449 | patie nt#4 | Peptide pool S | Low/ Nega | Low/ Nega | High | DB3_CGATGGCTCA GCGATT |
| TRBV5-4 | CASSFGGPTSYEQ YF | TRB J2-7 | TRAV12 -1 | CVVEDTGRRAL TF | TRA J5 | 15992 | patie nt#4 | inactivat ed virus | Low/ Nega | Low/ Nega | High | DB3_CGCCAAGAGC CAGTTT |
| TRBV6-1 | CASTPDKEYNQP QHF | TRB J1-5 | TRAV10 | CVVSGNTGGFK TIF | TRA J9 | 19544 | patie nt#4 | Peptide pool M+N | Low/ Nega | Low/ Nega | High | DB3_CCTCAGTGTT CAGTAC |
| TRBV1 9 | CASSMRAGEGGE TQYF | TRB J2-5 | | | | 51817 | patie nt#4 | inactivat ed virus | Low/ Nega | Low/ Nega | Low/ Nega | DB3_CGCGTTTTCT GGAGCC |
| TRBV2 | CASRPQGNGGYT F | TRB J1-2 | | | | 31602 | patie nt#4 | Peptide pool S | High | Low/ Nega | Low/ Nega | DB3_CGGAGCTAGA TCCCAT |
| TRBV1 2-4 | CASRPRDRSNSPL HF | TRB dl-6 | TRAV9-2 | CALSQNTGGFK TIF | TRA J9 | 20424 | patie nt#4 | inactivat ed virus | Low/ Nega | Low/ Nega | High | DB3_CGTAGCGGTT TGGCGC |
| TRBV3 0 | CAWSPLGSGDEK LFF | TRB J1-4 | TRAV9-2 | CALSGQAGTALI F | TRA J15 | 24920 | patie nt#4 | Peptide pool S | Mediu m | Low/ Nega | Low/ Nega | DB3_CGTCCATTCG TACGGC |
| TRBV5-1 | CASSLRQGGTGE LFF | TRB J2-2 | | | | 13710 | patie nt#4 | recombin ant S protein | Low/ Nega | Low/ Nega | Low/ Nega | DB3_CTACACCTCG TTTAGG |
| TRBV1 1-2 | CASSLGTEETQYF | TRB J2-5 | TRAV30 | CGTERTGGTSY GKLTF | TRA J52 | 44725 | patie nt#4 | Peptide pool M+N | High | High | Low/ Nega | DB3_CTACGTCCAT CTCCCA |
| TRBV6-5 | CASSYSGQGRSPL HF | TRB J1-6 | TRAV1-2 | CAVKMDSSYKLI F | TRA J12 | 20677 | patie nt#4 | Peptide pool M+N | Mediu m | Low/ Nega | Low/ Nega | DB3_TTTGTCACAT GCCTTC |

**[Table 3-11]**

| | | | | | | | | | | | | |
|---|---|---|---|---|---|---|---|---|---|---|---|---|
| TRBV2 8 | CASSSGQKLAKNI QYF | TRB J2-4 | TRAV27 | CAGPRGSNYKL TF | TRA J53 | 21951 | patie nt#5 | Peptide pool S | Low/ Nega | Low/ Nega | Low/ Nega | DB3_GAGCAGAGTA CTTAGC |
| TRBV4-1 | CASSPGGQGRYQ PQHF | TRB 11-5 | TRAV9-2 | CALSTNAGKST F | TRA J27 | 50390 | patie nt#5 | recombin ant S protein | Low/ Nega | Low/ Nega | Low/ Nega | DB3_CAGCGACGTT ATTCTC |
| TRBV7-3 | CASSSTGGQETQ YF | TRB J2-5 | TRAV13 -1 | CAAFQGAQKLV F | TRA J54 | 38135 | patie nt#5 | inactivat ed virus | Low/ Nega | High | Low/ Nega | DB3_AGTCTTTCAC CGAAAG |
| TRBV1 9 | CASSSPGRQVEAF F | TRB J1-1 | TRAV8-3 | CARIGYSTLTF | TRA J11 | 34375 | patie nt#5 | inactivat ed virus | Mediu m | High | Low/ Nega | DB3_GCAGCCATCA TGTCCC |
| TRBV3-1 | CASSPTLGGASYE QYF | TRB J2-7 | TRAV29 /DV5 | CAARATYSGAG SYQLTF | TRA J28 | 51305 | patie nt#5 | Peptide pool M+N | High | Low/ Nega | Low/ Nega | DB3_ACTGCTCGTC TCCACT |
| TRBV1 1-3 | CASSLHPGQGYE EQYF | TRB J2-7 | TRAV13 -1 | CAASRAGTALIF | TRA J15 | 31056 | patie nt#5 | inactivat ed virus | Low/ Nega | Low/ Nega | Low/ Nega | DB3_TGCCCTAAGA GTAATC |
| TRBV6-1 | CASTTDSAARPQ HF | TRB 11-5 | TRAV12 -1 | CVVNMRSSNTG KLIF | TRA J37 | 38619 | patie nt#5 | recombin ant S protein | Low/ Nega | Mediu m | Low/ Nega | DB3_CTGCGGATCT ATCCTA |
| TRBV5-4 | CASSRTSLADPNE QFF | TRB J2-1 | TRAV5 | CAAEADAGNML TF | TRA J39 | 22454 | patie nt#5 | Peptide pool S | Low/ Nega | Low/ Nega | Low/ Nega | DB3_GGTGAAGCAA GAGGCT |

Among these, TCRαβ pairs shared between patients were detected.

These TCRαβ pairs were designated as clones 1 and 2, derived from patients Ts-017 and Ts-018, respectively, sharing identical Vα, Jα, Vβ, and Jβ usage. Furthermore, it was found that the CDR3α sequences of clones 1 and 2 were identical, and that CDR3β was identical except for one amino acid (Table 4 and Fig. 2). Although clones 1 and 2 were derived from different subjects, they were presumed to recognize the same epitope based on sequence similarity. Furthermore, both were identified from the Tfh cluster, and it was expected that the epitope would be an epitope that easily induces Tfh.

**[Table 4]**

| | TRBV | CDR3β | TRIM | TRAV | CDR3 α | TRAJ | **donor** |
|---|---|---|---|---|---|---|---|
| Clone 1 | TRBV11-2 | CASSQTYEQYF | TRBJ2-7 | TRAV12-1 | CVVNRGSSYKLIF | TRAJ12 | pAtient #3 |
| Clone 2 | TRBV11-2 | CASSPTYEQYF | TRBJ2-7 | TRAV12-1 | CVVNRGSSYKLIF | TRAJ12 | patient #4 |
| Clone 2 | TRBV11-2 | CASSPTYEQYF | TRBJ2-7 | TRAV12-1 | CVVNRGSSYKLIF | TRAJ12 | patient #4 |

In patient Ts-018, clone 2 was detected as two different barcoded cells that share exactly the same TCR sequence. To examine the antigen-specificity of TCR, clonotypes 1 and 2, the respective TCR α and β chains were transfected into TCR-deficient T-cell hybridomas and TCR-reconstituted CD3+ cells were established (Fig. 1B).

These TCR transfectants were stimulated with various antigens in the presence of Epstein-Barr virus (EBV)-transformed B cells derived from each corresponding patient.

Clones 1 and 2 responded to the recombinant S protein and S peptide pools, but not to the M+N peptide pool. This was consistent with the initial antigen-specificity revealed by single cell analysis (Fig. 3A).

Activation was excluded in the absence of antigen-presenting cells, confirming that activation is mediated by MHC molecules (Fig. 3B).

Of the two pooled vials #1 (S1-643) and #2 (S633-1273), only the latter pool stimulated clones 1 and 2. It was found that the antigenic peptide was contained at positions 633-1273 of the S protein (Fig. 3C).

However, another available SARS-CoV-2 S peptide pool (PepTivator (registered trademark)) (Fig. 4) containing 175 peptides corresponding to positions 304-338, 421-475, 492-519, 683-707, 741-770, 785-802, and 885-1273 did not activate clones 1 and 2 (Fig. 3D).

These results suggested that the antigenic epitopes of clone 1 and clone 2 were located at amino acids 633-682, 708-740, 771-784, or 803-884 of the S protein (Fig. 4).

The HLA alleles constraining clones 1 and 2 were then determined to further reduce the number of peptide candidates for the epitope.

It was found that antigen-presenting cells (APCs) from both patients Ts-017 and Ts-018 can activate clones 1 and 2. This indicates that APCs are interchangeable (Fig. 3E).

Therefore, each of the MHC class II alleles shared between Ts-017 and Ts-018, such as DRB1*15:01, DPA1*02:02-DPB1*05:01, DQA1*01:02-DQB1*06, and the like, is considered an HLA candidate. These alleles were then individually transfected to examine recognition of the S peptide by clones 1 and 2.

Both clones 1 and 2 responded to S peptide pool #2 only in the presence of DRA*01:01 and DRB1*15:01, and DRA was monomorphic, suggesting that the responsiveness is determined by DRB1*15:01 (Fig. 5B).

Therefore, NetMHC server software (http://www.cbs.dtu.dk/services/NetMHCIIpan/) was used to search for candidate peptides predicted to bind to DRA-DRB1*15:01 (Fig. 5C).

Two strong binders (S₈₂₈₋₈₄₆ and S₈₆₄₋₈₈₂) were predicted as candidates within narrowed regions of specific viral proteins (Fig. 5D). Therefore, by synthesizing two peptides and stimulating clones 1 and 2, peptide S₈₆₄₋₈₈₂ was determined as an epitope for both clones 1 and 2 (Fig. 5E).

TCRs of clones 1 and 2 differ by one amino acid in CDR3 (Pro and Gln at position 89). Since both TCRs recognize the same epitope presented by the same MHC molecule and are from different donors, it was suggested that clones 1 and 2 are public clones. This epitope has low homology with the corresponding amino acid sequence of human coronavirus (HCoV)-OC43.

Indeed, neither clone 1 nor 2 responded at all to peptide pools and total proteins derived from HCoV-OC43S protein in the presence of APC expressing DRA-DRB1*15:01 (Fig. 6A). Furthermore, since none of the other HCoVs, including severe acute respiratory syndrome coronavirus (SARS-CoV) and Middle East respiratory syndrome-associated coronavirus (MERS-CoV), have similar epitopes (Fig. 6B), it was suggested that clone 1/2 is unlikely to cross-react with related human coronaviruses.

It is next examined whether MHC class II alleles other than DRB1*15:01 can present this antigenic peptide recognized by clone 1/2.

As a result, S₈₆₄₋₈₈₂ was also predicted as a strong binder to DRB1*15:02 and 15:01 (Fig. 6C). Furthermore, the amino acid sequence identity between DRB1*15:01 and *15:02 is 99.6%. Therefore, it is speculated that DRB1*15:02 is also the allele responsible for epitope recognition, and APC from another individual who has DRB1*15:02 but not DRB1*15:01 was tested. As a result, it was found that DRB1*15:02 can stimulate both clones 1 and 2 in the presence of S₈₆₄₋₈₈₂ peptide (Fig. 6D) .

Considering the frequency of DRB1*15:02 (10.6%) and *15:01 (7.7%) in the Japanese population (http://hla.or.jp/), if these clonotypes were present, clones 1 and 2 were expected to respond to this peptide in 18.3% of the population. In this Example, clone 1/2 was confirmed in 2 out of 6 patients (33%).

Next, the occurrence of the clone 2/3 clonotype in Japanese blood donor samples before the SARS-CoV-2 pandemic was examined. It was found that 9 out of 27 individuals had the clone 1/2 TCRβ clonotype. This is similar to the ratio in this Example.

Next, considering that clone 1/2 is SARS-CoV-2-specific T cell exhibiting a Tfh profile, it is hypothesized that clone 1/2 was clonally expanded during infection to promote protective humoral immunity and the following examination was performed.

DRB1*15:01/15:02 is not a minor allele in the world (see http://www.allelefrequencies.net/tools/Report.aspx). Thus, frequency of clone 1/2 clonotypes in healthy individuals (n=786) and individuals (n=1,413) who have recovered from COVID-19 was examined in a public database of US citizens (immune response actions to COVID-19 events; ImmuneRace, https://immunerace.adaptivebiotech.com/). Clonotypes were detected at a certain frequency in each of the healthy group and the recovered group. Clonotypes were detected in approximately 17% and 24% of the healthy and COVID-19 recovered groups, respectively (Fig. 6E, top). Notably, the frequency of this clonotype among each individual was significantly increased (p=0.0028) in the recovered group compared to healthy controls, suggesting that this S protein-specific clone is clonally expanded in different individuals during infection (Fig. 4E, bottom). Interestingly, the frequency of clonotype 1/2 in recovered patients is greater than the allele frequency of DRB1*15:01 or 15:02 worldwide (11.3%). Thus, it is possible that clone 1/2 recognized other epitopes derived from SARS-CoV-2 presented by other HLA alleles.

Taken together, the first identification of public T cell clones that recognize SARS-CoV-2 and its epitopes was considered to contribute to the development of diagnostic strategies or options and next-generation vaccines.

### (Example 3)

### Restimulation assay of peripheral blood mononuclear cell (PBMC) by peptide of Example 1

PBMC 1.0×10⁵ cells obtained from experiment participants in Example 1 are suspended in 100 µl of RPMI1640 medium (added with 5% human serum and penicillin/streptomycin), Example 1 peptide is added to 1 µg/ml, and the cells are cultured in a 96-well plate (U bottom) (Day 0).

Next, human IL-2 is added at 10 IU/ml on Day 4 and Day 7. Passage of the medium is performed as appropriate.

On Day 10, the cells are sorted as follows using cell sorter SH-800S (SONY). The cells are stained using CD3, CD4 antibody and PI, and sorted in the order of FSC SSC gating, PI negative gating, CD3 positive gating, and CD4 positive cells. At that time, staining is performed using CXCR5, CD45RA, and PD-1 antibodies, and FCM analysis is also performed.

CD4 positive cells (2.0×10⁴ cells) obtained by sorting and B cells (3.0×10³ cells) derived from a patient immortalized by EBV infection are suspended in 50 µl of RPMI1640 medium (5% human serum) and cultured in multiple wells using a 96-well plate (U bottom) . To half of them, the SARS-CoV-2 S₈₆₄₋₈₈₂ peptide is added to the medium at 1 µg/ml.

After 24 hr, the supernatant of the medium is collected and human IL-21 is measured using ELISA.

The measurement results show that when stimulated with the peptide of Example 1, the concentration of IL-21 in the medium is higher than when it is not stimulated. This result suggests that the peptide stimulation of Example 1 promotes the formation of germinal centers and the production of anti-affinity antibodies by follicular T cells.

### (Example 4)

### Restimulation assay of peripheral blood mononuclear cell (PBMC) by SARS-CoV-2 S₈₆₄₋₈₈₂ peptide

PBMC 1.0×10⁵ cells obtained from experiment participants were suspended in 100 µl of RPMI1640 medium (added with 5% human serum and penicillin/streptomycin), SARS-CoV-2 S₈₆₄₋₈₈₂ peptide was added to 1 µg/ml, and the cells were cultured in a 96-well plate (U bottom) (Day 0).

Next, human IL-2 was added at 10 IU/ml on Day 4 and Day 7. Passage of the medium was performed as appropriate.

On Day 10, the cells were sorted as follows using cell sorter SH-800S (SONY). The cells were stained using CD3, CD4 antibody and PI, and sorted in the order of FSC SSC gating, PI negative gating, CD3 positive gating, and CD4 positive cells. At that time, staining was performed using CXCR5, CD45RA, and PD-1 antibodies, and FCM analysis was also performed.

CD4 positive cells (2.0×10⁴ cells) obtained by sorting and B cells (3.0×10³ cells) derived from a patient immortalized by EBV infection were suspended in 50 µl of RPMI1640 medium (5% human serum) and cultured in multiple wells using a 96-well plate (U bottom). To half of them, the SARS-CoV-2 S₈₆₄₋₈₈₂ peptide was added to the medium at 1 µg/ml.

After 24 hr, the supernatant of the medium was collected and human IL-21 is measured using ELISA. The measurement results are shown in Fig. 7.

The measurement results show that when stimulated with SARS-CoV-2 S₈₆₄₋₈₈₂ peptide, the concentration of IL-21 in the medium was higher than when it was not stimulated. This result suggests that the SARS-CoV-2 S₈₆₄₋₈₈₂ peptide stimulation promoted the formation of germinal centers and the production of anti-affinity antibodies by follicular T cells.

### (Example 5: Evaluation of infection history)

In this Example, the infection history is evaluated.

Infection history evaluation using antibodies is often performed. Identification of infection source-specific immune cells is particularly useful in investigating infection history, since specific immune cells are maintained for longer periods of time than antibodies. T cell detection method using a probe containing infection source-specific epitope is useful as a convenient method for detecting immune cells established and maintained after infection. The presence or absence of T cells that react with the probe can be easily detected by a system using ELISPOT or flow cytometry using peripheral blood mononuclear cells (PBMC). When probe-reactive T cells are detected, past infection is determined.

### (Material and method)

### (Material)

### For influenza virus

Influenza A, Hemagglutinin, and Brisbane (funakoshi) are used as antigens.

### For enterohemorrhagic Escherichia coli infection

Lipopolysaccharide derived from E. coli 0157 (Wako) is used as an antigen.

### For fungal pneumonia

Standard strain (KWIK-STIK 2pack) Aspergillus fumigatus derived from ATCC 204305 01021P (As One) is used as an antigen.

### (Protocol)

The presence or absence of infection source-specific immune cells in the subject's peripheral blood is examined using an ELISPOT assay. The ELISPOT assay used here uses a plate coated with an antibody that can detect IL-21 produced by follicular T cells. Peripheral blood mononuclear cells (PBMC) are added to the wells and the cells are stimulated with the antigens mentioned above for 36 hr. Secreted IL-21 binds to capture antibodies around the producing cells. After removing cells by washing, a cytokine antibody for detection is added to detect spots. As a negative control, an assay without stimulation with antigen derived from the infection source is performed.

### (Result)

When secretion of IL-21 is confirmed, an infection history can be determined.

### (Example 6 : Evaluation of vaccine efficacy · evaluation of re-contraction defensive ability)

In the present Example, evaluation of vaccine efficacy evaluation of re-contraction defensive ability are performed.

Infection source-specific immune cells can be used to evaluate defensive ability of immune cell since they are maintained for a longer period of time than antibodies and can respond quickly upon reinfection. Among many T cell fractions, follicular T cells are important for the induction and enhancement of humoral immunity (affinity maturation). Thus, in particular, subjects with T cells that react with a probe containing the infection source epitope that induces follicular T cells are judged to have stronger or qualitatively superior infection defensive ability. Therefore, it is considered effective to examine the presence or absence of probe-reactive cells in PBMC as an evaluation method of immunity established after infection or vaccination. As for the evaluation method, ELISPOT or flow cytometry can be used in the same manner as above. By changing the cytokines to be detected or by combining surface antigen probes, these methods can detect whether and what percentage of T cells that responded to epitope probes are actually follicular T cells. It is also possible to design an experimental system according to detection sensitivity and judgment criteria.

### (Material and method)

### (Material)

### For influenza virus

Influenza A, Hemagglutinin, and Brisbane (funakoshi) are used as antigens.

### For enterohemorrhagic Escherichia coli infection

Lipopolysaccharide derived from E. coli 0157 (Wako) is used as an antigen.

### For fungal pneumonia

Standard strain (KWIK-STIK 2pack) Aspergillus fumigatus derived from ATCC 204305 01021P (As One) is used as an antigen.

### (Protocol)

The presence or absence of infection source-specific immune cells in the subject's peripheral blood is examined using an ELISPOT assay. The ELISPOT assay used here uses a plate coated with an antibody that can detect IL-21 produced by follicular T cells. Peripheral blood mononuclear cells (PBMC) are added to the wells and the cells are stimulated with the antigens mentioned above for 36 hr. Secreted IL-21 binds to capture antibodies around the producing cells. After removing cells by washing, a cytokine antibody for detection is added to detect spots. As a negative control, an assay without stimulation with antigen derived from the infection source is performed.

### (Result)

When secretion of IL-21 is confirmed, the defensive ability of immune cell can be determined to be effective.

### (Example 7: Effectiveness evaluation of cancer immunodrugs (immune checkpoint and the like))

In this Example, the effectiveness of cancer immunodrugs (immune checkpoint and the like) is evaluated.
Higher antitumor properties are expected if a humoral immune response to cancer antigens is elicited. Therefore, subjects with more follicular T cells that induce antitumor humoral immune responses are more likely to activate their antitumor immunity, and drugs such as immune checkpoint inhibitors that enhance their antitumor immunity are considered to be more effective.

By evaluating the reactivity of T cells obtained from tumor biopsy samples or peripheral blood with epitope probes, it is possible to evaluate the degree of antitumor immune activity in subjects and to help select treatment methods. In addition, by combining epitope probes and flow cytometry to evaluate the activation and exhaustion of epitope-specific follicular T cells (tumor-specific follicular T cells) during drug administration, it is expected to examine whether antitumor immune activity is maintained. This is useful in determining whether immune checkpoint inhibitors will continue to be effective or whether treatment should be discontinued and another treatment method should be sought.

### (Material and method)

### (Material)

### For lung cancer

A lung cancer marker CEA is used as an antigen.

### For gastric cancer

A gastric cancer marker CA19-9 is used as an antigen.

### (Protocol)

The presence or absence of antigen-specific immune cells in the subject's peripheral blood is examined using an ELISPOT assay. The ELISPOT assay used here uses a plate coated with an antibody that can detect IL-21 produced by follicular T cells. Peripheral blood mononuclear cells (PBMC) are added to the wells and the cells are stimulated with the antigens mentioned above for 36 hr. Secreted IL-21 binds to capture antibodies around the producing cells. After removing cells by washing, a cytokine antibody for detection is added to detect spots. As a negative control, an assay without stimulation with antigen is performed.

### (Result)

When an increase in IL-21 secretion is confirmed as compared to the negative control, it can be determined that the cancer immunodrug acts effectively.

### (Example 8: Risk evaluation of autoimmune disease associated with autoantibody (predisease period), pathology evaluation)

In this Example, risk evaluation of autoimmune disease associated with autoantibody (predisease period), and pathology evaluation are performed.

There are many autoimmune diseases that involve autoantibodies, among which in diseases where autoantibodies are considered pathogenic (e.g., ANCA-associated vasculitis), the presence or absence·increase or decrease of follicular T cells that interact with B cells that produce autoantibodies may lead to the onset or aggravation/relapse of the disease as the B cells mature. Therefore, it is considered that disease risk (before onset) and disease activity can be evaluated by detecting follicular T cells that present epitopes derived from disease-specific self-antigens.

The test method is considered to involve examining probe-reactive cells in PBMC collected from subjects, by using ELISPOT or flow cytometry.

### (Material and method)

### (Material)

### For atopic dermatitis

An autoantibody that induces atopic dermatitis is used as an antigen.

### For allergic rhinitis

An autoantibody that induces allergic rhinitis is used as an antigen.

### For asthma

An autoantibody that induces asthma is used as an antigen.

### (Protocol)

The presence or absence of antigen-specific immune cells in the subject's peripheral blood is examined using an ELISPOT assay. The ELISPOT assay used here uses a plate coated with an antibody that can detect IL-21 produced by follicular T cells. Peripheral blood mononuclear cells (PBMC) are added to the wells and the cells are stimulated with the antigens mentioned above for 36 hr. Secreted IL-21 binds to capture antibodies around the producing cells. After removing cells by washing, a cytokine antibody for detection is added to detect spots. As a negative control, an assay without stimulation with antigen derived from the infection source is performed.

### (Result)

When an increase in IL-21 secretion is confirmed compared to the negative control, it can be determined that the disease risk (before onset) and disease activity are high.

### (Example 9: Digital health application example)

As discussed in previous examples, epitope-specific follicular T cell responses can be applied to a variety of diseases. On the other hand, it is also true that there are diseases for which the epitope or antigen itself is unknown, and for this reason, it is considered that data on follicular T cell responses to various epitopes, including known disease-related epitopes, in various patients should be shared within the research period and used as big data as a basis for obtaining new knowledge.

The test method is considered to involve examining probe-reactive cells in PBMC collected from subjects, by using ELISPOT or flow cytometry.

### (Material and method)

### (Material)

Systemic lupus erythematosus, for which the specific antigen is unknown, is considered as a target disease.

In this respect, a panel of epitopes derived from known infectious disease antigens and randomly prepared epitopes is used to evaluate the responses and levels of follicular T cells to individual epitopes.

### (Protocol)

The presence or absence of antigen-specific immune cells in the subject's peripheral blood is examined using an ELISPOT assay. The ELISPOT assay used here uses a plate coated with an antibody that can detect IL-21 produced by follicular T cells. Peripheral blood mononuclear cells (PBMC) are added to the wells and the cells are stimulated with the antigens mentioned above for 36 hr. Secreted IL-21 binds to capture antibodies around the producing cells. After removing cells by washing, a cytokine antibody for detection is added to detect spots. As a negative control, an assay without stimulation with antigen derived from the infection source is performed.

### (Results)

Negative control and IL-21 secretion information and clinical information for each epitope will be shared and stored as data, and used for future diagnostic method development, disease subdivision, and the development of treatment selection method.

Note that the present disclosure is not limited to the above embodiments, and various modifications can be made within the scope of the gist of the present disclosure.

### (Note)

As described above, while the present disclosure has been illustrated using the preferred embodiments of the present disclosure, the present disclosure should not be construed as being limited to those embodiments. It is understood that the present disclosure is to be construed in scope only by the claims. It is understood that a person skilled in the art can implement an equivalent range from the description of specific preferred embodiments of the present disclosure based on the description of the present disclosure and common general technical knowledge. It is also understood that any patent, any patent application, and any references cited herein should be incorporated herein by reference in the same manner as the contents are specifically described herein. This application is based on a patent application No. 2021-42670 filed in Japan (filing date: March 16, 2021), the contents of which are incorporated in full herein by reference.

### [Industrial Applicability]

The present disclosure can be used in fields such as vaccine development, cell therapy, diagnostic technology, and the like.

## Claims

1. A method for testing an item relating to a disease in a subject, comprising measuring a level or amount of a follicular T cell reactive with a disease-related factor of the disease in the subject, and comparing the level or amount with a predetermined standard.

2. The method according to claim 1, wherein the standard relates to at least one selected from the group consisting of the history of the disease, the effectiveness of a prophylactic agent or vaccine against the disease, the effectiveness of a therapeutic agent against the disease, defensive ability against re-contraction of the disease, the pathology of the disease, and the risk of contracting the disease.

3. The method according to claim 1 or 2, wherein the standard relates to the disease in the subject.

4. The method according to claim 1, wherein the standard relates to the infection history of the disease in the subject.

5. The method according to claim 1, wherein the standard relates to the vaccine effectiveness and re-contraction defensive ability against the disease in the subject.

6. The method according to claim 1, wherein the standard relates to the effectiveness evaluation of a cancer immunodrug against the disease in the subject.

7. The method according to claim 1, wherein the disease-related factor is an autoimmune disease-related factor, the measurement comprises a measurement of the level or amount of follicular T cells reactive with the autoimmune disease-related factor, and the standard relates to the risk and pathology of an autoimmune disease associated with an autoantibody of the subject.

8. The method according to any one of claims 1 to 7, wherein the measurement is performed by ELISPOT, flow cytometry, peripheral blood mononuclear cell (PBMC) restimulation assay, next generation sequencer/genetic testing (repertoire analysis), immunostaining (histopathological examination), or in situ analysis.

9. The method according to any one of claims 1 to 8, wherein the comparison further comprises comparing the measured level, spatial distribution, or amount of follicular T cells reactive with the disease-related factor with a mean or median value of the level, spatial distribution, or amount of the follicular T cell reactive with the disease-related factor of a test subject not affected with the disease.

10. The method according to any one of claims 1 to 6, 8 and 9, wherein the disease is an infectious disease or cancer.

11. The method according to any one of claims 1 to 6, 8 and 9, wherein the disease is a viral infectious disease.

12. The method according to any one of claims 1 to 9, wherein the disease is an allergy or an autoimmune disease.

13. A reagent or device for evaluating a disease in a subject, comprising a reagent or a device for measuring a level, spatial distribution, or amount of a follicular T cell reactive with a disease-related factor of the disease in the subject.

14. The reagent or device according to claim 13, comprising a reagent that specifically reacts with a follicular T cell.

15. The reagent or device according to claim 13, wherein the device is used to perform ELISPOT, flow cytometry, peripheral blood mononuclear cell (PBMC) restimulation assay, next generation sequencer/genetic testing (repertoire analysis), immunostaining (histopathological examination), or in situ analysis.

16. The reagent or device according to any one of claims 13 to 15, wherein the disease is an infectious disease or cancer.

17. The reagent or device according to any one of claims 13 to 15, wherein the disease is a viral infectious disease.

18. The reagent or device according to any one of claims 13 to 15, wherein the disease is an allergy or an autoimmune disease.

19. A system for evaluating a disease in a subject, comprising a reagent or a device for measuring a level, spatial distribution, or amount of a follicular T cell reactive with a disease-related factor of the disease in the subject, and
an evaluation unit that evaluates measurement results by the reagent or device with regard to the disease.

20. The system according to claim 19, wherein the evaluation unit is configured to perform at least one selected from the group consisting of evaluation of the history of the disease, evaluation of the effectiveness of a prophylactic agent or vaccine against the disease, evaluation of the effectiveness of a therapeutic agent against the disease, evaluation of defensive ability against re-contraction of the disease, pathology evaluation of the disease, and evaluation of the risk of contracting the disease.

21. The system according to claim 19 or 20, further comprising an inspection and diagnosis unit that inspects or diagnoses the disease in the subject.

22. The system according to claim 19, further comprising an infection history evaluation unit that evaluates a history of infection with the disease in the subject.

23. The system according to claim 19, further comprising a vaccine effectiveness evaluation and re-contraction defensive ability evaluation unit that evaluates effectiveness of vaccine and re-contraction defensive ability against the disease in the subject.

24. The system according to claim 19, further comprising a cancer immunodrug effectiveness evaluation unit that evaluates effectiveness of a cancer immunodrug against the disease in the subject.

25. The system according to claim 19, wherein the disease-related factor is an autoimmune disease-related factor, and the system comprises a follicular T cell measurement unit that measures the level, spatial distribution, or amount of the follicular T cell reactive with the autoimmune disease-related factor, and an autoimmune disease risk evaluation and pathology evaluation unit that performs risk evaluation and pathology evaluation of an autoimmune disease associated with an autoantibody of the subject.

26. The system according to any one of claims 19 to 25, comprising a reagent that specifically reacts with the follicular T cell.

27. The system according to any one of claims 19 to 25,
wherein the device is used to perform ELISPOT, flow cytometry, peripheral blood mononuclear cell (PBMC) restimulation assay, next generation sequencer/genetic testing (repertoire analysis), immunostaining (histopathological examination), or in situ analysis.

28. The system according to any one of claims 19 to 27, further comprising a comparison unit that compares the measured level, spatial distribution, or amount of follicular T cells reactive with the disease-related factor with a mean or median value of the level, spatial distribution, or amount of the follicular T cell reactive with the disease-related factor of a test subject not affected with the disease.

29. The system according to any one of claims 19 to 24 and 26 to 28, wherein the disease is an infectious disease or cancer.

30. The system according to any one of claims 19 to 24 and 26 to 28, wherein the disease is a viral infectious disease.

31. The system according to any one of claims 19 to 24 and 26 to 28, wherein the disease is an allergy or an autoimmune disease.

32. A method for testing an item relating to a disease in a subject, comprising a step of providing information relating to a level, spatial distribution, or amount of a follicular T cell reactive with a disease-related factor of the disease in the subject, and a step of comparing information relating to the level, spatial distribution, or amount of the follicular T cell with a predetermined standard.

33. The method according to claim 32, wherein the standard relates to at least one selected from the group consisting of the presence or absence of history of the disease, the effectiveness of a prophylactic agent or vaccine against the disease, the effectiveness of a therapeutic agent against the disease, the presence or absence of defensive ability against re-contraction of the disease, pathology evaluation of the disease, and the risk of contracting the disease.

34. The method according to claim 32, wherein the standard relates to the presence or absence of the infection history of the disease in the subject.

35. The method according to claim 32, wherein the standard relates to the presence or absence of the vaccine effectiveness and re-contraction defensive ability against the disease in the subject.

36. The method according to claim 32, wherein the standard relates to the effectiveness of a cancer immunodrug against the disease in the subject.

37. The method according to claim 32, wherein the disease-related factor is an autoimmune disease-related factor, the measurement comprises a measurement of the level or amount of the follicular T cell reactive with the autoimmune disease-related factor, and the standard relates to the risk and pathology of an autoimmune disease associated with an autoantibody of the subject.

38. The method according to any one of claims 32 to 37, wherein the step of providing information is performed by ELISPOT, flow cytometry, peripheral blood mononuclear cell (PBMC) restimulation assay, next generation sequencer/genetic testing (repertoire analysis), immunostaining (histopathological examination), or in situ analysis.

39. The method according to any one of claims 32 to 38,
wherein the comparison further comprises comparing the information relating to the level, spatial distribution, or amount of follicular T cells reactive with the disease-related factor of the disease in the subject with a mean or median value of the information relating to the level, spatial distribution, or amount of the follicular T cell reactive with the disease-related factor of the disease in a test subject not affected with the disease.

40. The method according to any one of claims 32 to 36, 38 and 39, wherein the disease is an infectious disease or cancer.

41. The method according to any one of claims 32 to 36, 38 and 39, wherein the disease is a viral infectious disease.

42. The method according to any one of claims 32 to 41,
wherein the disease is an allergy or an autoimmune disease.

43. A program comprising a computer readable code for implementing, on a computer, a method for evaluating a disease in a subject, comprising a step of providing information relating to a level, spatial distribution, or amount of a follicular T cell reactive with a disease-related factor of the disease in the subject, and a step of evaluating information relating to the level, spatial distribution, or amount of the follicular T cell with regard to the disease.

44. The program according to claim 43, wherein the step of evaluating further comprises performing at least one selected from the group consisting of evaluation of the history of the disease, evaluation of the effectiveness of a prophylactic agent or vaccine against the disease, evaluation of the effectiveness of a therapeutic agent against the disease, evaluation of defensive ability against re-contraction of the disease, pathology evaluation of the disease, and evaluation of the risk of contracting the disease.

45. The program according to claim 43, wherein the method comprises evaluating the history of infection with the disease in the subject.

46. The program according to claim 43, wherein the method comprises evaluating the effectiveness of a vaccine against the disease and evaluating defensive ability against re-contraction of the disease in the subject.

47. The program according to claim 43, wherein the method comprises evaluating the effectiveness of a cancer immunodrug against the disease in the subject.

48. The program according to claim 43, wherein the disease-related factor is an autoimmune disease-related factor, and in the method, the measurement further comprises measuring the level or amount of the follicular T cell reactive with the autoimmune disease-related factor, and performing risk evaluation and pathology evaluation of an autoimmune disease associated with an autoantibody of the subject.

49. The program according to any one of claims 43 to 48, wherein the step of providing information is performed by ELISPOT, flow cytometry, peripheral blood mononuclear cell (PBMC) restimulation assay, next generation sequencer/genetic testing (repertoire analysis), immunostaining (histopathological examination), or in situ analysis.

50. The program according to any one of claims 43 to 49,
wherein the method further comprises comparing the information relating to the level, spatial distribution, or amount of the follicular T cell reactive with the disease-related factor of the disease in the subject with a mean or median value of the information relating to the level, spatial distribution, or amount of the follicular T cell reactive with the disease-related factor of the disease in a test subject not affected with the disease.

51. The program according to any one of claims 43 to 47, 49 and 50, wherein the disease is an infectious disease or cancer.

52. The program according to any one of claims 43 to 47, 49 and 50, wherein the disease is a viral infectious disease.

53. The program according to any one of claims 43 to 50,
wherein the disease is an allergy or an autoimmune disease.

54. A computer readable recording medium storing the program according to any one of claims 43 to 53.

55. A method for evaluating a disease in a subject, comprising measuring the level or amount of follicular T cells reactive with a disease-related factor of the disease in the subject.

56. A method for evaluating a disease in a subject, comprising a step of providing information relating to a level, spatial distribution, or amount of a follicular T cell reactive with a disease-related factor of the disease in the subject, and a step of evaluating information relating to the level, spatial distribution, or amount of the follicular T cell with regard to the disease.
